# EUROPEAN PATENT SPECIFICATION

(11) **EP 1 274 861 B1**
(45) Date of publication and mention of the grant of the patent: **23.12.2009**
(21) Application number: 01922785.9
(22) Date of filing: 27.03.2001
(51) Int. Cl.: C12Q 1/68

(54) **COMPOSITIONS AND METHODS FOR IDENTIFYING AND TARGETING CANCER CELLS**
ZUSAMMENSETZUNGEN UND METHODEN ZUR IDENTIFIZIERUNG VON KREBSZELLEN
COMPOSITIONS ET PROCEDES D'IDENTIFICATION ET DE CIBLAGE DE CELLULES CANCEREUSES

(30) Priority: 27.03.2000 US 192229 P
(43) Date of publication of application: 15.01.2003
(73) Proprietor: THOMAS JEFFERSON UNIVERSITY, Philadelphia, PA (US)
(72) Inventor: WALDMAN, Scott, A., Ardmore, PA 19003 (US); PARK, Jason, Philadelphia, PA 19107 (US); SCHULZ, Stephanie, West Chester, PA 19380 (US)
(74) Representative: Hallybone, Huw George
(86) International application number: PCT/US2001/009918
(87) International publication number: WO 2001/073133

(56) References cited:
- WO-A1-00/20640
- US-A- 5 518 888
- US-A- 5 601 990
- US-A- 5 731 159
- MAKINO MASATO ET AL: "p53 as an indicator of lymph node metastases in invasive early colorectal cancer" ANTICANCER RESEARCH, vol. 20, no. 3B, May 2000 (2000-05), pages 2055-2060, XP008046541 ISSN: 0250-7005
- WILTZ O ET AL: "EXPRESSION OF ENZYMATICALLY ACTIVE SUCRASE ISOMALTASE IS A UBIQUITOUS PROPERTY OF COLON ADENOCARCINOMAS" GASTROENTEROLOGY, vol. 100, no. 5 PART 1, 1991, pages 1266-1278, XP008046527 ISSN: 0016-5085
- LISE M ET AL: "Association between sucrase-isomaltase and p53 expression in colorectal cancer." ANNALS OF SURGICAL ONCOLOGY : THE OFFICIAL JOURNAL OF THE SOCIETY OF SURGICAL ONCOLOGY. MAR 1997, vol. 4, no. 2, March 1997 (1997-03), pages 176-183, XP008046558 ISSN: 1068-9265
- ZWEIBAUM A ET AL: "SUCRASE ISO MALTASE A MARKER OF FETAL AND MALIGNANT EPITHELIAL CELLS OF THE HUMAN COLON" INTERNATIONAL JOURNAL OF CANCER, vol. 32, no. 4, 1983, pages 407-412, XP008046567 ISSN: 0020-7136
- JESSUP J M ET AL: "Sucrase-isomaltase is an independent prognostic marker for colorectal carcinoma." DISEASES OF THE COLON AND RECTUM. DEC 1995, vol. 38, no. 12, December 1995 (1995-12), pages 1257-1264, XP008046582 ISSN: 0012-3706
- MALLO GUSTAVO VIDAL ET AL: "Expression of the Cdx1 and Cdx2 homeotic genes leads to reduced malignancy in colon cancer-derived cells" JOURNAL OF BIOLOGICAL CHEMISTRY, vol. 273, no. 22, 29 May 1998 (1998-05-29), pages 14030-14036, XP002338465 ISSN: 0021-9258
- MALLO G V ET AL: "MOLECULAR CLONING, SEQUENCING AND EXPRESSION OF THE MRNA ENCODING HUMAN CDX1 AND CDX2 HOMEOBOX. DOWN-REGULATION OF CDX1 AND CDX2 MRNA EXPRESSION DURING COLORECTAL CARCINOGENESIS" INTERNATIONAL JOURNAL OF CANCER, NEW YORK, NY, US, vol. 74, no. 1, 20 February 1997 (1997-02-20), pages 35-44, XP000946393 ISSN: 0020-7136
- DI GUGLIELMO^1 M D ET AL: "Nucleotide requirements for CDX2 binding to the cis promoter element mediating intestine-specific expression of guanylyl cyclase C" FEBS LETTERS, ELSEVIER SCIENCE PUBLISHERS, AMSTERDAM, NL, vol. 507, no. 2, 26 October 2001 (2001-10-26), pages 128-132, XP004312033 ISSN: 0014-5793
- MALLO ET AL.: 'Molecular cloning, sequencing and expression of the mRNA encoding human CDX1 and CDX2 homeobox' INTERNATIONAL JOURNAL OF CANCER vol. 74, 1997, pages 35 - 44, XP002944929
- WU ET AL.: 'Sucrase-isomaltase gene expression in Barrett's esophagus and adenocarcinoma' GASTEROENLOGY vol. 105, no. 3, 1993, pages 837 - 844, XP002944930

### SEQUENCE LISTING

<110> Thomas Jefferson University
   Waldman, Scott A.
   Park, Jason
   Schulz, Stephanie
<120> Compositions And Methods For Identifying And Targeting Cancer Cells
<130> TJU2470
<160> 12
<170> PatentIn version 3.0
<210> 1
   <211> 6021
   <212> DNA
   <213> Homo sapiens
<400> 1
   tattttggca gccttatcca agtctggtac aacatagcaa agagaacagg ctatgaaata      60
   agatggcaag aaagaaattt agtggattgg aaatctctct gattgtcctt tttgtcatag     120
   ttactataat agctattgcc ttaattgttg ttttagcaac taagacacct gctgttgatg     180
   aaattagtga ttctacttca actccagcta ctactcgtgt gactacaaat ccttctgatt     240
   caggaaaatg tccaaatgtg ttaaatgatc ctgtcaatgt gagaataaac tgcattccag     300
   aacaattccc aacagaggga atttgtgcac agagaggctg ctgctggagg ccgtggaatg     360
   actctcttat tccttggtgc ttcttcgttg ataatcatgg ttataacgtt caagacatga     420
   caacaacaag tattggagtt gaagccaaat taaacaggat accttcacct acactatttg     480
   gaaatgacat caacagtgtt ctcttcacaa ctcaaaatca gacacccaat cgtttccggt     540
   tcaagattac tgatccaaat aatagaagat atgaagttcc tcatcagtat gtaaaagagt     600
   ttactggacc cacagtttct gatacgttgt atgatgtgaa ggttgcccaa aacccattta     660
   gcatccaagt tattaggaaa agcaacggta aaactttgtt tgacaccagc attggtccct     720
   tagtgtactc tgaccagtac ttacagatct cagcccgtct tccaagtgat tatatttatg     780
   gtattggaga acaagttcat aagagatttc gtcatgattt atcctggaaa acatggccaa     840
   tttttactcg agaccaactt cctggtgata ataataataa tttatacggc catcaaacat     900
   tctttatgtg tattgaagat acatctggaa agtcattcgg tgttttttta atgaatagca     960
   atgcaatgga gatttttatc cagcctactc caatagtaac atatagagtt accggtggca    1020
   ttctggattt ttacatcctt ctaggagata caccagaaca agtagttcaa cagtatcaac    1080
   agcttgttgg actaccagca atgccagcat attggaatct tggattccaa ctaagtcgct    1140
   ggaattataa gtcactagat gtagtgaaag aagtggtaag gagaaaccgg gaagctggca    1200
   taccatttga tacacaggtc actgatattg actacatgga agacaagaaa gactttactt    1260
   atgatcaagt tgcgtttaac ggactccctc aatttgtgca agatttgcat gaccatggac    1320
   agaaatatgt catcatcttg gaccctgcaa tttccatagg tcgacgtgcc aatggaacaa   1380
   catatgcaac ctatgagagg ggaaacacac aacatgtgtg gataaatgag tcagatggaa   1440
   gtacaccaat tattggagag gtatggccag gattaacagt ataccctgat ttcactaatc   1500
   caaactgcat tgattggtgg gcaaatgaat gcagtatttt ccatcaagaa gtgcaatatg   1560
   atggactttg gattgacatg aatgaagttt ccagctttat tcaaggttca acaaaaggat   1620
   gtaatgtaaa caaattgaat tatccaccgt ttactcctga tattcttgac aaactcatgt   1680
   attccaaaac aatttgcatg gatgctgtgc agaactgggg taaacagtat gatgttcata   1740
   gcctctatgg atacagcatg gctatagcca cagagcaagc tgtacaaaaa gtttttccta   1800
   ataagagaag cttcattctt acccgctcaa catttgctgg atctggaaga catgctgctc   1860
   attggttagg agacaatact gcttcatggg aacaaatgga atggtctata actggaatgc   1920
   tggagttcag tttgtttgga atacctttgg ttggagcaga catctgtgga tttgtggctg   1980
   aaaccacaga agaactttgc agaagatgga tgcaacttgg ggcattttat ccattttcca   2040
   gaaaccataa ttctgacgga tatgaacatc aggatcctgc attttttggg cagaattcac   2100
   ttttggttaa atcatcaagg cagtatttaa ctattcgcta caccttatta cccttcctct   2160
   acactctgtt ttataaagcc catgtgtttg gagaaacagt agcaagacca gttcttcatg   2220
   agttttatga ggatacgaac agctggattg aggacactga gtttttgtgg ggccctgcat   2280
   tacttattac tcctgttcta aaacagggag cagatactgt gagtgcctac atccctgatg   2340
   ctatttggta tgattatgaa tctggtgcaa aaaggccatg gaggaaacaa cgggttgata   2400
   tgtatcttcc agcagacaaa ataggattac atcttagagg aggttatatc atccccattc   2460
   aagaaccaga tgtaacaaca acagcaagcc gtaagaatcc tctaggactt atagtcgcat   2520
   taggtgaaaa caacacagcc aaaggagact tttctgggga tgatggagaa actaaagata   2580
   caatacaaaa tggcaactac atattatata cattttcagt ttctaataac acattagata   2640
   ttgtgtgcac acattcatca tatcaggaag gaactacctt agcatttcag actgtaaaaa   2700
   tccttgggtt gacagacagt gttacagaag ttagagtggc ggaaaataat caaccaatga   2760
   acgctcattc caatttcact tatgatgctt ctaaccaggt tctcctaatt gcagatctca   2820
   aacttaatct tggaagaaac tttagtgttc aatggaatca aattttctca gaaaatgaaa   2880
   gatttaattg ttatccagat gcagatttgg caactgaaca aaagtgcaca caacgtggct   2940
   gtgtatggag aacgggttct tctctatcca aagcacctga gtgttacttt cccagacaag   3000
   ataactctta ttcagtcaac tcagctcgct attcatccat gggtataaca gctgacctcc   3060
   aactaaatac tgcaaatgcc agaataaagt taccttctga ccccatctca actcttcgtg   3120
   tggaggtgaa atatcacaaa aatgatatgt tgcagtttaa gatttatgat ccccaaaaga   3180
   agagatatga agtaccagta ccgttaaaca ttccaaccac cccaataagt acttatgaag   3240
   acagacttta tgatgtggaa atcaaggaaa atcctttggg catccagatt cgacggagaa   3300
   gcagtggaag agtcatttgg gattcttggc tgcctggatt tgcttttaat gaccagttca 3360
   ttcaaatatc gactcgcctg ccatcagaat atatatatgg ttttggggaa gtggaacata 3420
   cagcatttaa gcgagatctg aactggaata cttggggaat gttcacaaga gaccaacccc 3480
   ctggttacaa acttaattcc tatggatttc atccctatta catggctctg gaagaggagg 3540
   gcaatgctca tggtgttttc ttactcaaca gcaatgcaat ggatgttaca ttccagccaa 3600
   ctcctgctct aacttaccgt acagttggag ggatcttgga tttttatatg tttttgggcc 3660
   caactccaca agttgcaaca aagcaatacc atgaagtaat tggccatcca gtcatgccag 3720
   cttattgggc tttgggattc caattatgtc gttatggata tgcaaatact tcagaggttc 3780
   gggaattata tgacgctatg gtggctgcta acatccccta tgatgttcag tacacagaca 3840
   ttgactacat ggaaaggcag ctagacttta caattggtga agcattccag gaccttcctc 3900
   agtttgttga caaaataaga ggagaaggaa tgagatacat tattatcctg gatccagcaa 3960
   tttcaggaaa tgaaacaaag acttaccctg catttgaaag aggacagcag aatgatgtct 4020
   ttgtcaaatg gccaaacacc aatgacattt gttgggcaaa ggtttggcca gatttgccca 4080
   acataacaat agataaaact ctaacggaag atgaagctgt taatgcttcc agagctcatg 4140
   tagctttccc agatttcttc aggacttcca cagcagagtg gtgggccaga gaaattgtgg 4200
   acttttacaa tgaaaagatg aagtttgatg gtttgtggat tgatatgaat gagccatcaa 4260
   gttttgtaaa tggaacaact actaatcaat gcagaaatga cgaactaaat tatccacctt 4320
   atttcccaga actcacaaaa agaactgatg gattacattt cagaacaatt tgcatggaag 4380
   ctgagcagat tcttagtgat ggaacatcag ttttgcatta cgatgttcac aatctctatg 4440
   gatggtcaca gatgaaacct actcatgatg cattgcaaaa gacaactgga aaaagaggga 4500
   ttgtaatttc tcgttccacg tatcctacta gtggacgatg gggaggacac tggcttggag 4560
   acaactatgc acgatgggac aacatggaca aatcaatcat tggtatgatg gaatttagtc 4620
   tgtttggaat atcatatact ggagcagaca tctgtggttt tttcaacaac tcagaatatc 4680
   atctctgtac ccgctggatg caacttggag cattttatcc atactcaagg aatcacaaca 4740
   ttgcaaatac tagaagacaa gatcccgctt cctggaatga aacttttgct gaaatgtcaa 4800
   ggaatattct aaatattaga tacaccttat tgccctattt ttacacacaa atgcatgaaa 4860
   ttcatgctaa tggtggcact gttatccgac cccttttgca tgagttcttt gatgaaaaac 4920
   caacctggga tatattcaag cagttcttat ggggtccagc atttatggtt accccagtac 4980
   tggaacctta tgttcaaact gtaaatgcct acgtccccaa tgctcggtgg tttgactacc 5040
   atacaggcaa agatattggc gtcagaggac aatttcaaac atttaatgct tcttatgaca 5100
   caataaacct acatgtccgt ggtggtcaca tcctaccatg tcaagagcca gctcaaaaca 5160
   cattttacag tcgacaaaaa cacatgaagc tcattgttgc tgcagatgat aatcagatgg 5220
   cacagggttc tctgttttgg gatgatggag agagtataga cacctatgaa agagacctat   5280
   atttatctgt acaatttaat ttaaaccaga ccaccttaac aagcactata ttgaagagag   5340
   gttacataaa taaaagtgaa acgaggcttg gatcccttca tgtatggggg aaaggaacta   5400
   ctcctgtcaa tgcagttact ctaacgtata acggaaataa aaattcgctt ccttttaatg   5460
   aagacactac caacatgata ttacgtattg atctgaccac acacaatgtt actctagaag   5520
   aaccaataga aatcaactgg tcatgaagat caccatcaat tttagttgtc aatgggaaaa   5580
   aacaccagga tttaagtttc acagcactta caattttccc tcttcacttg gttcttgtac   5640
   tctacaaaat atagctttca taacatcgaa aagttatttt gtagcgtaca tcaatgataa   5700
   tgctaatttt attatagtaa tgtgacttgg attcaatttt aaggcatatt taacaaaatt   5760
   tgaatagccc tatttatcct tgttaagtat cagctacaat tgtaaactag ttactaaaca   5820
   tgtatgtaaa tagctaagat ataatttaaa cgtgattttt aaattaaata aaatttttat   5880
   gtaattatat atactatatt tttctcaatg tttagcagat ttaagatatg taacaacaat   5940
   tatttgaaga tttaattact tcttagtatg tgcatttaat tagaaaaaga gaataaaaaa   6000
   tgtaagtgta aaaaaaaaaa a                                             6021
<210> 2
   <211> 1827
   <212> PRT
   <213> Homo sapiens
<400> 2
   Met Ala Arg Lys Lys Phe Ser Gly Leu Glu Ile Ser Leu Ile Val Leu
   1               5                   10                  15
   Phe Val Ile Val Thr Ile Ile Ala Ile Ala Leu Ile Val Val Leu Ala
               20                  25                  30
   Thr Lys Thr Pro Ala Val Asp Glu Ile Ser Asp Ser Thr Ser Thr Pro
           35                  40                  45
   Ala Thr Thr Arg Val Thr Thr Asn Pro Ser Asp Ser Gly Lys Cys Pro
       50                  55                  60
   Asn Val Leu Asn Asp Pro Val Asn Val Arg Ile Asn Cys Ile Pro Glu
   65                  70                  75                  80
   Gln Phe Pro Thr Glu Gly Ile Cys Ala Gln Arg Gly Cys Cys Trp Arg
                   85                  90                  95
   Pro Trp Asn Asp Ser Leu Ile Pro Trp Cys Phe Phe Val Asp Asn His
               100                 105                 110
   Gly Tyr Asn Val Gln Asp Met Thr Thr Thr Ser Ile Gly Val Glu Ala
           115                 120                 125
   Lys Leu Asn Arg Ile Pro Ser Pro Thr Leu Phe Gly Asn Asp Ile Asn
       130                 135                 140
   Ser Val Leu Phe Thr Thr Gln Asn Gln Thr Pro Asn Arg Phe Arg Phe
   145                 150                 155                 160
   Lys Ile Thr Asp Pro Asn Asn Arg Arg Tyr Glu Val Pro His Gln Tyr
                   165                 170                 175
   Val Lys Glu Phe Thr Gly Pro Thr Val Ser Asp Thr Leu Tyr Asp Val
               180                 185                 190
   Lys Val Ala Gln Asn Pro Phe Ser Ile Gln Val Ile Arg Lys Ser Asn
           195                 200                 205
   Gly Lys Thr Leu Phe Asp Thr Ser Ile Gly Pro Leu Val Tyr Ser Asp
       210                 215                 220
   Gln Tyr Leu Gln Ile Ser Ala Arg Leu Pro Ser Asp Tyr Ile Tyr Gly
   225                 230                 235                 240
   Ile Gly Glu Gln Val His Lys Arg Phe Arg His Asp Leu Ser Trp Lys
                   245                 250                 255
   Thr Trp Pro Ile Phe Thr Arg Asp Gln Leu Pro Gly Asp Asn Asn Asn
               260                 265                 270
   Asn Leu Tyr Gly His Gln Thr Phe Phe Met Cys Ile Glu Asp Thr Ser
           275                 280                 285
   Gly Lys Ser Phe Gly Val Phe Leu Met Asn Ser Asn Ala Met Glu Ile
       290                 295                 300
   Phe Ile Gln Pro Thr Pro Ile Val Thr Tyr Arg Val Thr Gly Gly Ile
   305                 310                 315                 320
   Leu Asp Phe Tyr Ile Leu Leu Gly Asp Thr Pro Glu Gln Val Val Gln
                   325                 330                 335
   Gln Tyr Gln Gln Leu Val Gly Leu Pro Ala Met Pro Ala Tyr Trp Asn
               340                 345                 350
   Leu Gly Phe Gln Leu Ser Arg Trp Asn Tyr Lys Ser Leu Asp Val Val
           355                 360                 365
   Lys Glu Val Val Arg Arg Asn Arg Glu Ala Gly Ile Pro Phe Asp Thr
       370                 375                 380
   Gln Val Thr Asp Ile Asp Tyr Met Glu Asp Lys Lys Asp Phe Thr Tyr
   385                 390                 395                 400
   Asp Gln Val Ala Phe Asn Gly Leu Pro Gln Phe Val Gln Asp Leu His
                   405                 410                 415
   Asp His Gly Gln Lys Tyr Val Ile Ile Leu Asp Pro Ala Ile Ser Ile
               420                 425                 430
   Gly Arg Arg Ala Asn Gly Thr Thr Tyr Ala Thr Tyr Glu Arg Gly Asn
           435                 440                 445
   Thr Gln His Val Trp Ile Asn Glu Ser Asp Gly Ser Thr Pro Ile Ile
       450                 455                 460
   Gly Glu Val Trp Pro Gly Leu Thr Val Tyr Pro Asp Phe Thr Asn Pro
   465                 470                 475                 480
   Asn Cys Ile Asp Trp Trp Ala Asn Glu Cys Ser Ile Phe His Gln Glu
                   485                 490                 495
   Val Gln Tyr Asp Gly Leu Trp Ile Asp Met Asn Glu Val Ser Ser Phe
   500 505 510
   Ile Gln Gly Ser Thr Lys Gly Cys Asn Val Asn Lys Leu Asn Tyr Pro
   515 520 525
   Pro Phe Thr Pro Asp Ile Leu Asp Lys Leu Met Tyr Ser Lys Thr Ile
   530 535 540
   Cys Met Asp Ala Val Gln Asn Trp Gly Lys Gln Tyr Asp Val His Ser
   545 550 555 560
   Leu Tyr Gly Tyr Ser Met Ala Ile Ala Thr Glu Gln Ala Val Gln Lys
   565 570 575
   Val Phe Pro Asn Lys Arg Ser Phe Ile Leu Thr Arg Ser Thr Phe Ala
   580 585 590
   Gly Ser Gly Arg His Ala Ala His Trp Leu Gly Asp Asn Thr Ala Ser
   595 600 605
   Trp Glu Gln Met Glu Trp Ser Ile Thr Gly Met Leu Glu Phe Ser Leu
   610 615 620
   Phe Gly Ile Pro Leu Val Gly Ala Asp Ile Cys Gly Phe Val Ala Glu
   625 630 635 640
   Thr Thr Glu Glu Leu Cys Arg Arg Trp Met Gln Leu Gly Ala Phe Tyr
   645 650 655
   Pro Phe Ser Arg Asn His Asn Ser Asp Gly Tyr Glu His Gln Asp Pro
   660 665 670
   Ala Phe Phe Gly Gln Asn Ser Leu Leu Val Lys Ser Ser Arg Gln Tyr
   675 680 685
   Leu Thr Ile Arg Tyr Thr Leu Leu Pro Phe Leu Tyr Thr Leu Phe Tyr
   690 695 700
   Lys Ala His Val Phe Gly Glu Thr Val Ala Arg Pro Val Leu His Glu
   705 710 715 720
   Phe Tyr Glu Asp Thr Asn Ser Trp Ile Glu Asp Thr Glu Phe Leu Trp
   725 730 735
   Gly Pro Ala Leu Leu Ile Thr Pro Val Leu Lys Gln Gly Ala Asp Thr
   740 745 750
   Val Ser Ala Tyr Ile Pro Asp Ala Ile Trp Tyr Asp Tyr Glu Ser Gly
   755 760 765
   Ala Lys Arg Pro Trp Arg Lys Gln Arg Val Asp Met Tyr Leu Pro Ala
   770 775 780
   Asp Lys Ile Gly Leu His Leu Arg Gly Gly Tyr Ile Ile Pro Ile Gln
   785 790 795 800
   Glu Pro Asp Val Thr Thr Thr Ala Ser Arg Lys Asn Pro Leu Gly Leu
   805 810 815
   Ile Val Ala Leu Gly Glu Asn Asn Thr Ala Lys Gly Asp Phe Phe Trp
   820 825 830
   Asp Asp Gly Glu Thr Lys Asp Thr Ile Gln Asn Gly Asn Tyr Ile Leu
   835 840 845
   Tyr Thr Phe Ser Val Ser Asn Asn Thr Leu Asp Ile Val Cys Thr His
       850                 855                 860
   Ser Ser Tyr Gln Glu Gly Thr Thr Leu Ala Phe Gln Thr Val Lys Ile
   865                 870                 875                 880
   Leu Gly Leu Thr Asp Ser Val Thr Glu Val Arg Val Ala Glu Asn Asn
                   885                 890                 895
   Gln Pro Met Asn Ala His Ser Asn Phe Thr Tyr Asp Ala Ser Asn Gln
               900                 905                 910
   Val Leu Leu Ile Ala Asp Leu Lys Leu Asn Leu Gly Arg Asn Phe Ser
           915                 920                 925
   Val Gln Trp Asn Gln Ile Phe Ser Glu Asn Glu Arg Phe Asn Cys Tyr
       930                 935                 940
   Pro Asp Ala Asp Leu Ala Thr Glu Gln Lys Cys Thr Gln Arg Gly Cys
   945                 950                 955                 960
   Val Trp Arg Thr Gly Ser Ser Leu Ser Lys Ala Pro Glu Cys Tyr Phe
                   965                 970                 975
   Pro Arg Gln Asp Asn Ser Tyr Ser Val Asn Ser Ala Arg Tyr Ser Ser
               980                 985                 990
   Met Gly Ile Thr Ala Asp Leu Gln  Leu Asn Thr Ala Asn  Ala Arg Ile
           995                 1000                 1005
   Lys Leu  Pro Ser Asp Pro Ile  Ser Thr Leu Arg Val  Glu Val Lys
       1010                 1015                 1020
   Tyr His  Lys Asn Asp Met Leu  Gln Phe Lys Ile Tyr  Asp Pro Gln
       1025                 1030                 1035
   Lys Lys  Arg Tyr Glu Val Pro  Val Pro Leu Asn Ile  Pro Thr Thr
       1040                 1045                 1050
   Pro Ile  Ser Thr Tyr Glu Asp  Arg Leu Tyr Asp Val  Glu Ile Lys
       1055                 1060                 1065
   Glu Asn  Pro Phe Gly Ile Gln  Ile Arg Arg Arg Ser  Ser Gly Arg
       1070                 1075                 1080
   Val Ile  Trp Asp Ser Trp Leu  Pro Gly Phe Ala Phe  Asn Asp Gln
       1085                 1090                 1095
   Phe Ile  Gln Ile Ser Thr Arg  Leu Pro Ser Glu Tyr  Ile Tyr Gly
       1100                 1105                 1110
   Phe Gly  Glu Val Glu His Thr  Ala Phe Lys Arg Asp  Leu Asn Trp
       1115                 1120                 1125
   Asn Thr  Trp Gly Met Phe Thr  Arg Asp Gln Pro Pro  Gly Tyr Lys
       1130                 1135                 1140
   Leu Asn  Ser Tyr Gly Phe His  Pro Tyr Tyr Met Ala  Leu Glu Glu
       1145                 1150                 1155
   Glu Gly  Asn Ala His Gly Val  Phe Leu Leu Asn Ser  Asn Ala Met
       1160                 1165                 1170
   Asp Val  Thr Phe Gln Pro Thr  Pro Ala Leu Thr Tyr  Arg Thr Val
       1175                 1180                 1185
   Gly Gly Ile Leu Asp Phe Tyr Met Phe Leu Gly Pro Thr Pro Gln
   1190 1195 1200
   Val Ala Thr Lys Gln Tyr His Glu Val Ile Gly His Pro Val Met
   1205 1210 1215
   Pro Ala Tyr Trp Ala Leu Gly Phe Gln Leu Cys Arg Tyr Gly Tyr
   1220 1225 1230
   Ala Asn Thr Ser Glu Val Arg Glu Leu Tyr Asp Ala Met Val Ala
   1235 1240 1245
   Ala Asn Ile Pro Tyr Asp Val Gln Tyr Thr Asp Ile Asp Tyr Met
   1250 1255 1260
   Glu Arg Gln Leu Asp Phe Thr Ile Gly Glu Ala Phe Gln Asp Leu
   1265 1270 1275
   Pro Gln Phe Val Asp Lys Ile Arg Gly Glu Gly Met Arg Tyr Ile
   1280 1285 1290
   Ile Ile Leu Asp Pro Ala Ile Ser Gly Asn Glu Thr Lys Thr Tyr
   1295 1300 1305
   Pro Ala Phe Glu Arg Gly Gln Gln Asn Asp Val Phe Val Lys Trp
   1310 1315 1320
   Pro Asn Thr Asn Asp Ile Cys Trp Ala Lys Val Trp Pro Asp Leu
   1325 1330 1335
   Pro Asn Ile Thr Ile Asp Lys Thr Leu Thr Glu Asp Glu Ala Val
   1340 1345 1350
   Asn Ala Ser Arg Ala His Val Ala Phe Pro Asp Phe Phe Arg Thr
   1355 1360 1365
   Ser Thr Ala Glu Trp Trp Ala Arg Glu Ile Val Asp Phe Tyr Asn
   1370 1375 1380
   Glu Lys Met Lys Phe Asp Gly Leu Trp Ile Asp Met Asn Glu Pro
   1385 1390 1395
   Ser Ser Phe Val Asn Gly Thr Thr Thr Asn Gln Cys Arg Asn Asp
   1400 1405 1410
   Glu Leu Asn Tyr Pro Pro Tyr Phe Pro Glu Leu Thr Lys Arg Thr
   1415 1420 1425
   Asp Gly Leu His Phe Arg Thr Ile Cys Met Glu Ala Glu Gln Ile
   1430 1435 1440
   Leu Ser Asp Gly Thr Ser Val Leu His Tyr Asp Val His Asn Leu
   1445 1450 1455
   Tyr Gly Trp Ser Gln Met Lys Pro Thr His Asp Ala Leu Gln Lys
   1460 1465 1470
   Thr Thr Gly Lys Arg Gly Ile Val Ile Ser Arg Ser Thr Tyr Pro
   1475 1480 1485
   Thr Ser Gly Arg Trp Gly Gly His Trp Leu Gly Asp Asn Tyr Ala
   1490 1495 1500
   Arg Trp Asp Asn Met Asp Lys Ser Ile Ile Gly Met Met Glu Phe
       1505                1510                1515
   Ser Leu  Phe Gly Ile Ser Tyr  Thr Gly Ala Asp Ile  Cys Gly Phe
       1520                1525                1530
   Phe Asn  Asn Ser Glu Tyr His  Leu Cys Thr Arg Trp  Met Gln Leu
       1535                1540                1545
   Gly Ala  Phe Tyr Pro Tyr Ser  Arg Asn His Asn Ile  Ala Asn Thr
       1550                1555                1560
   Arg Arg  Gln Asp Pro Ala Ser  Trp Asn Glu Thr Phe  Ala Glu Met
       1565                1570                1575
   Ser Arg  Asn Ile Leu Asn Ile  Arg Tyr Thr Leu Leu  Pro Tyr Phe
       1580                1585                1590
   Tyr Thr  Gln Met His Glu Ile  His Ala Asn Gly Gly  Thr Val Ile
       1595                1600                1605
   Arg Pro  Leu Leu His Glu Phe  Phe Asp Glu Lys Pro  Thr Trp Asp
       1610                1615                1620
   Ile Phe  Lys Gln Phe Leu Trp  Gly Pro Ala Phe Met  Val Thr Pro
       1625                1630                1635
   Val Leu  Glu Pro Tyr Val Gln  Thr Val Asn Ala Tyr  Val Pro Asn
       1640                1645                1650
   Ala Arg  Trp Phe Asp Tyr His  Thr Gly Lys Asp Ile  Gly Val Arg
       1655                1660                1665
   Gly Gln  Phe Gln Thr Phe Asn  Ala Ser Tyr Asp Thr  Ile Asn Leu
       1670                1675                1680
   His Val  Arg Gly Gly His Ile  Leu Pro Cys Gln Glu  Pro Ala Gln
       1685                1690                1695
   Asn Thr  Phe Tyr Ser Arg Gln  Lys His Met Lys Leu  Ile Val Ala
       1700                1705                1710
   Ala Asp  Asp Asn Gln Met Ala  Gln Gly Ser Leu Phe  Trp Asp Asp
       1715                1720                1725
   Gly Glu  Ser Ile Asp Thr Tyr  Glu Arg Asp Leu Tyr  Leu Ser Val
       1730                1735                1740
   Gln Phe  Asn Leu Asn Gln Thr  Thr Leu Thr Ser Thr  Ile Leu Lys
       1745                1750                1755
   Arg Gly  Tyr Ile Asn Lys Ser  Glu Thr Arg Leu Gly  Ser Leu His
       1760                1765                1770
   Val Trp  Gly Lys Gly Thr Thr  Pro Val Asn Ala Val  Thr Leu Thr
       1775                1780                1785
   Tyr Asn  Gly Asn Lys Asn Ser  Leu Pro Phe Asn Glu  Asp Thr Thr
       1790                1795                1800
   Asn Met  Ile Leu Arg Ile Asp  Leu Thr Thr His Asn  Val Thr Leu
       1805                1810                1815
   Glu Glu  Pro Ile Glu Ile Asn  Trp Ser
       1820                1825
<210> 3
   <211> 1745
   <212> DNA
   <213> Homo sapiens
<400> 3
   gcgcccctgg cagccttcaa cgtcggtccc caggcagcat ggtgaggtct gctcccggac      60
   cctcgccacc atgtacgtga gctacctcct ggacaaggac gtgagcatgt accctagctc     120
   cgtgcgccac tctggcggcc tcaacctggc gccgcagaac ttcgtcagcc ccccgcagta     180
   cccggactac ggcggttacc acgtggcggc cgcagctgca gcgcagaact tggacagcgc     240
   gcagtccccg gggccatcct ggccggcagc gtatggcgcc ccactccggg aggactggaa     300
   tggctacgcg cccggaggcg cggccgccgc caacgccgtg gctcacgcgc tcaacggtgg     360
   ctccccggcc gcagccatgg gctacagcag ccccgcagac taccatccgc accaccaccc     420
   gcatcaccac ccgcaccacc cggccgccgc gccttcctgc gcttctgggc tgctgcaaac     480
   gctcaacccc ggccctcctg ggcccgccgc caccgctgcc gccgagcagc tgtctcccgg     540
   cggccagcgg cggaacctgt gcgagtggat gcggaagccg gcgcagcagt ccctcggcag     600
   ccaagtgaaa accaggacga aagacaaata tcgagtggtg tacacggacc accagcggct     660
   ggagctggag aaggagtttc actacagtcg ctacatcacc atccggagga aagccgagct     720
   agccgccacg ctggggctct ctgagaggca ggttaaaatc tggtttcaga accgcagagc     780
   aaaggagagg aaaatcaaca agaagaagtt gcagcagcaa cagcagcagc agccaccaca     840
   gccgcctccg ccgccaccac agcctcccca gcctcagcca ggtcctctga gaagtgtccc     900
   agagcccttg agtccggtgt cttccctgca agcctcagtg tctggctctg tccctggggt     960
   tctggggcca actggggggg tgctaaaccc caccgtcacc cagtgaccca ccggggtctg    1020
   cagcggcaga gcaattccag gctgagccat gaggagcgtg gactctgcta gactcctcag    1080
   gagagacccc tcccctccca cccacagcca tagacctaca gacctggctc tcagaggaaa    1140
   aatgggagcc aggagtaaga caagtgggat ttggggcctc aagaaatata ctctcccaga    1200
   tttttacttt ttccatctgg ctttttctgc cactgaggag acagaaagcc tccgctgggc    1260
   ttcattccgg actggcagaa gcattgcctg gactgaccac accaaccagc ttcatctatc    1320
   cgactcttct cttcctagat ctgcaggctg cacctctggc tagagccgag gggagagagg    1380
   gactcaaggg aaaggcaagc ttgaggccaa gatggctgct gcctgctcat ggccctcgga    1440
   ggtccagctg ggcctcctgc ctccgggcag caaggtttac actgcggaac gcaaaggcag    1500
   ctaagataga aagctggact gaccaaagac tgcagaaccc ccaggtggcc ctgcgtcttt    1560
   tttctcttcc ctttcccaga ccaggaaagg cttggctggt gtatgcacag ggtgtggtat    1620
   gagggggtgg ttattggact ccaggcctga ccagggggcc cgaacaggac ttgttagaga    1680
   gcctgtcacc agagcttctc tgggctgaat gtatgtcagt gctataaatg ccagagccaa    1740
   cctgg                                                                1745
<210> 4
   <211> 311
   <212> PRT
   <213> Homo sapiens
<400> 4
   Met Tyr Val Ser Tyr Leu Leu Asp Lys Asp Val Ser Met Tyr Pro Ser
   1               5                   10                  15
   Ser Val Arg His Ser Gly Gly Leu Asn Leu Ala Pro Gln Asn Phe Val
               20                  25                  30
   Ser Pro Pro Gln Tyr Pro Asp Tyr Gly Gly Tyr His Val Ala Ala Ala
           35                  40                  45
   Ala Ala Ala Gln Asn Leu Asp Ser Ala Gln Ser Pro Gly Pro Ser Trp
       50                  55                  60
   Pro Ala Ala Tyr Gly Ala Pro Leu Arg Glu Asp Trp Asn Gly Tyr Ala
   65                  70                  75                  80
   Pro Gly Gly Ala Ala Ala Ala Asn Ala Val Ala His Ala Leu Asn Gly
                   85                  90                  95
   Gly Ser Pro Ala Ala Ala Met Gly Tyr Ser Ser Pro Ala Asp Tyr His
               100                 105                 110
   Pro His His His Pro His His His Pro His His Pro Ala Ala Ala Pro
           115                 120                 125
   Ser Cys Ala Ser Gly Leu Leu Gln Thr Leu Asn Pro Gly Pro Pro Gly
       130                 135                 140
   Pro Ala Ala Thr Ala Ala Ala Glu Gln Leu Ser Pro Gly Gly Gln Arg
   145                 150                 155                 160
   Arg Asn Leu Cys Glu Trp Met Arg Lys Pro Ala Gln Gln Ser Leu Gly
                   165                 170                 175
   Ser Gln Val Lys Thr Arg Thr Lys Asp Lys Tyr Arg Val Val Tyr Thr
               180                 185                 190
   Asp His Gln Arg Leu Glu Leu Glu Lys Glu Phe His Tyr Ser Arg Tyr
           195                 200                 205
   Ile Thr Ile Arg Arg Lys Ala Glu Leu Ala Ala Thr Leu Gly Leu Ser
       210                 215                 220
   Glu Arg Gln Val Lys Ile Trp Phe Gln Asn Arg Arg Ala Lys Glu Arg
   225                 230                 235                 240
   Lys Ile Asn Lys Lys Lys Leu Gln Gln Gln Gln Gln Gln Gln Pro Pro
                   245                 250                 255
   Gln Pro Pro Pro Pro Pro Pro Gln Pro Pro Gln Pro Gln Pro Gly Pro
               260                 265                 270
   Leu Arg Ser Val Pro Glu Pro Leu Ser Pro Val Ser Ser Leu Gln Ala
           275                 280                 285
   Ser Val Ser Gly Ser Val Pro Gly Val Leu Gly Pro Thr Gly Gly Val
       290                 295                 300
   Leu Asn Pro Thr Val Thr Gln
   305                 310
<210> 5
   <211> 1699
   <212> DNA
   <213> Homo sapiens
<400> 5
   aggtgagcgg ttgctcgtcg tcggggcggc cggcagcggc ggctccaggg cccagcatgc     60
   gcgggggacc ccgcggccac catgtatgtg ggctatgtgc tggacaagga ttcgcccgtg    120
   taccccggcc cagccaggcc agccagcctc ggcctgggcc cgcaagccta cggccccccg    180
   gccccgcccc cggcgccccc gcagtacccc gacttctcca gctactctca cgtggagccg    240
   gcccccgcgc ccccgacggc ctggggggcg cccttccctg cgcccaagga cgactgggcc    300
   gccgcctacg gcccgggccc cgcggcccct gccgccagcc cagcttcgct ggcattcggg    360
   ccccctccag actttagccc ggtgccggcg ccccctgggc ccggcccggg cctcctggcg    420
   cagcccctcg ggggcccggg cacaccgtcc tcgcccggag cgcagaggcc gacgccctac    480
   gagtggatgc ggcgcagcgt ggcggccgga ggcggcggtg gcagcggtaa gactcggacc    540
   aaggacaagt accgcgtggt ctacaccgac caccaacgcc tggagctgga gaaggagttt    600
   cattacagcc gttacatcac aatccggcgg aaatcagagc tggctgccaa tctggggctc    660
   actgaacggc aggtgaagat ctggttccaa aaccggcggg caaaggagcg caaagtgaac    720
   aagaagaaac agcagcagca acagccccca cagccgccga tggcccacga catcacggcc    780
   accccagccg ggccatccct ggggggcctg tgtcccagca acaccagcct cctggccacc    840
   tcctctccaa tgcctgtgaa agaggagttt ctgccatagc cccatgccca gcctgtgcgc    900
   cgggggacct ggggactcgg gtgctgggag tgtggctcct gtgggcccag gaggtctggt    960
   ccgagtctca gccctgacct tctgggacat ggtggacagt cacctatcca ccctctgcat   1020
   ccccttggcc catctgtgca gtaagcctgt tggataaaga ccttccagct cctgtgttct   1080
   agacctctgg gggataaggg agtccagggt ggatgatctc aatctcccgt gggcatctca   1140
   agccccaaat ggttggggga ggggcctaga caaggctcca ggccccacct cctcctccat   1200
   acgttcagag gtgcagctgg aggctgctgt ggggaccaca ctgatcctgg agaaaaggga   1260
   tggagctgaa aaagatggaa tgcttgcaga gcatgacctg aggagggagg aacgtggtca   1320
   actcacacct gcctcttcct gcagcctcac ttctacctgc ccccatcata agggcactga   1380
   gcccttccca ggctggatac taagcacaaa gcccatagca ctgggctctg atggctgctc   1440
   cactgggtta cagaatcaca gccctcatga tcattctcag tgagggctct ggattgagag   1500
   ggaggccctg ggaggagaga agggggcaga gtcttcccta ccaggtttct acacccccgc   1560
   caggctgccc atcagggccc agggagcccc cagaggactt tattcggacc aagcagagct   1620
   cacagctgga caggtgttgt atatagagtg gaatctcttg gatgcagctt caagaataaa   1680
   tttttcttct cttttcaaa                                                1699
<210> 6
   <211> 265
   <212> PRT
   <213> Homo sapiens
<400> 6
   Met Tyr Val Gly Tyr Val Leu Asp Lys Asp Ser Pro Val Tyr Pro Gly
   1               5                   10                  15
   Pro Ala Arg Pro Ala Ser Leu Gly Leu Gly Pro Gln Ala Tyr Gly Pro
               20                  25                  30
   Pro Ala Pro Pro Pro Ala Pro Pro Gln Tyr Pro Asp Phe Ser Ser Tyr
           35                  40                  45
   Ser His Val Glu Pro Ala Pro Ala Pro Pro Thr Ala Trp Gly Ala Pro
       50                  55                  60
   Phe Pro Ala Pro Lys Asp Asp Trp Ala Ala Ala Tyr Gly Pro Gly Pro
   65                  70                  75                  80
   Ala Ala Pro Ala Ala Ser Pro Ala Ser Leu Ala Phe Gly Pro Pro Pro
                   85                  90                  95
   Asp Phe Ser Pro Val Pro Ala Pro Pro Gly Pro Gly Pro Gly Leu Leu
               100                 105                 110
   Ala Gln Pro Leu Gly Gly Pro Gly Thr Pro Ser Ser Pro Gly Ala Gln
           115                 120                 125
   Arg Pro Thr Pro Tyr Glu Trp Met Arg Arg Ser Val Ala Ala Gly Gly
       130                 135                 140
   Gly Gly Gly Ser Gly Lys Thr Arg Thr Lys Asp Lys Tyr Arg Val Val
   145                 150                 155                 160
   Tyr Thr Asp His Gln Arg Leu Glu Leu Glu Lys Glu Phe His Tyr Ser
                   165                 170                 175
   Arg Tyr Ile Thr Ile Arg Arg Lys Ser Glu Leu Ala Ala Asn Leu Gly
               180                 185                 190
   Leu Thr Glu Arg Gln Val Lys Ile Trp Phe Gln Asn Arg Arg Ala Lys
           195                 200                 205
   Glu Arg Lys Val Asn Lys Lys Lys Gln Gln Gln Gln Gln Pro Pro Gln
       210                 215                 220
   Pro Pro Met Ala His Asp Ile Thr Ala Thr Pro Ala Gly Pro Ser Leu
   225                 230                 235                 240
   Gly Gly Leu Cys Pro Ser Asn Thr Ser Leu Leu Ala Thr Ser Ser Pro
                   245                 250                 255
   Met Pro Val Lys Glu Glu Phe Leu Pro
               260                 265
<210> 7
   <211> 22
   <212> DNA
<213> Homo sapiens
<400> 7
   gcccatagct ctgacctttc tg 22
<210> 8
   <211> 24
   <212> DNA
   <213> Homo sapiens
<400> 8
   agagagatta gctgggcctc accc 24
<210> 9
   <211> 34
   <212> DNA
   <213> Homo sapiens
<400> 9
   cagctaatct ctctgtttat agctctgacc tttc 34
<210> 10
   <211> 35
   <212> DNA
   <213> Homo sapiens
<400> 10
   atctctctgt ttatagctct gacctttctg ggtgc 35
<210> 11
   <211> 34
   <212> DNA
   <213> Homo sapiens
<400> 11
   cagctaatct ctctgcccat agctctgacc tttc 34
<210> 12
   <211> 38
   <212> DNA
   <213> Homo sapiens
<400> 12
   gatccggctg gtgagggtgc aataaaactt tatgagta 38

## Description

### FIELD OF THE INVENTION

The present invention relates to *in vitro* diagnostic methods for detecting cancer cells of the alimentary canal, particularly primary and metastatic stomach and esophageal cancer and metastatic colorectal cancer, and to kits and reagents for performing such methods. The present invention relates to compounds and methods for *in vivo* imaging and treatment of tumors originating from the alimentary canal, particularly primary and metastatic stomach and esophageal tumors and metastatic colorectal tumors. The present invention relates to methods and compositions for making and using targeted gene therapy, antisense and drug compositions. The present invention relates to prophylactic and therapeutic vaccines against cancer cells of the alimentary canal, particularly primary and metastatic stomach and esophageal cancer and metastatic colorectal cancer and compositions and methods of making and using the same.

### BACKGROUND OF THE INVENTION

This application claims priority to U.S. Provisional Application Number 60/192,229 filed March 27, 2000.

This application is also related to U.S. Patent Number 5,518,888, issued May 21, 1996, U.S. Patent Number 5,601,990 issued February 11, 1997, U.S. Patent Number 6,060,037 issued April 26, 2000, U.S. Patent Number 5,962,220 issued October 5, 1999, and U.S. Patent Number 5,879,656 issued March 9, 1999, and U.S. Patent Application Serial Number 09/180,237 filed March 12, 1997.

WO 98/09510 discloses a genetically manipulated live animal model comprising a mutation in a CDX2 *Drosophila* caudal gene homologue. Mallo et al. (1997), Int. J. Cancer (Pred. Oncol.): 74, 35-44 reports that a decrease in human CDX1 and/or CDX2 expression is associated with colorectal tumourigenesis.

There is a need for reagents, kits and methods for screening, diagnosing and monitoring individuals with cancer originating from the alimentary canal, particularly primary and metastatic stomach and esophageal cancer and metastatic colorectal cancer. There is a need for reagents, kits and methods for identifying and confirming that a cancer of unknown origin is originating from the alimentary canal and for analyzing tissue and cancer samples to identify and confirm cancer originating from the alimentary canal and to determine the level of migration of such cancer cells. There is a need for compositions which can specifically target colorectal, stomach and esophageal cancer cells. There is a need for imaging agents which can specifically bind to colorectal, stomach and esophageal cancer cells. There is a need for improved methods of imaging colorectal, stomach and esophageal cancer cells. There is a need for therapeutic agents which can specifically bind to colorectal, stomach and esophageal cancer cells. There is a need for improved methods of treating individuals who are suspected of suffering from primary and/or metastatic stomach or esophageal cancer or metastatic colorectal cancer. There is a need for vaccine composition to treat colorectal, stomach and esophageal cancer. There is a need for vaccine composition to treat and prevent metastasized colorectal, stomach and esophageal cancer. There is a need for therapeutic agents which can specifically deliver gene therapeutics, antisense compounds and other drugs to colorectal, stomach and esophageal cancer cells.

### SUMMARY OF THE INVENTION

The invention further relates *to in vitro* methods of determining whether or not an individual has metastatic colorectal cancer. The present invention relates to *in vitro* methods of examining samples of non-colorectal tissue and body fluids from an individual to determine whether or not CDX2, which is expressed by normal colon cells and by colorectal, tumor cells, is being expressed by cells in samples other than colon. The presence of CDX2 protein or of CDX2 gene transcript in samples outside the colorectal track is indicative of expression of CDX2 and is evidence that the individual may be suffering from metastasized colon cancer. In patients suspected of suffering from colorectal cancer, the presence of one of more of SI, CDX1 or CDX2 protein or of one of more of SI, CDX1 or CDX2 gene transcript in samples outside the colorectal track is supportive of the conclusion that the individual is suffering from metastatic colorectal cancer. The diagnosis of metastatic colorectal cancer may be made or confirmed. In patients suspected of suffering from stomach or esophageal cancer, the presence of one of more of SI, CDX1 or CDX2 protein or of one of more of SI, CDX1 or CDX2 gene transcript in samples outside the colorectal track is supportive of the conclusion that the individual is suffering from primary and/or metastatic stomach or esophageal cancer. The diagnosis of primary and/or metastatic stomach or esophageal cancer may be made or confirmed.

The description further discloses in vitro methods of determining whether or not tumor cells are colorectal, stomach or esophageal in origin. *In vitro* methods of diagnosing whether or not an individual suffering from cancer is suffering from colorectal, stomach or esophageal cancer are disclosed. *In vitro* methods of examining samples of tumors from an individual to determine whether or not one of more of SI, CDX1 or CDX2 protein, which is expressed by colorectal, stomach or esophageal tumor cells, is being expressed by the tumor cells are disclosed. The presence of one of more of SI, CDX1 or CDX2 protein or of one of more of SI, CDX1 or CDX2 gene transcript is indicative of expression of one of more of SI, CDX1 or CDX2 and evidence that the individual may be suffering from colorectal, stomach or esophageal cancer. In tumors which are suspected of being colorectal, stomach or esophageal tumors, the presence of one of more of SI, CDX1 or CDX2 protein or of one of more of SI, CDX1 or CDX2 gene transcript supports the conclusion that the tumors are of colorectal, stomach or esophageal cancer and the diagnosis of colorectal, stomach or esophageal cancer.

*In vitro* kits for practicing the methods of the invention and to reagents and compositions useful as components in such *in vitro* kits of the invention is disclosed.

The description discloses a method of imaging primary and metastatic stomach and esophageal tumors and metastatic colorectal tumors and to methods of treating an individual suspected of suffering from primary and metastatic stomach and esophageal tumors and metastatic colorectal tumors comprising the steps of administering to said individual a pharmaceutical compositions according to the invention, wherein the compositions or conjugated compounds are present in an amount effective for therapeutic or diagnostic use in humans suffering from primary and/or metastatic stomach or esophageal tumors and metastatic colorectal tumors cancer.

Further is disclosed a method of delivering an active agent to primary and metastatic stomach and esophageal tumor cells and metastatic colorectal tumors cells comprising the steps of administering to an individual who has primary and/or metastatic stomach or esophageal tumors or metastatic colorectal cancer, a pharmaceutical composition comprising a pharmaceutically acceptable carrier or diluent, and an unconjugated compositions that comprises a liposome that includes SI ligands on its surface and an active component encapsulated therein.

The description further relates to killed or inactivated colorectal, stomach or esophageal tumor cells that comprise a protein comprising at least one epitope of one of more of SI, CDX1 or CDX2 protein; and to vaccines comprising the same. In some embodiments, the killed or inactivated cells or particles comprise one of more of SI, CDX1 or CDX2 protein. In some embodiments, the killed or inactivated cells or particles are haptenized.

The description further relates to methods of treating individuals suffering from colorectal, stomach or esophageal cancer and to methods of treating individuals susceptible colorectal, stomach or esophageal cancer. The method provides administering to such individuals an effective amount of such vaccines. The description further relates to the use of such vaccines as immunotherapeutics.

The present description discloses a method for the isolation of tissue-specific molecular markers that are useful in the diagnosis of metastatic cancer. A method to identify molecular markers useful for detecting tumor cells that have metastasized from an origin tissue to a destination tissue or fluid is disclosed. The method comprises the steps of down-regulating in a population of origin tissue cells the activity of a transcription factor associated with terminal differentiation in the origin tissue, comparing an expression profile of the population of down-regulated origin cells with an expression profile of a population of control origin cells, identifying candidate markers which are expressed in the population of control origin cells but not the population of down-regulated origin cells, and comparing expression of the candidate markers in populations of control origin cells, cancerous origin cells and destination cells, wherein a candidate marker which is expressed in population of control origin cells and cancerous origin cells, but not the population of destination cells is a useful marker for the detection of cancer metastasized from the origin tissue to the destination tissue. The method may comprise the additional step of isolating the molecular marker. The method may also comprise the additional steps of identifying the transcription factor that binds to regulatory regions of a gene associated with terminal differentiation of the origin tissue.

### BRIEF DESCRIPTION OF THE DRAWINGS

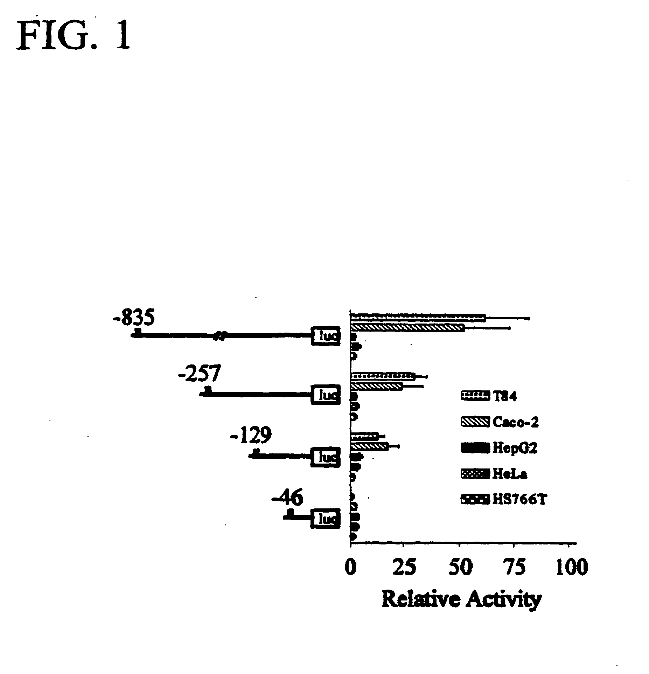

**Figure 1**. Functional characterization of deletion mutants of the human GC-C gene promoter. Deletion mutants of the GC-C gene 5'-flanking region were linked to luciferase and co-transfected with the *Renilla* luciferase control plasmid pRL-TK into intestinal (T84, Caco2) and extra-intestinal (HepG2, HeLa, HS766T) cell lines. Data are expressed as luciferase activity relative to the pGL3 Basic promoterless construct (Relative Activity). Each bar represents the mean ± the standard error of at least 3 independent transfections performed in duplicate.

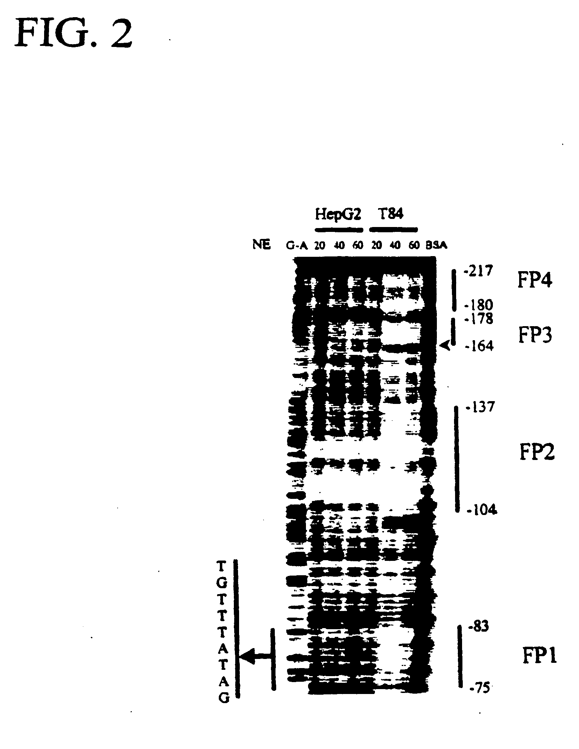

**Figure 2**. DNAse I protection of the proximal human GC-C promoter. Footprinting reactions included the indicated mg quantities (NE) of HepG2 or T84 nuclear extract and the -46 to -257 promoter fragment labeled at the 5'-end of the coding strand. A control digestion contained 60 mg of bovine serum albumin (BSA). Protected bases were identified by a Maxam-Gilbert sequencing reaction (G + A) of the labeled fragment. The sequence of FP1 is given. Arrowhead indicates DNAse I hypersensitivity site at base -163.

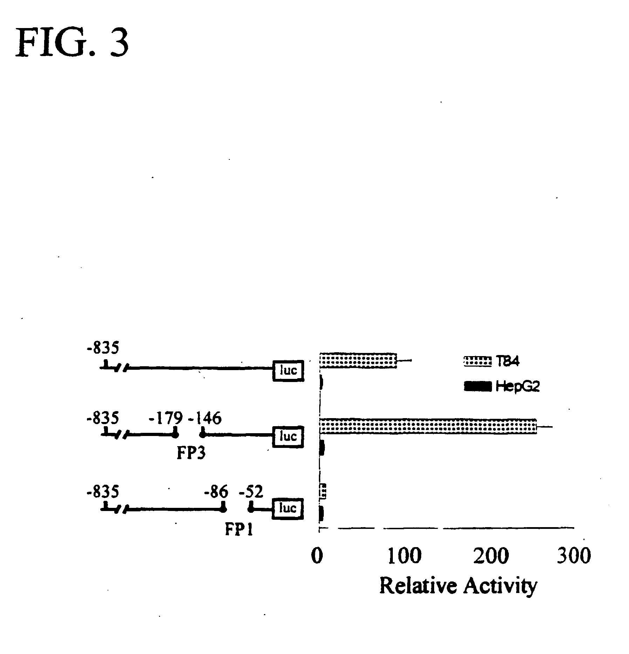

**Figure 3**. Regulation of reporter gene expression by intestine-specific protected elements. FP1 and FP3 were deleted from the -835 luciferase construct by *in vitro* mutagenesis, and wild-type and deletion constructs were expressed in HepG2 and T84 cells. Results are expressed as luciferase activity relative to a promoterless construct and represent the mean ± the standard error of 3 independent transfections performed in duplicate.

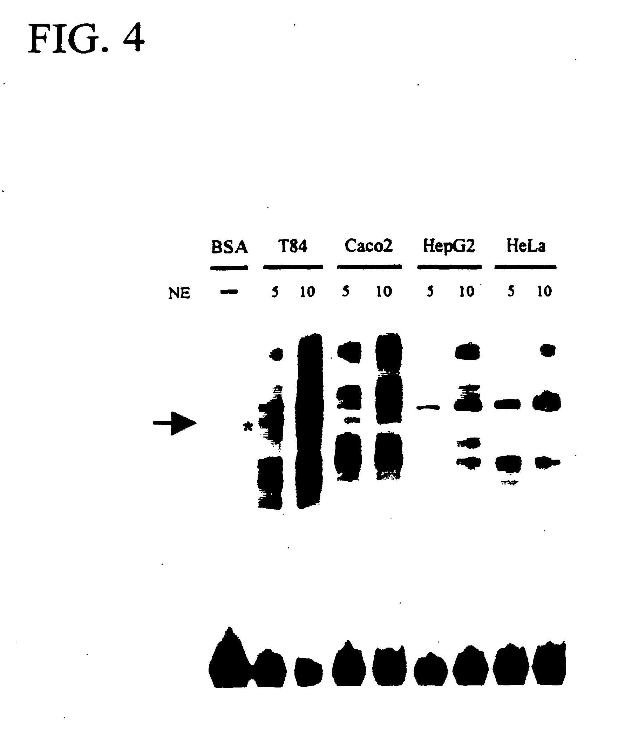

**Figure 4****.** Intestinal specificity of FP1 probe EMSA. Nuclear extracts from intestinal or extra-intestinal cells, or BSA (10 mg), were incubated with labeled FP1 for 30 min. at room temp prior to separation on a non-denaturing 6% polyacrylamide gel.

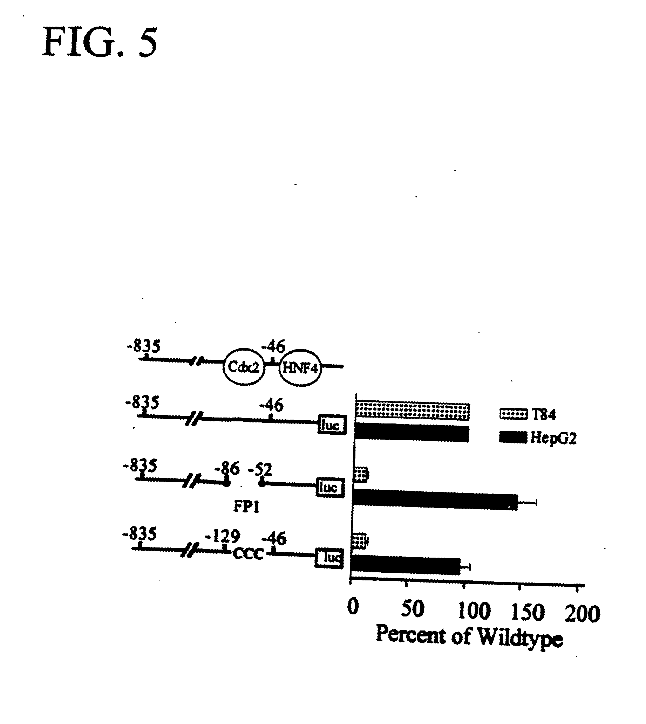

**Figure 5**. Cdx2 binding element FP1 is required for GC-C reporter gene activation. Putative binding sites for Cdx2 and HNF-4a are indicated on the -835 construct. T84 and HepG2 cells were transfected with the -835 reporter construct from which FP1 was deleted, or that construct containing the 'CCC' mutation. Results are expressed as (luciferase activity of mutant construct , luciferase activity of wild type construct) x 100, and represent the mean ± the standard error of 3 independent transfections performed in duplicate. The values expressed as relative luciferase activities are, respectively, (wildtype; FP1 deletion; 'CCC' mutation): T84 (16.2±2.7; 1.9±0.3; 2.3±0.1) and HepG2 (2.1±0.1; 2.9±0.3; 2.2±0.1).

### DESCRIPTION OF PREFERRED EMBODIMENTS

### Definitions

As used herein, the term "SI" is meant to refer to the cellular protein also known as sucrase isomaltase which is expressed by normal colorectal cells, as well as primary and metastasized colorectal, stomach and esophageal cancer cells.

As used herein, the term "CDX1" is meant to refer to the cellular protein CDX1 which is expressed by normal colorectal cells, as well as primary and metastasized colorectal, stomach and esophageal cancer cells.

As used herein, the term "CDX2" is meant to refer to the cellular protein CDX2 which is expressed by normal colorectal cells, as well as primary and metastasized colorectal, stomach and esophageal cancer cells.

As used herein, the term "functional fragment" as used in the term "functional fragment of one of more of SI, CDX1 or CDX2 gene transcript" is meant to refer to fragments of SI, CDX1 or CDX2 gene transcript which are functional with respect to nucleic acid molecules with full length sequences. For example, a functional fragment may be useful as an oligonucleotide or nucleic acid probe, a primer, an antisense oligonucleotide or nucleic acid molecule or a coding sequence.

The nucleotide sequence encoding human SI protein is disclosed in Chantret, I et al. Ann. Hum. Genet. 52 (Pt 1), 57-61 (1988) and GenBank Accession No. NM 001041.

The amino acid of the CDX1 protein and the nucleotide sequence of the CDX1 gene transcript is set forth in GenBank Accession No. XM 003791.

The amino acid of the CDX2 protein and the nucleotide sequence of the CDX2 gene transcript is set forth in Mallo, G.V. et al. 1991 Intl. J. Cancer 74(1):35-44 and GenBank Accession No. U51096.

As used herein, the term "functional fragment" as used in the term "functional fragment of SI, CDX1 or CDX2 protein" is meant to fragments of SI, CDX1 or CDX2 protein which function in the same manner as SI, CDX1 or CDX2 protein with full length sequences. For example, an immunogenically functional fragment of a SI protein comprises an epitope recognized by an anti-SI antibody. A ligand-binding functional fragment of SI comprises a sequence which forms a structure that can bind to a ligand which recognizes and binds to SI protein.

As used herein, the term "epitope recognized by an anti-SI protein antibody" refers to those epitopes specifically recognized by an anti-SI protein antibody.

As used herein, the term "epitope recognized by an anti-CDX1 protein antibody" refers to those epitopes specifically recognized by an anti-CDX1 protein antibody.

As used herein, the term "epitope recognized by an anti-CDX2 protein antibody" refers to those epitopes specifically recognized by an anti-CDX2 protein antibody.

As used herein, the term "antibody" is meant to refer to complete, intact antibodies, and Fab fragments and F(ab)₂ fragments thereof. Complete, intact antibodies include monoclonal antibodies such as murine monoclonal antibodies, chimeric antibodies and humanized antibodies.

As used herein, the term "SI ligand" is meant to refer to compounds which specifically bind to a SI protein. Antibodies that bind to SI are SI ligands. A SI ligand may be a protein, peptide or a non-peptide.

As used herein, the term "active agent" is meant to refer to compounds that are therapeutic agents or imaging agents.

As used herein, the term "radiostable" is meant to refer to compounds which do not undergo radioactive decay; i.e. compounds which are not radioactive.

As used herein, the term "therapeutic agent" is meant to refer to chemotherapeutics, toxins, radiotherapeutics, targeting agents or radiosensitizing agents.

As used herein, the term "chemotherapeutic" is meant to refer to compounds that, when contacted with and/or incorporated into a cell, produce an effect on the cell including causing the death of the cell, inhibiting cell division or inducing differentiation.

As used herein, the term "toxin" is meant to refer to compounds that, when contacted with and/or incorporated into a cell, produce the death of the cell.

As used herein, the term "radiotherapeutic" is meant to refer to radionuclides which when contacted with and/or incorporated into a cell, produce the death of the cell.

As used herein, the term "targeting agent" is meant to refer compounds which can be bound by and or react with other compounds. Targeting agents may be used to deliver chemotherapeutics, toxins, enzymes, radiotherapeutics, antibodies or imaging agents to cells that have targeting agents associated with them and/or to convert or otherwise transform or enhance co-administered active agents. A targeting agent may include a moiety that constitutes a first agent that is localized to the cell which when contacted with a second agent either is converted to a third agent which has a desired activity or causes the conversion of the second agent into an agent with a desired activity. The result is the localized agent facilitates exposure of an agent with a desired activity to the cancer cell.

As used herein, the term "radiosensitizing agent" is meant to refer to agents which increase the susceptibility of cells to the damaging effects of ionizing radiation. A radiosensitizing agent permits lower doses of radiation to be administered and still provide a therapeutically effective dose.

As used herein, the term "imaging agent" is meant to refer to compounds which can be detected.

As used herein, the term "SI binding moiety" is meant to refer to the portion of a conjugated compound that constitutes an SI ligand.

As used herein, the term "active moiety" is meant to refer to the portion of a conjugated compound that constitutes an active agent.

As used herein, the terms "conjugated compound" and "conjugated composition" are used interchangeably and meant to refer to a compound which comprises a SI binding moiety and an active moiety and which is capable of binding to SI. Conjugated compounds according to the present invention comprise a portion which constitutes an SI ligand and a portion which constitutes an active agent. Thus, conjugated compounds according to the present invention are capable of specifically binding to the SI and include a portion which is a therapeutic agent or imaging agent. Conjugated compositions may comprise crosslinkers and/or molecules that serve as spacers between the moieties.

As used herein, the terms "crosslinker", "crosslinking agent", "conjugating agent", "coupling agent", "condensation reagent" and "bifunctional crosslinker" are used interchangeably and are meant to refer to molecular groups which are used to attach the SI ligand and the active agent to thus form the conjugated compound.

As used herein, the term "colorectal cancer" is meant to include the well-accepted medical definition that defmes colorectal cancer as a medical condition characterized by cancer of cells of the intestinal tract below the small intestine (i.e. the large intestine (colon), including the cecum, ascending colon, transverse colon, descending colon, and sigmoid colon, and rectum). Additionally, as used herein, the term "colorectal cancer" is meant to further include medical conditions which are characterized by cancer of cells of the duodenum and small intestine (jejunum and ileum). The definition of colorectal cancer used herein is more expansive than the common medical definition but is provided as such since the cells of the duodenum and small intestine also contain SI, CDX1 and CDX2.

As used herein, the term "stomach cancer" is meant to include the well-accepted medical definition that defines stomach cancer as a medical condition characterized by cancer of cells of the stomach.

As used herein, the term "esophageal cancer" is meant to include the well-accepted medical definition that defines esophageal cancer as a medial condition characterized by cancer of cells of the esophagus.

As used herein, the term "metastasis" is meant to refer to the process in which cancer cells originating in one organ or part of the body relocate to another part of the body and continue to replicate. Metastasized cells subsequently form tumors which may further metastasize. Metastasis thus refers to the spread of cancer from the part of the body where it originally occurs to other parts of the body.

As used herein, the term "metastasized colorectal cancer cells" is meant to refer to colorectal cancer cells which have metastasized. Metastasized colorectal cancer cells localized in a part of the body other than the duodenum, small intestine (jejunum and ileum), large intestine (colon), including the cecum, ascending colon, transverse colon, descending colon, and sigmoid colon, and rectum.

As used herein, the term "metastasized stomach cancer cells" is meant to refer to stomach cancer cells which have metastasized. Metastasized stomach cancer cells localized in a part of the body other than the stomach.

As used herein, the term "metastasized esophageal cancer cells" is is meant to refer to colorectal cancer cells which have metastasized. Metastasized esophageal cancer cells localized in a part of the body other than the esophagus.

As used herein, the term "non-colorectal sample" and "extra-intestinal sample" are used interchangeably and meant to refer to a sample of tissue or body fluid from a source other than colorectal tissue. In some preferred embodiments, the non-colorectal sample is a sample of tissue such as lymph nodes. In some preferred embodiments, the non-colorectal sample is a sample of extra-intestinal tissue which is an adenocarcinoma of unconfirmed origin. In some preferred embodiments, the non-colorectal sample is a blood sample.

As used herein, "an individual suffering from an adenocarcinoma of unconfirmed origin" is meant to refer to an individual who has a tumor in which the origin has not been definitively identified.

As used herein, "an individual is suspected of being susceptible to colorectal, stomach or esophageal cancer" is meant to refer to an individual who is at a particular risk of developing colorectal, stomach or esophageal cancer. Examples of individuals at a particular risk of developing colorectal, stomach or esophageal cancer are those whose family medical history indicates above average incidence of colorectal, stomach or esophageal cancer among family members and/or those who have already developed colorectal, stomach or esophageal cancer and have been effectively treated who therefore face a risk of relapse and recurrence.

As used herein, the term "antisense composition" and "antisense molecules" are used interchangeably and are meant to refer to compounds that regulate transcription or translation by hybridizing to DNA or RNA and inhibiting and/or preventing transcription or translation from taking place. Antisense molecules include nucleic acid molecules and derivatives and analogs thereof. Antisense molecules hybridize to DNA or RNA in the same manner as complementary nucleotide sequences do regardless of whether or not the antisense molecule is a nucleic acid molecule or a derivative or analog. Antisense molecules may inhibit or prevent transcription or translation of genes whose expression is linked to cancer.

As used herein, the term "SI immunogen" is meant to refer to SI protein or a fragment thereof or a protein that comprises the same or a haptenized product thereof, cells and particles which display at least one SI epitope, and haptenized cells and haptenized particles which display at least one SI epitope.

As used herein, the term "CDX1 immunogen" is meant to refer to CDX1 protein or a fragment thereof or a protein that comprises the same or a haptenized product thereof, cells and particles which display at least one CDX1 epitope, and haptenized cells and haptenized particles which display at least one CDX1 epitope.

As used herein, the term "CDX2 immunogen" is meant to refer to CDX2 protein or a fragment thereof or a protein that comprises the same or a haptenized product thereof, cells and particles which display at least one CDX2 epitope, and haptenized cells and haptenized particles which display at least one CDX21 epitope.

As used herein, the term "recombinant expression vector" is meant to refer to a plasmid, phage, viral particle or other vector which, when introduced into an appropriate host, contains the necessary genetic elements to direct expression of the coding sequence that encodes the protein. The coding sequence is operably linked to the necessary regulatory sequences. Expression vectors are well known and readily available. Examples of expression vectors include plasmids, phages, viral vectors and other nucleic acid molecules or nucleic acid molecule containing vehicles useful to transform host cells and facilitate expression of coding sequences.

As used herein, the term "illegitimate transcription" is meant to refer to the low level or background expression of tissue-specific genes in cells from other tissues. The phenomenon of illegitimate transcription thus provides copies of mRNA for a tissue specific transcript in other tissues. If detection techniques used to detect gene expression are sufficiently sensitive to detect illegitimate transcription, the expression level of the transcript in negative samples due to illegitimate transcription must be discounted using controls and/or quantitative assays and/or other means to eliminate the incidence of false positive due to illegitimate transcription. Alternatively, detection of evidence of one of more of SI, CDX1 or CDX2 gene expression in sample is achieved without detecting one of more of SI, CDX1 or CDX2 gene transcript present due to illegitimate transcription. This is accomplished using techniques which are not sufficiently sensitive to detect the one of more of SI, CDX1 or CDX2 gene transcript present due to illegitimate transcription which is present as background.

### SI

Carcinomas derived from the colorectal cells, stomach or esophagus express SI, CDX1 and CDX2. The expression of SI, CDX1 and CDX2 by such tumors enables this protein and its mRNA to be a specific biomarker for the presence of cancer cells in extra-intestinal tissues and blood. Indeed, this characteristic permits the detection of SI, CDX1 or CDX2 mRNA by RT-PCR analysis to be a diagnostic test to stage patients with colorectal, stomach or esophageal cancer and follow patients after surgery for evidence of recurrent disease in their blood as well as to detect colorectal, stomach and esophageal cancers. Further, the SI may be targeted with a ligand conjugated to an active agent in order to deliver the active agent to tumor cells *in vivo.*

U.S. Patent No. 5,518,888 issued May 21,1996 to Waldman, PCT application PCT/US94/12232 filed October 26, 1994, U.S. Application Serial No. 08/467,920 filed June 6, 1995, and U.S. Application Serial No. 08/583,447 filed January 5, 1996, disclose that metastasized colorectal tumors can be targeted for delivery of active compounds by targeting ST receptors (also referred to as guanylin cyclase C or GCC). The presence of ST receptors on cells outside of the intestinal tract as a marker for colorectal cancer allows for the screening, identification and treatment of individuals with metastasized colorectal tumors. ST receptors may also be used to target delivery of gene therapeutics and antisense compounds to colorectal cells.

U.S. Patent No. 5,601,990 issued February 11, 1997 to Waldman, PCT application PCT/US94/12232 filed October 26, 1994, and PCT application PCT/US97/07467 filed May 2, 1997, disclose that detection of evidence of expression of ST receptors in samples of tissue and body fluid from outside the intestinal track indicate metastasized colorectal cancer.

PCT application PCT/US97/07565 filed May 2, 1997, disclose that immunogens with epitopes that can be targeted by antibodies that react with ST receptors can be used in vaccines compositions useful as prophylactic and therapeutic anti-metastatic colorectal cancer compositions.

It has been discovered that in addition to normal colon cells, primary and metastasized colon, stomach and esophageal carcinoma cells express SI, CDX1 and CDX2. Normal stomach and esophageal cells do not express SI, CDX1 and CDX2. Thus, the present invention provides the use of SI, CDX1 and CDX2 as a specific molecular diagnostic marker for the diagnosis, staging, and post-operative surveillance of patients with metastasized colon cancer and primary and metastasized stomach and esophageal cancer.

Detection of the expression of SI, CDX1 and CDX2. employing molecular techniques, including, but not limited to, RT-PCR, can be employed to diagnose and stage patients, follow the development of recurrence after surgery and/or remission, and, potentially, screen normal people for the development of colorectal, stomach or esophageal cancer.

SI, CDX1 and CDX2 are unique in that they are only expressed in normal intestinal cells. Mucosal cells lining the intestine are joined together by tight junctions which form a barrier against the passage of intestinal contents into the blood stream and components of the blood stream into the intestinal lumen. Therefore, the apical location of cells expressing SI results in the isolation of such cells from the circulatory system so that they may be considered to exist separate from the rest of the body; essentially the "outside" of the body. Therefore, the rest of the body is considered "outside" the intestinal tract. Compositions administered "outside" the intestinal tract are maintained apart and segregated from the only cells which normally express SI, CDX1 and CDX2. Conversely, tissue samples taken from tissue outside of the intestinal tract do not normally contain cells which express SI, CDX1 and CDX2.

In individuals suffering from colorectal cancer, the cancer cells are often derived from cells that produce and display the SI, CDX1 and CDX2 and these cancer cells continue to produce SI, CDX1 and CDX2. It has been observed that SI, CDX1 and CDX2 are expressed by colorectal cancer cells. Likewise, SI, CDX1 and CDX2 are expressed by stomach and esophageal cancer cells.

The expression of SI, CDX1 and CDX2 by colorectal tumor cells provides a detectable target for *in vitro* screening, monitoring and staging as well as a target for *in vivo* delivery of conjugated compositions that comprise active agents for the imaging and treatment. SI, CDX1 and CDX2 can also serve as targets for vaccines which may be used to protect against metastasized colorectal cancer or to treat individiuals with metastasized colorectal cancer.

The expression of SI, CDX1 and CDX2 by stomach and esophageal tumor cells provides a detectable target for *in vitro* screening, monitoring and staging as well as a target for *in vivo* delivery of conjugated compositions that comprise active agents for the imaging and treatment. SI, CDX1 and CDX2 can also serve as targets for vaccines which may be used to protect against primary and metastatic stomach and esophageal cancer or to treat individiuals with primary and metastatic stomach and esophageal cancer.

### In vitro Diagnostics

According to some embodiments of the invention, compositions, kits and *in vitro* methods are provided for screening, diagnosing and analyzing patients and patient samples to detect evidence of one or more of SI, CDX1 and CDX2 expression by cells outside of the intestinal tract wherein the expression of SI may be suggestive of metastasized colorectal cancer or primary or metastatic stomach or esophageal cancer. In patients suspected of having metastasized colorectal cancer or primary or metastatic stomach or esophageal cancer evidence of one or more of SI, CDX1 and CDX2 expression by cells outside of the intestinal tract is indicative of metastasized colorectal cancer or primary or metastatic stomach or esophageal cancer and can be used in the diagnosis, monitoring and staging of such patients. Furthermore, the present invention relates to methods, compositions and kits useful in the *in vitro* screening, and analysis of patient and patient samples to detect evidence of one or more of SI, CDX1 and CDX2 expression by tumor cells outside of the intestinal tract wherein the presence of cells that express one or more of SI, CDX1 and CDX2 suggests or confirms that a tumor is of colorectal or stomach or esophageal cancer origin. In an additional aspect of the invention, compositions, kits and methods are provided which are useful to visualize metastasized colorectal cancer or primary or metastatic stomach or esophageal cancer cells.

*In vitro* screening and diagnostic compositions, methods and kits can be used in the monitoring of individuals who are in high risk groups for colorectal, stomach or esophageal cancer such as those who have been diagnosed with localized disease and/or metastasized disease and/or those who are genetically linked to the disease. *In vitro* screening and diagnostic compositions, methods and kits can be used in the monitoring of individuals who are undergoing and/or have been treated for primary colorectal, stomach or esophageal cancer to determine if the cancer has metastasized. *In vitro* screening and diagnostic compositions, methods and kits can be used in the monitoring of individuals who are undergoing and/or have been treated for colorectal, stomach or esophageal cancer to determine if the cancer has been eliminated. *In vitro* screening and diagnostic compositions, methods and kits can be used in the monitoring of individuals who are otherwise susceptible, i.e. individuals who have been identified as genetically predisposed such as by genetic screening and/or family histories. Advancements in the understanding of genetics and developments in technology as well as epidemiology allow for the determination of probability and risk assessment an individual has for developing stomach or esophageal cancer. Using family health histories and/or genetic screening, it is possible to estimate the probability that a particular individual has for developing certain types of cancer including colorectal, stomach or esophageal cancer. Those individuals that have been identified as being predisposed to developing a particular form of cancer can be monitored or screened to detect evidence of colorectal, stomach or esophageal cancer. Upon discovery of such evidence, early treatment can be undertaken to combat the disease. Accordingly, individuals who are at risk for developing colorectal, stomach or esophageal cancer may be identified and samples may be isolated form such individuals. The invention is particularly useful for monitoring individuals who have been identified as having family medical histories which include relatives who have suffered from colorectal, stomach or esophageal cancer. Likewise, the invention is particularly useful to monitor individuals who have been diagnosed as having colorectal, stomach or esophageal cancer and, particularly those who have been treated and had tumors removed and/or are otherwise experiencing remission including those who have been treated for colorectal, stomach or esophageal cancer.

*In vitro* screening and diagnostic compositions, methods and kits can be used in the analysis of tumors. Expression of one or more of SI, CDX1 and CDX2 as markers for cell type and suggests the origin of adenocarcinoma of unconfirmed origin may be colorectal, stomach or esophageal tumors. Detection of one or more of SI, CDX1 and CDX2 expression can also be used to assist in an initial diagnosis of colorectal, stomach or esophageal cancer or to confirm such diagnosis. Tumors believed to be colorectal, stomach or esophageal in origin can be confirmed as such using the compositions, methods and kits of the invention.

*In vitro* screening and diagnostic compositions, kits and methods of the invention can be used to analyze tissue samples from the stomach or esophagus to identify primary stomach or esophageal cancer.

*In vitro* screening and diagnostic compositions, kits and methods of the invention can be used to analyze tissue samples from the colon to detect the amount of invasion by primary colorectal cancer into the intestinal tissue.

According to the invention, compounds are provided which bind to SI, CDX1 or CDX2 SI gene transcript or protein. Normal tissue in the body does not have SI, CDX1 and CDX2 transcript or protein except cells of the intestinal tract. The expression of SI, CDX1 and CDX2 as markers for cell type and is useful in the identification of colorectal, stomach or esophageal cancer in extra-intestinal samples.

In some embodiments of the invention, non-colorectal tissue and fluid samples or tumor samples may be screened to identify the presence or absence of one or more of SI, CDX1 and CDX2 protein. Techniques such as ELISA assays and Western blots may be performed to determine whether one or more of SI, CDX1 and CDX2 is present in a sample.

In some embodiments of the invention, non-colorectal tissue and fluid samples or tumor samples may be screened to identify whether one or more of SI, CDX1 and CDX2 are being expressed in cells outside of the colorectal tract by detecting the presence or absence of SI gene transcript. The presence of one or more of SI, CDX1 and CDX2 gene transcript or cDNA generated therefrom can be determined using techniques such as PCR amplification, branched oligonucleotide technology, Northern Blots (mRNA), Southern Blots (cDNA), or oligonucleotide hybridization.

In some embodiments of the invention, cells of non-colorectal tissue samples or tumor samples may be examined to identify the presence or absence of one or more of SI, CDX1 and CDX2 proteins. Techniques such as immunohistochemistry blots may be performed on tissue sections to determine whether one or more of SI, CDX1 and CDX2 are present in a sample.

In some embodiments of the invention, cells of non-colorectal tissue samples or tumor samples may be examined to determine whether one or more of SI, CDX1 and CDX2 are being expressed in cells outside of the colorectal tract by detecting the presence or absence of the SI gene transcript. The presence of one or more of SI, CDX1 and CDX2 gene transcript or cDNA generated therefrom in cells from tissue sections can be determined using techniques such as *in situ* hybridization.

The presence of one or more of SI, CDX1 and CDX2 in non-colorectal tissue and fluid samples or on cells from non-colorectal tissue samples suggests possible stomach or esophageal cancer. The presence of one or more of SI, CDX1 and CDX2 in a tumor sample or on tumor cells suggests that the tumor may be colorectal, stomach or esophageal in origin. The presence of one or more of SI, CDX1 and CDX2 gene transcript in non-colorectal tissue and fluid samples or in cells from non-colorectal tissue samples suggests possible colorectal, stomach or esophageal cancer. The presence of one or more of SI, CDX1 and CDX2 gene transcript in tumor samples and tumor cells suggests that the tumor may be colorectal, stomach or esophageal in origin. Samples may be obtained from resected tissue or biopsy material including needle biopsy. Tissue section preparation for surgical pathology may be frozen and prepared using standard techniques. Immunohistochemistry and *in situ* hybridization binding assays on tissue sections are performed in fixed cells. Extra-intestinal samples may be homogenized by standard techniques such as sonication, mechanical disruption or chemical lysis such as detergent lysis. It is also contemplated that tumor samples in body fluids such as blood, urine, lymph fluid, cerebral spinal fluid, amniotic fluid, vaginal fluid, semen and stool samples may also be screened to determine if such tumors are colorectal, stomach or espophageal in origin.

Non-colorectal tissue samples may be obtained from any tissue except those of the colorectal tract, i.e. the intestinal tract below the small intestine (i.e. the large intestine (colon), including the cecum, ascending colon, transverse colon, descending colon, and sigmoid colon, and rectum) and additionally the duodenum and small intestine (jejunum and ileum). The normal cells of all tissue except those of the colorectal tract do not express SI, CDX1 and CDX2. Thus if SI, CDX1 and CDX2 protein or SI, CDX1 and CDX2 gene transcript are detected in non-colorectal samples, the possible presence of colorectal, stomach or esophageal cancer cells is suggested. In some preferred embodiments, the tissue samples are lymph nodes.

Tissue samples may be obtained by standard surgical techniques including use of biopsy needles. One skilled in the art would readily appreciate the variety of test samples that may be examined for one or more of SI, CDX1 and CDX2 and recognize methods of obtaining tissue samples.

Tissue samples may be homogenized or otherwise prepared for screening for the presence of SI by well known techniques such as sonication, mechanical disruption, chemical lysis such as detergent lysis or combinations thereof.

Examples of body fluid samples include blood, urine, lymph fluid, cerebral spinal fluid, amniotic fluid, vaginal fluid and semen. In some preferred embodiments, blood is used as a sample of body fluid. Cells may be isolated from fluid sample such as centrifugation. One skilled in the art would readily appreciate the variety of test samples that may be examined for one or more of SI, CDX1 and CDX2. Test samples may be obtained by such methods as withdrawing fluid with a syringe or by a swab. One skilled in the art would readily recognize other methods of obtaining test samples.

In an assay using a blood sample, the blood plasma may be separated from the blood cells. The blood plasma may be screened for one or more of SI, CDX1 and CDX2 including truncated proteins which are released into the blood when one or more of SI, CDX1 and CDX2 are cleaved from or sloughed off from tumor cells. In some embodiments, blood cell fractions are screened for the presence of colorectal, stomach or esophageal tumor cells. In some embodiments, lymphocytes present in the blood cell fraction are screened by lysing the cells and detecting the presence of one or more of SI, CDX1 and CDX2 protein or one or more of SI, CDX1 and CDX2 gene transcript which may be present as a result of the presence of any stomach or esophageal tumor cells that may have been engulfed by the blood cell. In some preferred embodiments, CD34+ cells are removed prior to isolation of mRNA from samples using commercially available immuno-columns.

Aspects of the present invention include various methods of determining whether a sample contains cells that express SI, CDX1 and CDX2 by nucleotide sequence-based molecular analysis to detect the SI, CDX1 and CDX2 gene transcript. Several different methods are available for doing so including those using Polymerase Chain Reaction (PCR) technology, branched oligonucleotide technology, Northern blot technology, oligonucleotide hybridization technology, and *in situ* hybridization technology.

The invention relates to oligonucleotide probes and primers used in the methods of identifying the SI, CDX1 and CDX2 gene transcript and to diagnostic kits which comprise such components.

The mRNA sequence-based methods for detect the SI gene transcript include but are not limited to polymerase chain reaction technology, branched oligonucleotide technology, Northern and Southern blot technology, *in situ* hybridization technology and oligonucleotide hybridization technology.

The methods described herein are meant to exemplify how the present invention may be practiced and are not meant to limit the scope of invention. It is contemplated that other sequence-based methodology for detecting the presence of SI, CDX1 and CDX2 gene transcript in non-colorectal samples may be employed according to the invention.

A preferred method to detectinggene transcript in genetic material derived from non-colorectal samples uses polymerase chain reaction (PCR) technology. PCR technology is practiced routinely by those having ordinary skill in the art and its uses in diagnostics are well known and accepted. Methods for practicing PCR technology are disclosed in "PCR Protocols: A Guide to Methods and Applications", Innis, M.A., et al. Eds. Academic Press, Inc. San Diego, CA (1990). Applications of PCR technology are disclosed in "Polymerase Chain Reaction" Erlich, H.A., et al., Eds. Cold Spring Harbor Press, Cold Spring Harbor, NY (1989). U.S. Patent Number 4,683,202, U.S. Patent Number 4,683,195, U.S. Patent Number 4,965,188 and U.S. Patent Numbers 5,075,216, describe methods of performing PCR. PCR may be routinely practiced using Perkin Elmer Cetus GENE AMP RNA PCR kit, Part No. N808-0017.

PCR technology allows for the rapid generation of multiple copies of DNA sequences by providing 5' and 3' primers that hybridize to sequences present in an RNA or DNA molecule, and further providing free nucleotides and an enzyme which fills in the complementary bases to the nucleotide sequence between the primers with the free nucleotides to produce a complementary strand of DNA. The enzyme will fill in the complementary sequences adjacent to the primers. If both the 5' primer and 3' primer hybridize to nucleotide sequences on the same small fragment of nucleic acid, exponential amplification of a specific double-stranded size product results. If only a single primer hybridizes to the nucleic acid fragment, linear amplification produces single-stranded products of variable length.

PCR primers can be designed routinely by those having ordinary skill in the art using sequence information. The nucleotide sequence of the SI gene transcript is set forth in SEQ ID NO: 1. The nucleotide sequence of the CDX1 gene transcript is set forth in SEQ ID NO:3. The nucleotide sequence of the CDX2 gene transcript is set forth in SEQ ID NO:5. To perform this method, RNA is extracted from cells in a sample and tested or used to make cDNA using well known methods and readily available starting materials. Those having ordinary skill in the art can readily prepare PCR primers. A set of primers generally contains two primers. When performing PCR on extracted mRNA or cDNA generated therefrom, if the SI gene transcript or cDNA generated therefrom is present, multiple copies of the mRNA or cDNA will be made. If it is not present, PCR will not generate a discrete detectable product. Primers are generally 8-50 nucleotides, preferably about 15-35 nucleotides, more preferably 18-28 nucleotides, which are identical or complementary to and therefor hybridize to the gene transcript or cDNA generated therefrom. In preferred embodiments, the primers are each 15-35 nucleotide, more preferably 18-28 nucleotide fragments The primer must hybridize to the sequence to be amplified. Typical primers are 18-28 nucleotides in length and are generally have 50% to 60% G+C composition. The entire primer is preferably complementary to the sequence it must hybridize to. Preferably, primers generate PCR products 100 base pairs to 2000 base pairs. However, it is possible to generate products of 50 to up to 10 kb and more. If mRNA is used as a template, the primers must hybridize to mRNA sequences. If cDNA is used as a template, the primers must hybridize to cDNA sequences.

The mRNA or cDNA is combined with the primers, free nucleotides and enzyme following standard PCR protocols. The mixture undergoes a series of temperature changes. If the gene transcript or cDNA generated therefrom is present, that is, if both primers hybridize to sequences on the same molecule, the molecule comprising the primers and the intervening complementary sequences will be exponentially amplified. The amplified DNA can be easily detected by a variety of well known means. If no gene transcript or cDNA generated therefrom is present, no PCR product will be exponentially amplified. The PCR technology therefore provides an extremely easy, straightforward and reliable method of detecting the gene transcript in a sample.

PCR product may be detected by several well known means. The preferred method for detecting the presence of amplified DNA is to separate the PCR reaction material by gel electrophoresis and stain the gel with ethidium bromide in order to visual the amplified DNA if present. A size standard of the expected size of the amplified DNA is preferably run on the gel as a control.

In some instances, such as when unusually small amounts of RNA are recovered and only small amounts of cDNA are generated therefrom, it is desirable or necessary to perform a PCR reaction on the first PCR reaction product. That is, if difficult to detect quantities of amplified DNA are produced by the first reaction, a second PCR can be performed to make multiple copies of DNA sequences of the first amplified DNA. A nested set of primers are used in the second PCR reaction. The nested set of primers hybridize to sequences downstream of the 5' primer and upstream of the 3' primer used in the first reaction.

The present invention includes oligonucleotide which are useful as primers for performing PCR methods to amplify the gene transcript or cDNA generated therefrom.

According to the invention, diagnostic kits can be assembled which are useful to practice methods of detecting the presence of the gene transcript or cDNA generated therefrom in non-colorectal samples. Such diagnostic kits comprise oligonucleotide which are useful as primers for performing PCR methods. It is preferred that diagnostic kits according to the present invention comprise a container comprising a size marker to be run as a standard on a gel used to detect the presence of amplified DNA. The size marker is the same size as the DNA generated by the primers in the presence of the gene transcript or cDNA generated therefrom. Additional components in some kits include instructions for carrying out the assay. Additionally the kit may optionally comprise depictions or photographs that represent the appearance of positive and negative results. Positive and negative controls may also be provided.

PCR assays are useful for detecting the gene transcript in homogenized tissue samples and cells in body fluid samples. It is contemplated that PCR on the plasma portion of a fluid sample could be used to detect the gene transcript.

Another method of determining whether a sample contains cells expressing SI, CDX1 or CDX2 by branched chain oligonucleotide hybridization analysis of mRNA extracted from a sample. Branched chain oligonucleotide hybridization may be performed as described in U.S. Patent Number 5,597,909, U.S. Patent Number 5,437,977 and U.S. Patent Number 5,430,138. Reagents may be designed following the teachings of those patents and that sequence of the gene transcript.

Another method of determining whether a sample contains cells expressing SI, CDX1 or CDX2 is by Northern Blot analysis of mRNA extracted from a non-colorectal sample. The techniques for performing Northern blot analyses are well known by those having ordinary skill in the art and are described in Sambrook, J. et al., (1989) Molecular Cloning: A Laboratory Manual, Cold Spring Harbor Laboratory Press, Cold Spring Harbor, NY. mRNA extraction, electrophoretic separation of the mRNA, blotting, probe preparation and hybridization are all well known techniques that can be routinely performed using readily available starting material.

The mRNA is extracted using poly dT columns and the material is separated by electrophoresis and, for example, transferred to nitrocellulose paper. Labeled probes made from an isolated specific fragment or fragments can be used to visualize the presence of a complementary fragment fixed to the paper. Probes useful to identify mRNA in a Northern Blot have a nucleotide sequence that is complementary to the gene transcript. Those having ordinary skill in the art could use the sequence information in the sequence listing herein to design such probes or to isolate and clone the gene transcript or cDNA generated therefrom to be used as a probe. Such probes are at least 15 nucleotides, preferably 30-200, more preferably 40-100 nucleotide fragments and may be the entire gene transcript.

According to the invention, diagnostic kits can be assembled which are useful to practice methods of detecting the presence of the gene transcript in non-colorectal samples by Northern blot analysis. Such diagnostic kits comprise oligonucleotide which are useful as probes for hybridizing to the mRNA. The probes may be radiolabeled. It is preferred that diagnostic kits according to the present invention comprise a container comprising a size marker to be run as a standard on a gel. It is preferred that diagnostic kits according to the present invention comprise a container comprising a positive control which will hybridize to the probe. Additional components in some kits include instructions for carrying out the assay. Additionally the kit may optionally comprise depictions or photographs that represent the appearance of positive and negative results.

Northern blot analysis is useful for detecting the gene transcript in homogenized tissue samples and cells in body fluid samples. It is contemplated that PCR on the plasma portion of a fluid sample could be used to detect the gene transcript.

Another method of detecting the presence of the gene transcript by oligonucleotide hybridization technology. Oligonucleotide hybridization technology is well known to those having ordinary skill in the art. Briefly, detectable probes which contain a specific nucleotide sequence that will hybridize to nucleotide sequence of the gene transcript. RNA or cDNA made from RNA from a sample is fixed, usually to filter paper or the like. The probes are added and maintained under conditions that permit hybridization only if the probes fully complement the fixed genetic material. The conditions are sufficiently stringent to wash off probes in which only a portion of the probe hybridizes to the fixed material. Detection of the probe on the washed filter indicate complementary sequences.

Probes useful in oligonucleotide assays at least 18 nucleotides of complementary DNA and may be as large as a complete complementary sequence to the gene transcript. In some preferred embodiments the probes of the invention are 30-200 nucleotides, preferably 40-100 nucleotides.

One having ordinary skill in the art, using the sequence information disclosed in the sequence listing can design probes useful in the invention. Hybridization conditions can be routinely optimized to minimize background signal by non-fully complementary hybridization. In some preferred embodiments, the probes are full length clones. Probes are at least 15 nucleotides, preferably 30-200, more preferably 40-100 nucleotide fragments and may be the entire gene transcript.

The present invention includes labeled oligonucleotide which are useful as probes for performing oligonucleotide hybridization. The labeled probes of the present invention are labeled with radiolabeled nucleotides or are otherwise detectable by readily available nonradioactive detection systems.

According to the invention, diagnostic kits can be assembled which are useful to practice oligonucleotide hybridization methods of the invention. Such diagnostic kits comprise a labeled oligonucleotide which encodes portions of the gene transcript. It is preferred that labeled probes of the oligonucleotide diagnostic kits according to the present invention are labeled with a radionucleotide. The oligonucleotide hybridization-based diagnostic kits according to the invention preferably comprise DNA samples that represent positive and negative controls. A positive control DNA sample is one that comprises a nucleic acid molecule which has a nucleotide sequence that is fully complementary to the probes of the kit such that the probes will hybridize to the molecule under assay conditions. A negative control DNA sample is one that comprises at least one nucleic acid molecule, the nucleotide sequence of which is partially complementary to the sequences of the probe of the kit. Under assay conditions, the probe will not hybridize to the negative control DNA sample. Additional components in some kits include instructions for carrying out the assay. Additionally the kit may optionally comprise depictions or photographs that represent the appearance of positive and negative results.

Oligonucleotide hybridization techniques are useful for detecting the gene transcript in homogenized tissue samples and cells in body fluid samples. It is contemplated that PCR on the plasma portion of a fluid sample could be used to detect the gene transcript.

The present invention relates to *in vitro* kits for evaluating samples of tumors to determine whether or not they are colorectal, stomach or esophageal in origin and to reagents and compositions useful to practice the same. In some embodiments of the invention, tumor samples may be isolated from individuals undergoing or recovery from surgery to remove tumors in the colorectal, stomach or esophagus, tumors in other organs or biopsy material. The tumor sample is analyzed to identify the presence or absence of the gene transcript. Techniques such as immunohistochemistry assays may be performed to determine whether SI, CDX1 and/or CDX2 are present in cells in the tumor sample. The presence of mRNA that encodes the protein or cDNA generated therefrom can be determined using techniques such as *in situ* hybridization, immunohistochemistry and *in situ* ST binding assay.

*In situ* hybridization technology is well known by those having ordinary skill in the art. Briefly, cells are fixed and detectable probes which contain a specific nucleotide sequence are added to the fixed cells. If the cells contain complementary nucleotide sequences, the probes, which can be detected, will hybridize to them.

Probes useful in oligonucleotide assays at least 18 nucleotides of complementary DNA and may be as large as a complete complementary sequence to the gene transcript. In some preferred embodiments the probes of the invention are 30-200 nucleotides, preferably 40-100 nucleotides.

One having ordinary skill in the art, using the sequence information set forth in sequence listing can design probes useful in *in situ* hybridization technology to identify cells that express SI, CDX1 or CDX2. Probes preferably hybridizes to a nucleotide sequence that corresponds to the gene transcript. Hybridization conditions can be routinely optimized to minimize background signal by non-fully complementary hybridization. Probes preferably hybridize to the full length gene transcript. Probes are at least 15 nucleotides, preferably 30-200, more preferably 40-100 nucleotide fragments and may be the gene transcript, more preferably 18-28 nucleotide fragments of the gene transcript.

The probes are fully complementary and do not hybridize well to partially complementary sequences. For *in situ* hybridization according to the invention, it is preferred that the probes are detectable by fluorescence. A common procedure is to label probe with biotin-modified nucleotide and then detect with fluorescently tagged avidin. Hence, probe does not itself have to be labeled with florescent but can be subsequently detected with florescent marker.

The present invention includes labeled oligonucleotide which are useful as probes for performing oligonucleotide hybridization. That is, they are fully complementary with mRNA sequences but not genomic sequences.The labeled probes of the present invention are labeled with radiolabeled nucleotides or are otherwise detectable by readily available nonradioactive detection systems.

The present invention relates to probes useful for *in situ* hybridization to identify cells that express SI, CDX1 or CDX2.

Cells are fixed and the probes are added to the genetic material. Probes will hybridize to the complementary nucleic acid sequences present in the sample. Using a fluorescent microscope, the probes can be visualized by their fluorescent markers.

According to the invention, diagnostic kits can be assembled which are useful to practice *in situ* hybridization methods of the invention are fully complementary with mRNA sequences but not genomic sequences. For example, the mRNA sequence includes different exon sequences. It is preferred that labeled probes of the *in situ* diagnostic kits according to the present invention are labeled with a fluorescent marker.

Immunohistochemistry techniques may be used to identify and essentially stain cells with SI, CDX1 or CDX2. Such "staining" allows for analysis of metastatic migration. Anti-SI antibodies such as those described above of contacted with fixed cells and the SI, CDX1 or CDX2 present in the cells reacts with the antibodies. The antibodies are detectably labeled or detected using labeled second antibody or protein A to stain the cells.

The techniques described herein for evaluating tumor sections can also be used to analyze tissue sections for samples of lymph nodes as well as other tissues to identify the presence of cells that express SI, CDX1 or CDX22. The samples can be prepared and "stained" to detect expression of SI, CDX1 or CDX2..

Immunoassay methods may be used in the diagnosis of individuals suffering from colorectal, stomach or esophageal cancer by detecting presence of SI, CDX1 or CDX2 in sample of non-colorectal tissue or body fluid from an individuals suspected of having or being susceptible to colorectal, stomach or esophageal cancer using antibodies which were produced in response to exposure to such SI, CDX1 or CDX2 protein. Moreover, immunoassay methods may be used to identify individuals suffering from colorectal, stomach or esophageal cancer by detecting presence of SI, CDX1 or CDX2 in sample of tumor using antibodies which were produced in response to exposure to such SI, CDX1 or CDX2 protein.

The antibodies are preferably monoclonal antibodies. The antibodies are preferably raised against SI, CDX1 or CDX2 made in human cells. Immunoassays are well known and there design may be routinely undertaken by those having ordinary skill in the art. Those having ordinary skill in the art can produce monoclonal antibodies which specifically bind to SI, CDX1 or CDX2 and are useful in methods and kits of the invention using standard techniques and readily available starting materials. The techniques for producing monoclonal antibodies are outlined in Harlow, E. and D. Lane, (1988) ANTIBODIES: A Laboratory Manual, Cold Spring Harbor Laboratory, Cold Spring Harbor NY, provide detailed guidance for the production of hybridomas and monoclonal antibodies which specifically bind to target proteins. It is within the scope of the present invention to include Fabs, recombinant Fabs, F(Ab)2s, recombinant F(Ab)2s which specifically bind to SI, CDX1 or CDX2 translation products in place of antibodies.

Briefly, SI, CDX1 or CDX2 protein is injected into mice. The spleen of the mouse is removed, the spleen cells are isolated and fused with immortalized mouse cells. The hybrid cells, or hybridomas, are cultured and those cells which secrete antibodies are selected. The antibodies are analyzed and, if found to specifically bind to the SI, CDX1 or CDX2, the hybridoma which produces them is cultured to produce a continuous supply of specific antibodies.

The antibodies are preferably monoclonal antibodies. The antibodies are preferably raised against SI, CDX1 or CDX2 made in human cells.

The means to detect the presence of a protein in a test sample are routine and one having ordinary skill in the art can detect the presence or absence of a protein or an antibody using well known methods. One well known method of detecting the presence of a protein is an immunoassay. One having ordinary skill in the art can readily appreciate the multitude of ways to practice an immunoassay to detect the presence of SI, CDX1 or CDX2 protein in a sample.

According to some embodiments, immunoassays comprise allowing proteins in the sample to bind a solid phase support such as a plastic surface. Detectable antibodies are then added which selectively binding to SI, CDX1 or CDX2. Detection of the detectable antibody indicates the presence of SI, CDX1 or CDX2. The detectable antibody may be a labeled or an unlabeled antibody. Unlabeled antibody may be detected using a second, labeled antibody that specifically binds to the first antibody or a second, unlabeled antibody which can be detected using labeled protein A, a protein that complexes with antibodies. Various immunoassay procedures are described in Immunoassays for the 80's, A. Voller et al., Eds., University Park, 1981.

Simple immunoassays may be performed in which a solid phase support is contacted with the test sample. Any proteins present in the test sample bind the solid phase support and can be detected by a specific, detectable antibody preparation. Such a technique is the essence of the dot blot, Western blot and other such similar assays.

Other immunoassays may be more complicated but actually provide excellent results. Typical and preferred immunometric assays include "forward" assays for the detection of a protein in which a first anti-protein antibody bound to a solid phase support is contacted with the test sample. After a suitable incubation period, the solid phase support is washed to remove unbound protein. A second, distinct anti-protein antibody is then added which is specific for a portion of the specific protein not recognized by the first antibody. The second antibody is preferably detectable. After a second incubation period to permit the detectable antibody to complex with the specific protein bound to the solid phase support through the first antibody, the solid phase support is washed a second time to remove the unbound detectable antibody. Alternatively, the second antibody may not be detectable. In this case, a third detectable antibody, which binds the second antibody is added to the system. This type of "forward sandwich" assay may be a simple yes/no assay to determine whether binding has occurred or may be made quantitative by comparing the amount of detectable antibody with that obtained in a control. Such "two-site" or "sandwich" assays are described by Wide, Radioimmune Assay Method, Kirkham, Ed., E. & S. Livingstone, Edinburgh, 1970, pp. 199-206.

Other types of immunometric assays are the so-called "simultaneous" and "reverse" assays. A simultaneous assay involves a single incubation step wherein the first antibody bound to the solid phase support, the second, detectable antibody and the test sample are added at the same time. After the incubation is completed, the solid phase support is washed to remove unbound proteins. The presence of detectable antibody associated with the solid support is then determined as it would be in a conventional "forward sandwich" assay. The simultaneous assay may also be adapted in a similar manner for the detection of antibodies in a test sample.

The "reverse" assay comprises the stepwise addition of a solution of detectable antibody to the test sample followed by an incubation period and the addition of antibody bound to a solid phase support after an additional incubation period. The solid phase support is washed in conventional fashion to remove unbound protein/antibody complexes and unreacted detectable antibody. The determination of detectable antibody associated with the solid phase support is then determined as in the "simultaneous" and "forward" assays. The reverse assay may also be adapted in a similar manner for the detection of antibodies in a test sample.

The first component of the immunometric assay may be added to nitrocellulose or other solid phase support which is capable of immobilizing proteins. The first component for determining the presence of SI, CDX1 or CDX2 in a test sample is an antibody. By "solid phase support" or "support" is intended any material capable of binding proteins. Well-known solid phase supports include glass, polystyrene, polypropylene, polyethylene, dextran, nylon, amylases, natural and modified celluloses, polyacrylamides, agaroses, and magnetite. The nature of the support can be either soluble to some extent or insoluble for the purposes of the present invention. The support configuration may be spherical, as in a bead, or cylindrical, as in the inside surface of a test tube or the external surface of a rod. Alternatively, the surface may be flat such as a sheet, test strip, etc. Those skilled in the art will know many other suitable "solid phase supports" for binding proteins or will be able to ascertain the same by use of routine experimentation. A preferred solid phase support is a 96-well microtiter plate.

To detect the presence of SI, CDX1 or CDX2, detectable antibodies are used. Several methods are well known for the detection of antibodies.

One method in which the antibodies can be detectably labeled is by linking the antibodies to an enzyme and subsequently using the antibodies in an enzyme immunoassay (EIA) or enzyme-linked immunosorbent assay (ELISA), such as a capture ELISA. The enzyme, when subsequently exposed to its substrate, reacts with the substrate and generates a chemical moiety which can be detected, for example, by spectrophotometric, fluorometric or visual means. Enzymes which can be used to detectably label antibodies include, but are not limited to malate dehydrogenase, staphylococcal nuclease, delta-5-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. One skilled in the art would readily recognize other enzymes which may also be used.

Another method in which antibodies can be detectably labeled is through radioactive isotopes and subsequent use in a radioimmunoassay (RIA) (see, for example, Work, T.S. et al., Laboratory Techniques and Biochemistry in Molecular Biology, North Holland Publishing Company, N.Y., 1978). The radioactive isotope can be detected by such means as the use of a gamma counter or a scintillation counter or by autoradiography. Isotopes which are particularly useful for the purpose of the present invention are ³H, ¹²⁵I, ¹³¹I, ³⁵S, and ¹⁴C. Preferably ¹²⁵I is the isotope. One skilled in the art would readily recognize other radioisotopes which may also be used.

It is also possible to label the antibody with a fluorescent compound. When the fluorescent-labeled antibody is exposed to light of the proper wave length, its presence can be detected due to its fluorescence. Among the most commonly used fluorescent labeling compounds are fluorescein isothiocyanate, rhodamine, phycoerythrin, phycocyanin, allophycocyanin, o-phthaldehyde and fluorescamine. One skilled in the art would readily recognize other fluorescent compounds which may also be used.

Antibodies can also be detectably labeled using fluorescence-emitting metals such as ¹⁵²Eu, or others of the lanthanide series. These metals can be attached to the protein-specific antibody using such metal chelating groups as diethylenetriaminepentaacetic acid (DTPA) or ethylenediamine-tetraacetic acid (EDTA). One skilled in the art would readily recognize other fluorescence-emitting metals as well as other metal chelating groups which may also be used.

Antibody can also be detectably labeled by coupling to a chemiluminescent compound. The presence of the chemiluminescent-labeled antibody is determined by detecting the presence of luminescence that arises during the course of a chemical reaction. Examples of particularly useful chemoluminescent labeling compounds are luminol, isoluminol, theromatic acridinium ester, imidazole, acridinium salt and oxalate ester. One skilled in the art would readily recognize other chemiluminescent compounds which may also be used.

Likewise, a bioluminescent compound may be used to label antibodies. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin. One skilled in the art would readily recognize other bioluminescent compounds which may also be used.

Detection of the protein-specific antibody, fragment or derivative may be accomplished by a scintillation counter if, for example, the detectable label is a radioactive gamma emitter. Alternatively, detection may be accomplished by a fluorometer if, for example, the label is a fluorescent material. In the case of an enzyme label, the detection can be accomplished by colorometric methods which employ a substrate for the enzyme. Detection may also be accomplished by visual comparison of the extent of enzymatic reaction of a substrate in comparison with similarly prepared standards. One skilled in the art would readily recognize other appropriate methods of detection which may also be used.

The binding activity of a given lot of antibodies may be determined according to well known methods. Those skilled in the art will be able to determine operative and optimal assay conditions for each determination by employing routine experimentation.

Positive and negative controls may be performed in which known amounts of proteins and no protein, respectively, are added to assays being performed in parallel with the test assay. One skilled in the art would have the necessary knowledge to perform the appropriate controls. In addition, the kit may comprise instructions for performing the assay. Additionally the kit may optionally comprise depictions or photographs that represent the appearance of positive and negative results.

SI, CDX1 or CDX2 may be produced as a reagent for positive controls routinely. One skilled in the art would appreciate the different manners in which the SI protein may be produced and isolated.

Antibody composition refers to the antibody or antibodies required for the detection of the protein. For example, the antibody composition used for the detection of SI, CDX1 or CDX2 in a test sample comprises a first antibody that binds to SI, CDX1 or CDX2 as well as a second or third detectable antibody that binds the first or second antibody, respectively.

To examine a test sample for the presence of SI, CDX1 or CDX2, a standard immunometric assay such as the one described below may be performed. A first antibody, which recognizes a specific portion of SI, CDX1 or CDX2, is added to a 96-well microtiter plate in a volume of buffer. The plate is incubated for a period of time sufficient for binding to occur and subsequently washed with PBS to remove unbound antibody. The plate is then blocked with a PBS/BSA solution to prevent sample proteins from non-specifically binding the microtiter plate. Test sample are subsequently added to the wells and the plate is incubated for a period of time sufficient for binding to occur. The wells are washed with PBS to remove unbound protein. Labeled antibodies, which recognize portions of SI, CDX1 or CDX2 not recognized by the first antibody, are added to the wells. The plate is incubated for a period of time sufficient for binding to occur and subsequently washed with PBS to remove unbound, labeled antibody. The amount of labeled and bound antibody is subsequently determined by standard techniques.

Kits which are useful for the detection of SI, CDX1 or CDX2 in a test sample comprise a container comprising anti-SI antibodies and a container or containers comprising controls. Controls include one control sample which does not contain SI, CDX1 or CDX2 and/or another control sample which contained the SI, CDX1 or CDX2. The antibodies used in the kit are detectable such as being detectably labeled. If the detectable antibody is not labeled, it may be detected by second antibodies or protein A for example which may also be provided in some kits in separate containers. Additional components in some kits include solid support, buffer, and instructions for carrying out the assay. Additionally the kit may optionally comprise depictions or photographs that represent the appearance of positive and negative results.

The immunoassay is useful for detecting SI, CDX1 or CDX2 in homogenized tissue samples and body fluid samples including the plasma portion or cells in the fluid sample.

Western Blots may be useful in assisting the diagnosis os individuals suffering from stomach or esophageal cancer by detecting presence of SI, CDX1 or CDX2 of non-colorectal tissue or body fluid. Western blots may also be used to detect presence of SI, CDX1 or CDX2 in sample of tumor from an individual suffering from cancer. Western blots use detectable antibodies to bind to any SI, CDX1 or CDX2 present in a sample and thus indicate the presence of the receptor in the sample.

Western blot techniques, which are described in Sambrook, J. et al., (1989) Molecular Cloning: A Laboratory Manual, Cold Spring Harbor Laboratory Press, Cold Spring Harbor, NY, are similar to immunoassays with the essential difference being that prior to exposing the sample to the antibodies, the proteins in the samples are separated by gel electrophoresis and the separated proteins are then probed with antibodies. In some preferred embodiments, the matrix is an SDS-PAGE gel matrix and the separated proteins in the matrix are transferred to a carrier such as filter paper prior to probing with antibodies. Antibodies described above are useful in Western blot methods.

Generally, samples are homogenized and cells are lysed using detergent such as Triton-X. The material is then separated by the standard techniques in Sambrook, J. et al., (1989) Molecular Cloning: A Laboratory Manual, Cold Spring Harbor Laboratory Press, Cold Spring Harbor, NY.

Kits which are useful for the detection of SI, CDX1 or CDX2 in a test sample by Western Blot comprise a container comprising anti-SI antibodies and a container or containers comprising controls. Controls include one control sample which does not contain SI and/or another control sample which contains SI, CDX1 or CDX2. The antibodies used in the kit are detectable such as being detectably labeled. If the detectable antibody is not labeled, it may be detected by second antibodies or protein A for example which may also be provided in some kits in separate containers. Additional components in some kits include instructions for carrying out the assay. Additionally the kit may optionally comprise depictions or photographs that represent the appearance of positive and negative results.

Western blots are useful for detecting SI, CDX1 or CDX2 in homogenized tissue samples and body fluid samples including the plasma portion or cells in the fluid sample.

### In vivo Imaging and Therapeutics

According to some embodiments of the invention, compositions and *in vivo* methods are provided for detecting, imaging, or treating metastatic colorectal cancer and primary and/or metastatic stomach or esophageal tumors in an individual.

When the conjugated compositions of the present invention are administered outside the intestinal tract such as when administered in the circulatory system, they remain segregated from the cells that line the intestinal tract and will bind only to cells outside the intestinal tract which express SI. The conjugated compositions will not bind to the nominal cells but will bind to metastatic colorectal cancer cells and primary and/or metastatic stomach or esophageal cells. Thus, the active moieties of conjugated compositions administered outside the intestinal tract are delivered to cells which express SI such as metastatic colorectal cancer cells and primary and/or metastatic stomach or esophageal cancer cells.

Therapeutic and diagnostic pharmaceutical compositions useful in the present invention include conjugated compounds that specifically target cells that express SI. These conjugated compounds include moieties that bind to SI which do not bind to cells of normal tissue in the body except cells of the intestinal tract since the cells of other tissues do not express SI.

Unlike normal colorectal cells, cancer cells that express SI are accessible to substances administered outside the intestinal tract, for example administered in the circulatory system. The only SI in normal tissue exist in the apical membranes of intestinal mucosa cells and thus effectively isolated from the targeted cancer chemotherapeutics and imaging agents administered outside the intestinal tract by the intestinal mucosa barrier. Thus, metastatic colorectal cancer and primary and/or metastatic stomach or esophageal cancer cells may be targeted by conjugated compounds of the present invention by introducing such compounds outside the intestinal tract such as for example by administering pharmaceutical compositions that comprise conjugated compounds into the circulatory system.

One having ordinary skill in the art can identify individuals suspected of suffering from metastatic colorectal cancer and primary and/or metastatic stomach or esophageal cancer. In those individuals diagnosed with colorectal, stomach or esophageal cancer, it is not unusual and in some cases standard therapy to suspect metastasis and aggressively attempt to eradicate metastasized cells. The present invention provides pharmaceutical compositions and methods for imaging and thereby will more definitively diagnose primary and metastastic disease. Further, the present invention provides pharmaceutical compositions comprising therapeutic agents and methods for specifically targeting and eliminating metastatic colorectal cancer and primary and/or metastatic stomach or esophageal cancer cells. Further, the present invention provides pharmaceutical compositions that comprise therapeutics and methods for specifically eliminating metastatic colorectal cancer and primary and/or metastatic stomach or esophageal cancer cells.

The pharmaceutical compositions which comprise conjugated compositions of the present invention may be used to diagnose or treat individuals suffering from metastatic colorectal cancer and primary and/or metastatic stomach or esophageal tumors.

The present invention relies upon the use of a SI binding moiety in a conjugated composition. The SI binding moiety is essentially a portion of the conjugated composition which acts as a ligand to a SI and thus specifically binds to it. The conjugated composition also includes an active moiety which is associated with the SI binding moiety; the active moiety being an active agent which is either useful to image, target, neutralize or kill the cell.

According to the present invention, the SI binding moiety is the SI ligand portion of a conjugated composition. In some embodiments, the SI ligand is an antibody.

In some preferred embodiments, conjugated compounds comprise SI binding moieties that comprise an anti-SI antibody.

It is preferred that the SI ligand used as the SI binding moiety be as small as possible. Thus it is preferred that the SI ligand be a non-peptide small molecule or small peptide, preferably less than 25 amino acids, more preferably less than 20 amino acids. In some embodiments, the SI ligand which constitute the SI binding moiety of a conjugated composition is less than 15 amino acids. SI binding peptide comprising less than 10 amino acids and SI binding peptide less than 5 amino acids may be used as SI binding moieties according to the present invention. It is within the scope of the present invention to include larger molecules which serve as SI binding moieties including, but not limited to molecules such as antibodies which specifically bind to SI.

Additionally, SI ligands may include any of the well known carbohydrate substrates normally processed by the enzyme including those substrates engineered to be recognized by the enzyme cleavage site but which are resistant to being processed. Horii, S et al. J. Med. Chem. 29:1038-1046 (1986), disclose examples of such compounds.

SI ligands useful as SI binding moieties may be identified using various well known combinatorial library screening technologies such as those set forth in Example 1 herein.

An assay may be used to test both peptide and non-peptide compositions to determine whether or not they are SI ligands or, to test conjugated compositions to determine if they possess SI - binding activity. Such compositions that specifically bind to SI can be identified by a competitive binding assay using antibodies known to bind to the SI. The competitive binding assay is a standard technique in pharmacology which can be readily performed by those having ordinary skill in the art using readily available starting materials.

SI may be produced synthetically, recombinantly or isolated from natural sources.

Using a solid phase synthesis as an example, the protected or derivatized amino acid is attached to an inert solid support through its unprotected carboxyl or amino group. The protecting group of the amino or carboxyl group is then selectively removed and the next amino acid in the sequence having the complementary (amino or carboxyl) group suitably protected is admixed and reacted with the residue already attached to the solid support. The protecting group of the amino or carboxyl group is then removed from this newly added amino acid residue, and the next amino acid (suitably protected) is then added, and so forth. After all the desired amino acids have been linked in the proper sequence, any remaining terminal and side group protecting groups (and solid support) are removed sequentially or concurrently, to provide the final peptide. The peptide of the invention are preferably devoid ofbenzylated or methylbenzylated amino acids. Such protecting group moieties may be used in the course of synthesis, but they are removed before the peptides are used. Additional reactions may be necessary, as described elsewhere, to form intramolecular linkages to restrain conformation.

Antibodies against SI may be routinely produced and used in competition assays to identify SI ligands or as starting materials for conjugated compounds according to the invention.

According to the present invention, the active moiety may be a therapeutic agent or an imaging agent. One having ordinary skill in the art can readily recognize the advantages of being able to specifically target cancer cells with an SI ligand and conjugate such a ligand with many different active agents.

Chemotherapeutics useful as active moieties which when conjugated to a SI binding moiety are specifically delivered to cells that express SI such as stomach or esophageal cancer cells, are typically small chemical entities produced by chemical synthesis. Chemotherapeutics include cytotoxic and cytostatic drugs. Chemotherapeutics may include those which have other effects on cells such as reversal of the transformed state to a differentiated state or those which inhibit cell replication. Examples of chemotherapeutics include common cytotoxic or cytostatic drugs such as for example: methotrexate (amethopterin), doxorubicin (adrimycin), daunorubicin, cytosinarabinoside, etoposide, 5-4 fluorouracil, melphalan, chlorambucil, and other nitrogen mustards (e.g. cyclophosphamide), cis-platinum, vindesine (and other vinca alkaloids), mitomycin and bleomycin. Other chemotherapeutics include: purothionin (barley flour oligopeptide), macromomycin. 1,4-benzoquinone derivatives and trenimon.

Toxins are useful as active moieties. When a toxin is conjugated to a SI binding moiety, the conjugated composition is specifically delivered to a cell that expresses SI such as stomach or esophageal cancer cells by way of the SI binding moiety and the toxin moiety kills the cell. Toxins are generally complex toxic products of various organisms including bacteria, plants, *etc.* Examples of toxins include but are not limited to: ricin, ricin A chain (ricin toxin), *Pseudomonas* exotoxin (PE), diphtheria toxin (DT), *Clostridium perfringens* phospholipase C (PLC), bovine pancreatic ribonuclease (BPR), pokeweed antiviral protein (PAP), abrin, abrin A chain (abrin toxin), cobra venom factor (CVF), gelonin (GEL), saporin (SAP), modeccin, viscumin and volkensin. As discussed above, when protein toxins are employed with SI binding peptides, conjugated compositions may be produced using recombinant DNA techniques. Briefly, a recombinant DNA molecule can be constructed which encodes both the SI ligand and the toxin on a chimeric gene. When the chimeric gene is expressed, a fusion protein is produced which includes a SI binding moiety and an active moiety. Protein toxins are also useful to form conjugated compounds with SI binding peptides through non-peptidyl bonds.

In addition, there are other approaches for utilizing active agents for the treatment of cancer. For example, conjugated compositions may be produced which include a SI binding moiety and an active moiety which is an active enzyme. The SI binding moiety specifically localizes the conjugated composition to the tumor cells. An inactive prodrug which can be converted by the enzyme into an active drug is administered to the patient. The prodrug is only converted to an active drug by the enzyme which is localized to the tumor. An example of an enzyme/prodrug pair includes alkaline phosphatase/etoposidephosphate. In such a case, the alkaline phosphatase is conjugated to a SI binding ligand. The conjugated compound is administered and localizes at the cancer cell. Upon contact with etoposidephosphate (the prodrug), the etoposidephosphate is converted to etoposide, a chemotherapeutic drug which is taken up by the cancer cell.

Radiosensitizing agents are substances that increase the sensitivity of cells to radiation. Examples of radiosensitizing agents include nitroimidazoles, metronidazole and misonidazole (see: DeVita, V.T. Jr. in Harrison's Principles of Internal Medicine, p.68, McGraw-Hill Book Co., N.Y. 1983). The conjugated compound that comprises a radiosensitizing agent as the active moiety is administered and localizes at the metastatic colorectal cancer cell and primary and/or metastatic stomach or esophageal cancer cell. Upon exposure of the individual to radiation, the radiosensitizing agent is "excited" and causes the death of the cell.

Radionuclides may be used in pharmaceutical compositions that are useful for radiotherapy or imaging procedures.

Examples of radionuclides useful as toxins in radiation therapy include: ⁴⁷Sc, ⁶⁷Cu, ⁹⁰Y, ¹⁰⁹Pd, ¹²³I, ¹²⁵I, ¹³¹I, ¹⁸⁶Re, ¹⁸⁸Re, ¹⁹⁹Au, ²¹¹At, ²¹²Pb and ²¹²B. Other radionuclides which have been used by those having ordinary skill in the art include: ³²P and ³³P, ⁷¹Ge, ⁷⁷As ¹⁰³Pb, ¹⁰⁵Rh, ¹¹¹Ag, ¹¹⁹Sb, ¹²¹Sn, ¹³¹Cs, ¹⁴³Pr, ¹⁶¹Tb, ¹⁷⁷Lu, ¹⁹¹Os, ^{193M}Pt, ¹⁹⁷Hg all beta negative and/or auger emitters. Some preferred radionuclides include: ⁹⁰Y, ¹³¹I ²¹¹At and ²¹²Pb/²¹²Bi.

According to the present invention, the active moieties may be an imaging agent. Imaging agents are useful diagnostic procedures as well as the procedures used to identify the location of cancer cells. Imaging can be performed by many procedures well-known to those having ordinary skill in the art and the appropriate imaging agent useful in such procedures may be conjugated to a SI ligand by well-known means. Imaging can be performed, for example, by radioscintigraphy, nuclear magnetic resonance imaging (MRI) or computed tomography (CT scan). The most commonly employed radionuclide imaging agents include radioactive iodine and indium. Imaging by CT scan may employ a heavy metal such as iron chelates. MRI scanning may employ chelates of gadolinium or manganese. Additionally, positron emission tomography (PET) may be possible using positron emitters of oxygen, nitrogen, iron, carbon, or gallium. Example of radionuclides useful in imaging procedures include: ⁴³K, ⁵²Fe, ⁵⁷Co, ⁶⁷Cu, ⁶⁷Ga, ⁶⁸Ga, ⁷⁷Br, ⁸¹Rb/^{81M}Kr, ^{87M}Sr, ^{99M}TC, ¹¹¹In, ^{113M}In, ¹²³I, ¹²⁵I, ¹²⁷Cs, ¹²⁹Cs, ¹³¹I, ¹³²I, ¹⁹⁷Hg, ²⁰³Pb and ²⁰⁶Bi.

It is preferred that the conjugated compositions be non-immunogenic or immunogenic at a very low level. Accordingly, it is preferred that the SI binding moiety be a small, poorly immunogenic or non-immunogenic peptide or a non-peptide. The SI binding moiety may be a humanized or primatized antibody or a human antibody.

SI ligands are conjugated to active agents by a variety of well-known techniques readily performed without undue experimentation by those having ordinary skill in the art. The technique used to conjugate the SI ligand to the active agent is dependent upon the molecular nature of the SI ligand and the active agent. After the SI ligand and the active agent are conjugated to form a single molecule, assays may be performed to ensure that the conjugated molecule retains the activities of the moieties. The competitive binding assay described above may be used to confirm that the SI binding moiety retains its binding activity as a conjugated compound. Similarly, the activity of the active moiety may be tested using various assays for each respective type of active agent. Radionuclides retain there activity, i.e. their radioactivity, irrespective of conjugation. With respect to active agents which are toxins, drugs and targeting agents, standard assays to demonstrate the activity of unconjugated forms of these compounds may be used to confirm that the activity has been retained.

Conjugation may be accomplished directly between the SI ligand and the active agent or linking, intermediate molecular groups may be provided between the SI ligand and the active agent. Crosslinkers are particularly useful to facilitate conjugation by providing attachment sites for each moiety. Crosslinkers may include additional molecular groups which serve as spacers to separate the moieties from each other to prevent either from interfering with the activity of the other.

One having ordinary skill in the art may conjugate a SI ligand to a chemotherapeutic drug using well-known techniques. For example, Magerstadt, M. Antibody Conjugates and Malignant Disease. (1991) CRC Press, Boca Raton, USA, pp. 110-152) teaches the conjugation of various cytostatic drugs to amino acids of antibodies. Such reactions may be applied to conjugate chemotherapeutic drugs to SI ligands, including anti-SI antibodies, with an appropriate linker. Most of the chemotherapeutic agents currently in use in treating cancer possess functional groups that are amenable to chemical crosslinking directly with proteins. For example, free amino groups are available on methotrexate, doxorubicin, daunorubicin, cytosinarabinoside, cis-platin, vindesine, mitomycin and bleomycin while free carboxylic acid groups are available on methotrexate, melphalan, and chlorambucil. These functional groups, that is free amino and carboxylic acids, are targets for a variety of homobifunctional and heterobifunctional chemical crosslinking agents which can crosslink these drugs directly to the single free amino group of an antibody. For example, one procedure for crosslinking SI ligands which have a free amino group to active agents which have a free amino group such as methotrexate, doxorubicin, daunorubicin, cytosinarabinoside, *cis*-platin, vindesine, mitomycin and bleomycin, or alkaline phosphatase, or protein- or peptide-based toxin employs homobifunctional succinimidyl esters, preferably with carbon chain spacers such as disuccinimidyl suberate (Pierce Co, Rockford, IL). In the event that a cleavable conjugated compound is required, the same protocol would be employed utilizing 3,3'- dithiobis (sulfosuccinimidylpropionate; Pierce Co.).

In order to conjugate a SI ligand that is a peptide or protein to a peptide-based active agent such as a toxin, the SI ligand and the toxin may be produced as a single, fusion protein either by standard peptide synthesis or recombinant DNA technology, both of which can be routinely performed by those having ordinary skill in the art. Alternatively, two peptides, the SI ligand peptide and the peptide-based toxin may be produced and/or isolated as separate peptides and conjugated using crosslinkers. As with conjugated compositions that contain chemotherapeutic drugs, conjugation of SI binding peptides and toxins can exploit the ability to modify the single free amino group of a SI binding peptide while preserving the receptor-binding function of this molecule.

One having ordinary skill in the art may conjugate a SI ligand to a radionuclide using well-known techniques. For example, Magerstadt, M. (1991) Antibody Conjugates And Malignant Disease, CRC Press, Boca Raton, FLA,; and Barchel, S.W. and Rhodes, B.H., (1983) Radioimaging and Radiotherapy, Elsevier, NY, NY, teach the conjugation of various therapeutic and diagnostic radionuclides to amino acids of antibodies.

The present invention provides pharmaceutical compositions that comprise the conjugated compounds of the invention and pharmaceutically acceptable carriers or diluents. The pharmaceutical composition of the present invention may be formulated by one having ordinary skill in the art. Suitable pharmaceutical carriers are described in *Remington's Pharmaceutical Sciences,* A. Osol, a standard reference text in this field. In carrying out methods of the present invention, conjugated compounds of the present invention can be used alone or in combination with other diagnostic, therapeutic or additional agents. Such additional agents include excipients such as coloring, stabilizing agents, osmotic agents and antibacterial agents. Pharmaceutical compositions are preferably sterile and pyrogen free.

The conjugated compositions of the invention can be, for example, formulated as a solution, suspension or emulsion in association with a pharmaceutically acceptable parenteral vehicle. Examples of such vehicles are water, saline, Ringer's solution, dextrose solution, and 5% human serum albumin. Liposomes may also be used. The vehicle may contain additives that maintain isotonicity (e.g., sodium chloride, mannitol) and chemical stability (e.g., buffers and preservatives). The formulation is sterilized by commonly used techniques. For example, a parenteral composition suitable for administration by injection is prepared by dissolving 1.5% by weight of active ingredient in 0.9% sodium chloride solution.

The pharmaceutical compositions according to the present invention may be administered as either a single dose or in multiple doses. The pharmaceutical compositions of the present invention may be administered either as individual therapeutic agents or in combination with other therapeutic agents. The treatments of the present invention may be combined with conventional therapies, which may be administered sequentially or simultaneously.

The pharmaceutical compositions of the present invention may be administered by any means that enables the conjugated composition to reach the targeted cells. In some embodiments, routes of administration include those selected from the group consisting of intravenous, intraarterial, intraperitoneal, local administration into the blood supply of the organ in which the tumor resides or directly into the tumor itself. In addition to an intraoperative spray, conjuagated compounds may be delivered intrathecally, intraventrically, stereotactically, intrahepatically such as via the portal vein, by inhalation, and intrapleurally. Intravenous administration is the preferred mode of administration. It may be accomplished with the aid of an infusion pump.

The dosage administered varies depending upon factors such as: the nature of the active moiety; the nature of the conjugated composition; pharmacodynamic characteristics; its mode and route of administration; age, health, and weight of the recipient; nature and extent of symptoms; kind of concurrent treatment; and frequency of treatment.

Because conjugated compounds are specifically targeted to cells with one or more SI molecules, conjugated compounds which comprise chemotherapeutics or toxins are administered in doses less than those which are used when the chemotherapeutics or toxins are administered as unconjugated active agents, preferably in doses that contain up to 100 times less active agent. In some embodiments, conjugated compounds which comprise chemotherapeutics or toxins are administered in doses that contain 10-100 times less active agent as an active moiety than the dosage of chemotherapeutics or toxins administered as unconjugated active agents. To determine the appropriate dose, the amount of compound is preferably measured in moles instead of by weight. In that way, the variable weight of different SI binding moieties does not affect the calculation. Presuming a one to one ratio of SI binding moiety to active moiety in conjugated compositions of the invention, less moles of conjugated compounds may be administered as compared to the moles of unconjugated compounds administered, preferably up to 100 times less moles.

Typically, chemotherapeutic conjugates are administered intravenously in multiple divided doses.

Up to 20 gm IV/dose of methotrexate is typically administered in an unconjugated form. When methotrexate is administered as the active moiety in a conjugated compound of the invention, there is a 10-to 100-fold dose reduction. Thus, presuming each conjugated compound includes one molecule of methotrexate conjugated to one SI binding moiety, of the total amount of conjugated compound administered, up to about 0.2 - 2.0 g of methotrexate is present and therefore administered. In some embodiments, of the total amount of conjugated compound administered, up to about 200 mg - 2g of methotrexate is present and therefore administered.

To dose conjugated compositions comprising SI binding moieties linked to active moieties that are radioisotopes in pharmaceutical compositions useful as imaging agents, it is presumed that each SI binding moiety is linked to one radioactive active moiety. The amount of radioisotope to be administered is dependent upon the radioisotope. Those having ordinary skill in the art can readily formulate the amount of conjugated compound to be administered based upon the specific activity and energy of a given radionuclide used as an active moiety. Typically 0.1-100 millicuries per dose of imaging agent, preferably 1-10 millicuries, most often 2-5 millicuries are administered. Thus, pharmaceutical compositions according to the present invention useful as imaging agents which comprise conjugated compositions comprising a SI binding moiety and a radioactive moiety comprise 0.1-100 millicuries, in some embodiments preferably 1-10 millicuries, in some embodiments preferably 2-5 millicuries, in some embodiments more preferably 1-5 millicuries. Examples of dosages include: ¹³¹I = between about 0.1-100 millicuries per dose, in some embodiments preferably 1-10 millicuries, in some embodiments 2-5 millicuries, and in some embodiments about 4 millicuries; ¹¹¹In = between about 0.1-100 millicuries per dose, in some embodiments preferably 1-10 millicuries, in some embodiments 1-5 millicuries, and in some embodiments about 2 millicuries; ^{99m}Tc = between about 0.1-100 millicuries per dose, in some embodiments preferably 5-75 millicuries, in some embodiments 10-50 millicuries, and in some embodiments about 27 millicuries. Wessels B.W. and R.D. Rogus (1984) Med. Phys. 11:638 and Kwok, C.S. et al. (1985) Med. Phys. 12:405, disclose detailed dose calculations for diagnostic and therapeutic conjugates which may be used in the preparation of pharmaceutical compositions of the present invention which include radioactive conjugated compounds.

One aspect of the present invention relates to a method of treating individuals suspected of suffering from metastatic colorectal cancer and primary and/or metastatic stomach or esophageal cancer. Such individuals may be treated by administering to the individual a pharmaceutical composition that comprises a pharmaceutically acceptable carrier or diluent and a conjugated compound that comprises a SI - binding moiety and an active moiety wherein the active moiety is a radiostable therapeutic agent. In some embodiments of the present invention, the pharmaceutical composition comprises a pharmaceutically acceptable carrier or diluent and a conjugated compound that comprises a SI binding moiety and an active moiety wherein the active moiety is a radiostable active agent and the SI binding moiety is an antibody. In some embodiments of the present invention, the pharmaceutical composition comprises a pharmaceutically acceptable carrier or diluent and a conjugated compound that comprises a SI binding moiety and an active moiety wherein the active moiety is a radiostable therapeutic agent. In some embodiments of the present invention, the pharmaceutical composition comprises a pharmaceutically acceptable carrier or diluent and a conjugated compound that comprises a SI binding moiety and an active moiety wherein the active moiety is a radiostable active agent selected from the group consisting of: methotrexate, doxorubicin, daunorubicin, cytosinarabinoside, etoposide, 5-4 fluorouracil, melphalan, chlorambucil, cis-platinum, vindesine, mitomycin, bleomycin, purothionin, macromomycin, 1,4-benzoquinone derivatives, trenimon, ricin, ricin A chain, *Pseudomonas* exotoxin, diphtheria toxin, *Clostridium perfringens* phospholipase C, bovine pancreatic ribonuclease, pokeweed antiviral protein, abrin, abrin A chain, cobra venom factor, gelonin, saporin, modeccin, viscumin, volkensin, alkaline phosphatase, nitroimidazole, metronidazole and misonidazole. The individual being treated may be diagnosed as having metastasized colorectal, stomach or esophageal cancer or may be diagnosed as having primary colorectal, stomach or esophageal cancer and may undergo the treatment proactively in the event that there is some metastasis as yet undetected. The pharmaceutical composition contains a therapeutically effective amount of the conjugated composition. A therapeutically effective amount is an amount which is effective to cause a cytotoxic or cytostatic effect on cancer cells without causing lethal side effects on the individual.

One aspect of the present invention relates to a method of treating individuals suspected of suffering from metastatic colorectal cancer and primary and/or metastatic stomach or esophageal cancer. Such individuals may be treated by administering to the individual a pharmaceutical composition that comprises a pharmaceutically acceptable carrier or diluent and a conjugated compound that comprises a SI binding moiety and an active moiety wherein the active moiety is a radioactive. In some embodiments of the present invention, the pharmaceutical composition comprises a pharmaceutically acceptable carrier or diluent and a conjugated compound that comprises a SI binding moiety and an active moiety wherein the active moiety is a radioactive and the SI binding moiety is an antibody. In some embodiments of the present invention, the pharmaceutical composition comprises a pharmaceutically acceptable carrier or diluent and a conjugated compound that comprises a SI binding moiety and an active moiety wherein the active moiety is a radioactive agent selected from the group consisting of: ⁴⁷Sc, ⁶⁷Cu, ⁹⁰Y, ¹⁰⁹Pd, ¹²³I, ¹²⁵I, ¹³¹I, ¹⁸⁶Re, ¹⁸⁸Re, ¹⁹⁹Au, ²¹¹At, ²¹²Pb, ²¹²B, ³²P and ³³P, ⁷¹Ge, ⁷⁷As, ¹⁰³Pb, ¹⁰⁵Rh, ¹¹¹Ag, ¹¹⁹Sb, ¹²¹Sn, ¹³¹Cs, ¹⁴³Pr, ¹⁶¹Tb, ¹⁷⁷Lu, ¹⁹¹Qs, ^{193M}Pt, ¹⁹⁷Hg, ³²P and ³³P, ⁷¹Ge, ⁷⁷As, ¹⁰³Pb, ¹⁰⁵Rh, ¹¹¹Ag, ¹¹⁹Sb, ¹²¹Sn, ¹³¹Cs, ¹⁴³Pr, ¹⁶¹Tb, ¹⁷⁷Lu, ¹⁹¹Os, ^{193M}Pt, ¹⁹⁷Hg, all beta negative and/or auger emitters. The individual being treated may be diagnosed as having metastasized cancer or may be diagnosed as having localized cancer and may undergo the treatment proactively in the event that there is some metastasis as yet undetected. The pharmaceutical composition contains a therapeutically effective amount of the conjugated composition. A therapeutically effective amount is an amount which is effective to cause a cytotoxic or cytostatic effect on metastatic colorectal cancer and primary and/or metastatic stomach or esophageal cancer cells without causing lethal side effects on the individual. The composition may be injected intratumorally into primary tumors.

One aspect of the present invention relates to a method of detecting metastatic colorectal cancer and primary and/or metastatic stomach or esophageal cancer cells in an individual suspected of suffering from primary or metastasized colorectal, stomach or esophageal cancer by radioimaging. Individuals may be suspected of having primary stomach or esophageal tumors which diagnosis can be confirmed by administering to the individual, an imaging agent which binds to SI. Tumors can be imaged by detecting localization at the stomach or esophagus. Individuals may be diagnosed as suffering from metastasized colorectal, stomach or esophageal cancer and the metastasized colorectal, stomach or esophageal cancer cells may be detected by administering to the individual, preferably by intravenous administration, a pharmaceutical composition that comprises a pharmaceutically acceptable carrier or diluent and a conjugated compound that comprises a SI binding moiety and an active moiety wherein the active moiety is a radioactive and detecting the presence of a localized accumulation or aggregation of radioactivity, indicating the presence of cells with SI. In some embodiments of the present invention, the pharmaceutical composition comprises a pharmaceutically acceptable carrier or diluent and a conjugated compound that comprises a SI binding moiety and an active moiety wherein the active moiety is a radioactive and the SI binding moiety is an antibody. In some embodiments of the present invention, the pharmaceutical composition comprises a pharmaceutically acceptable carrier or diluent and a conjugated compound that comprises an SI binding moiety and an active moiety wherein the active moiety is a radioactive agent selected from the group consisting of: radioactive heavy metals such as iron chelates, radioactive chelates of gadolinium or manganese, positron emitters of oxygen, nitrogen, iron, carbon, or gallium, ⁴³K, ⁵²Fe, ⁵⁷Co, ⁶⁷Cu, ⁶⁷Ga, ⁶⁸Ga, ⁷⁷Br, ⁸¹Rb/^{81M}Kr, ^{87M}Sr, ^{99M}Tc, ¹¹¹In, ^{113M}IN, ¹²³I, ¹²⁵I, ¹²⁷Cs, ¹²⁹Cs, ¹³¹I, ¹³²I, ¹⁹⁷Hg, ²⁰³Pb and ²⁰⁶Bi. The individual being treated may be diagnosed as having metastasizing colorectal, stomach or esophageal cancer or may be diagnosed as having localized colorectal, stomach or esophageal cancer and may undergo the treatment proactively in the event that there is some metastasis as yet undetected. The pharmaceutical composition contains a diagnostically effective amount of the conjugated composition. A diagnostically effective amount is an amount which can be detected at a site in the body where cells with SI are located without causing lethal side effects on the individual.

### Photodynamic imaging and therapy

According to some embodiments of the invention, SI binding moieties are conjugates to photoactivated imaging agents or therapeutics. Maier A. et al. Lasers in Surgery and Medicine 26:461-466 (2000) disclose an example of photodynamic therapy. QLT, Inc (Vancouver, BC) commercially distribute photosensitive active agents which can be linked to SI ligands. Such conjugated compounds can be used in photodynamic therapeutic and imaging protocols to activate the SI-bound conjugated agents which are thus targeted to tumor cells. In some embodiments, the conjugated compounds are applied as an intraoperative spray which is subsequently exposed to light to activate compounds bound to cells that express SI.

In some embodiments, the photodynamic agent is fluorophore or porphyrins. Examples of porphyrin include: hematoporphyrin derivative (HPD) and porfimer sodium (Photofrin®). A second generation photosensitizers is BPD verteporfin. In some embiodiments the fluorophore is tetramethylrotamine. Lasers are generally the primary light source used to activate porphyrins. Light Emitting Diodes (LEDs) and florescent light sources may also be used in some applications.

In addition to an intraoperative spray, conjuagated compounds may be delivered intrathecally, intraventrically, stereotactically, intrahepatically such as via the portal vein, by inhalation, and intrapleurally.

### Drug Delivery Targeted To Stomach or Esophageal Cancer Cells Generally

Another aspect of the invention relates to unconjugated and conjugated compositions which comprise a SI ligand used to deliver therapeutic agents to cells that comprise a SI such as metastatic colorectal cancer and primary and/or metastatic stomach or esophageal cancer cells. In some embodiments, the agent is a drug or toxin such as: methotrexate, doxorubicin, daunorubicin, cytosinarabinoside, etoposide, 5-4 fluorouracil, melphalan, chlorambucil, cis-platinum, vindesine, mitomycin, bleomycin, purothionin, macromomycin, 1,4-benzoquinone derivatives, trenimon, ricin, ricin A chain, *Pseudomonas* exotoxin, diphtheria toxin, *Clostridium perfringens* phospholipase C, bovine pancreatic ribonuclease, pokeweed antiviral protein, abrin, abrin A chain, cobra venom factor, gelonin, saporin, modeccin, viscumin, volkensin, alkaline phosphatase, nitroimidazole, metronidazole and misonidazole.. Genetic material is delivered to cancer cells to produce an antigen that can be targeted by the immune system or to produce a protein which kills the cell or inhibits its proliferation. In some embodiments, the SI ligand is used to deliver nucleic acids that encode nucleic acid molecules which replace defective endogenous genes or which encode therapeutic proteins. In some embodiments, the compositions are used in gene therapy protocols to deliver to individuals, genetic material needed and/or desired to make up for a genetic deficiency.

In some embodiments, the SI ligand is combined with or incorporated into a delivery vehicle thereby converting the delivery vehicle into a specifically targeted delivery vehicle. For example, a SI binding peptide may be integrated into the outer portion of a viral particle making such a virus a SI-bearing cell specific virus. Similarly, the coat protein of a virus may be engineered such that it is produced as a fusion protein which includes an active SI binding peptide that is exposed or otherwise accessible on the outside of the viral particle making such a virus a SI-bearing cell-specific virus. In some embodiments, a SI ligand may be integrated or otherwise incorporated into the liposomes wherein the SI ligand is exposed or otherwise accessible on the outside of the liposome making such liposomes specifically targeted to SI-bearing cells.

The active agent in the conjugated or unconjugated compositions according to this aspect of the invention is a drug, toxin or nucleic acid molecule. The nucleic acid may be RNA or preferably DNA. In some embodiments, the nucleic acid molecule is an antisense molecule or encodes an antisense sequence whose presence in the cell inhibits production of an undesirable protein. In some embodiments, the nucleic acid molecule encodes a ribozyme whose presence in the cell inhibits production of an undesirable protein. In some embodiments, the nucleic acid molecule encodes a protein or peptide that is desirably produced in the cell. In some embodiments, the nucleic acid molecule encodes a functional copy of a gene that is defective in the targeted cell. The nucleic acid molecule is preferably operably linked to regulatory elements needed to express the coding sequence in the cell.

Liposomes are small vesicles composed of lipids. Genetic constructs which encode proteins that are desired to be expressed in SI-bearing cells are introduced into the center of these vesicles. The outer shell of these vesicles comprise an a SI ligand. Liposomes Volumes 1, 2 and 3 CRC Press Inc. Boca Raton FLA, disclose preparation of liposome-encapsulated active agents which include antibodies in the outer shell. In the present invention, a SI ligand such as for example an anti-SI antibodies is associated with the in the outer shell. Unconjugated compositions which comprise a SI ligand in the matrix of a liposome with an active agent inside include such compositions in which the SI ligand is preferably an antibody.

In one embodiment, the delivery of normal copies of the p53 tumor suppressor gene to the cancer cells is accomplished using SI ligand to target the gene therapeutic. Mutations of the p53 tumor suppressor gene appears to play a prominent role in the development of many cancers. One approach to combating this disease is the delivery of normal copies of this gene to the cancer cells expressing mutant forms of this gene. Genetic constructs that comprise normal p53 tumor suppressor genes are incorporated into liposomes that comprise a SI ligand. The composition is delivered to the tumor. SI ligands specifically target and direct the liposomes containing the normal gene to correct the lesion created by mutation of p53 suppressor gene. Preparation of genetic constructs is with the skill of those having ordinary skill in the art. The present invention allows such construct to be specifically targeted by using the SI ligands of the present invention. The compositions of the invention include a SI ligand such as an anti-SI antibody associated with a delivery vehicle and a gene construct which comprises a coding sequence for a protein whose production is desired in the cells of the intestinal tract linked to necessary regulatory sequences for expression in the cells. For uptake by cells of the intestinal tract, the compositions are administered orally or by enema whereby they enter the intestinal tract and contact cells which comprise SI. The delivery vehicles associate with the SI by virtue of the SI ligand and the vehicle is internalized into the cell or the active agent/genetic construct is otherwise taken up by the cell. Once internalized, the construct can provide a therapeutic effect on the individual.

### Antisense

The present invention provides compositions, kits and methods which are useful to prevent and treat colorectal, stomach or esophageal cancer cells by providing the means to specifically deliver antisense compounds to colorectal, stomach or esophageal cancer cells and thereby stop expression of genes in such cells in which undesirable gene expression is taking place without negatively effecting cells in which no such expression occurs.

The conjugated compositions of the present invention are useful for targeting cells that express SI including colorectal, stomach or esophageal cancer cells. The conjugated compositions will not bind to non-colorectal derived cells. Non-colorectal cells, lacking SI, do not take up the conjugated compositions. Normal colorectal cells do have SI and will take up the compositions. The present invention provides compositions and methods of delivering antisense compositions to normal and cancerous colorectal cells and stomach or esophageal cancer cells.

The present invention provides a cell specific approach in which only normal and cancerous colorectal cells and primary and/or metastatic stomach or esophageal cancer cells are exposed to the active portion of the compound and only those cells are effected by the conjugated compound. The SI binding moiety binds to normal and cancerous colorectal cells and primary and/or metastatic stomach or esophageal cancer cells. Upon binding to these cells, the conjugated compound is internalized and the delivery of the conjugated compound including the antisense portion of the molecule is effected. The presence of the conjugated compound in normal colorectal cells has no effect on such cells because the cancer-associated gene for which the antisense molecule that makes up the active moiety of the conjugated compound is complementary is not being expressed. However, in colorectal cancer cells, the cancer gene for which the antisense molecule that makes up the active moiety of the conjugated compound is complementary is being expressed. The presence of the conjugated compound in colorectal cancer cells serves to inhibit or prevent transcription or translation of the cancer gene and thereby reduce or eliminate the transformed phenotype.

The invention can be used to combat primary and/or metastasized colorectal, stomach or esophageal cancer as well as to prevent the emergence of the transformed phenotype in normal colon cells. Thus the invention can be used therapeutically as well as prophylactically.

One having ordinary skill in the art can readily identify individuals suspected of suffering from stomach or esophageal cancer. In those individuals diagnosed with stomach or esophageal cancer, it is standard therapy to suspect metastasis and aggressively attempt to eradicate metastasized cells. The present invention provides pharmaceutical compositions and methods for specifically targeting and eliminating metastasized colorectal cancer cells and primary and/or metastatic stomach or esophageal cancer cells. Further, the present invention provides pharmaceutical compositions that comprise therapeutics and methods for specifically eliminating metastasized colorectal cancer cells and primary and/or metastatic stomach or esophageal cancer cells.

The present invention relies upon the use of a SI binding moiety in a conjugated composition. The SI product binding moiety is essentially a portion of the conjugated composition which acts as a ligand to the SI and thus specifically binds to these receptors. The conjugated composition also includes an active moiety which is associated with the SI binding moiety; the active moiety being an antisense composition useful to inhibit or prevent transcription or translation of expression of genes whose expression is associated with cancer.

According to the present invention, the active moiety is an antisense composition. In particular, the antisense molecule that makes up the active moiety of a conjugated compound hybridizes to DNA or RNA in a colorectal, stomach or esophageal cancer cell and inhibits and/or prevents transcription or translation of the DNA or RNA from taking place. The antisense compositions may be a nucleic acid molecule, a derivative or an analogs thereof. The chemical nature of the antisense composition may be that of a nucleic acid molecule or a modified nucleic acid molecule or a non-nucleic acid molecule which possess functional groups that mimic a DNA or RNA molecule that is complementary to the DNA or RNA molecule whose expression is to be inhibited or otherwise prevented. Antisense compositions inhibit or prevent transcription or translation of genes whose expression is linked to colorectal, stomach or esophageal cancer, i.e. cancer associated genes.

Point mutations insertions, and deletions in K-ras and H-ras have been identified in many tumors. Complex characteristics of the alterations of oncogenes HER-2/ERBB-2, HER-1/ERBB-1, HRAS-1, C-MYC and anti-oncogenes p53, RB1.

Chemical carcinogenesis in a rat model demonstrated point mutations in fos, an oncogene which mediates transcriptional regulation and proliferation. See: Alexander, RJ, et al. Oncogene alterations in rat colon tumors induced by N-methyl-N-nitrosourea. American Journal of the Medical Sciences. 303(1):16-24, 1992, Jan.

Chemical carcinogenesis in a rat model demonstrated point mutations in the oncogene abl. See: Alexander, RJ, et al. Oncogene alterations in rat colon tumors induced by N-methyl-N-nitrosourea. American Journal of the Medical Sciences. 303(1):16-24, 1992, Jan.

MYC is an oncogene that plays a role in regulating transcription and proliferation. A 15-base antisense oligonucleotide to myc complementary to the translation initiation region of exon II was incubated with colorectal cancer cells. This antisense molecule inhibited proliferation of colorectal cancer cells in a dos-dependent fashion. Interestingly, the uptake of this oligonucleotide was low (0.7%). Also, transfer of a normal chromosome 5 to colorectal cancer cells resulted in the regulation of myc expression and loss of proliferation. These data suggest that a tumor suppressor gene important in the regulation of myc is contained on this chromosome.

A novel protein tyrosine phosphatase, G1, has been identified. Examination of the mRNA encoding this protein in colorectal tumor cells revealed that it undergoes point mutations and deletions in these cells and may play a role in proliferation characteristic of these cells. Takekawa, M. et al. Chromosomal localization of the protein tyrosine phosphatase G1 gene and characterization of the aberrant transcripts in human colon cancer cells. FEBS Letters. 339(3):222-8, 1994 Feb. 21.

Gastrin regulates colon cancer cell growth through a cyclic AMP-dependent mechanism mediated by PKA. Antisense oligodeoxynucleotides to the regulatory subunit of a specific class of PKA inhibited the growth-promoting effects of cyclic AMP in colon carcinoma cells. See: Bold, RJ, et al. Experimental gene therapy of human colon cancer. Surgery. 116(2):189-95; discussion 195-6, 1994 Aug. and Yokozaki, H., et al. An antisense oligodeoxynucleotide that depletes RI alpha subunit of cyclic AMP-dependent protein kinase induces growth inhibition in human cancer cells. Cancer Research. 53(4):868-72, 1993 Feb 15.

CRIPTO is an epidermal growth factor-related gene expressed in a majority of colorectal cancer tumors. Antisense phosphorothioate oligodeoxynucleotides to the 5'-end of CRIPTO mRNA significantly reduced CRIPTO expression and inhibited colorectal tumor cell growth *in vitro* and *in vivo.* Ciardiello, F. et al. Inhibition of CRIPTO expression and tumorigenicity in human colon cancer cells by antisense RNA and oligodeoxynucleotides. Oncogene. 9(1):291-8, 1994 Jan.

Many carcinoma cells secrete transforming growth factor alpha. A 23 nucleotide antisense oligonucleotide to TGF alpha mRNA inhibited both DNA synthesis an proliferation of colorectal cancer cells. Sizeland, AM, Burgess, AW. Antisense transforming growth factor alpha oligonucleotides inhibit autocrine stimulated proliferation of a colon carcinoma cell line. Molecular Biology of the Cell. 3(11):1235-43, 1992 Nov.

Antisense compositions including oligonucleotides, derivatives and analogs thereof, conjugation protocols, and antisense strategies for inhibition of transcription and translation are generally described in: Antisense Research and Applications, Crooke, S. and B. Lebleu, eds. CRC Press, Inc. Boca Raton FLA 1993; Nucleic Acids in Chemistry and Biology Blackburn, G. and M.J. Gait, eds. IRL Press at Oxford University Press, Inc. New York 1990; and Oligonucleotides and Analogues: A Practical Approach Eckstein, F. ed., IRL Press at Oxford University Press, Inc. New York 1991.

The antisense molecules of the present invention comprise a sequence complementary to a fragment of a colorectal cancer gene. See Ullrich et al., EMBO J., 1986, 5:2503.

Antisense compositions which can make up an active moiety in conjugated compounds of the invention include oligonucleotides formed of homopyrimidines can recognize local stretches of homopurines in the DNA double helix and bind to them in the major groove to form a triple helix. See: Helen, C and Toulme, JJ. Specific regulation of gene expression by antisense, sense, and antigene nucleic acids. Biochem. Biophys Acta, 1049:99-125, 1990. Formation of the triple helix would interrupt the ability of the specific gene to undergo transcription by RNA polymerase. Triple helix formation using myc-specific oligonucleotides has been observed. See: Cooney, M, et al. Science 241:456-459.

Antisense oligonucleotides of DNA or RNA complementary to sequences at the boundary between introns and exons can be employed to prevent the maturation of newly-generated nuclear RNA transcripts of specific genes into mRNA for transcription.

Antisense RNA complimentary to specific genes can hybridize with the mRNA for tat gene and prevent its translation. Antisense RNA can be provided to the cell as "ready-to-use" RNA synthesized in vitro or as an antisense gene stably transfected into cells which will yield antisense RNA upon transcription. Hybridization with mRNA results in degradation of the hybridized molecule by RNAse H and/or inhibition of the formation of translation complexes. Both result in a failure to produce the product of the original gene.

Antisense sequences of DNA or RNA can be delivered to cells. Several chemical modifications have been developed to prolong the stability and improve the function of these molecules without interfering in their ability to recognize specific sequences. These include increasing their resistance to degradation by DNases, including phosphotriesters, methylphosphonates, phosphorothioates, alpha-anomers, increasing their affinity for their target by covalent linkage to various intercalating agents such as psoralens, and increasing uptake by cells by conjugation to various groups including polylysine. These molecules recognize specific sequences encoded in mRNA and their hybridization prevents translation of and increases the degradation of these messages.

Conjugated compositions of the invention provide a specific and effective means for terminating the expression of genes which cause nepplastic transformation. SI undergo ligand-induced endocytosis and can deliver conjugated compounds to the cytoplasm of cells.

SI - binding moieties are conjugated directly to antisense compositions such as nucleic acids which are active in inducing a response. For example, antisense oligonucleotides to MYC are conjugated directly to an anti-SI antibody. This has been performed employing peptides that bind to the CD4 receptor. See: Cohen, JS, ed. Oligodeoxynucleotides: Antisense Inhibitors of Gene Expression. Topics in Molecular and Structural Biology. CRC Press, Inc., Boca Raton, 1989. The precise backbone and its synthesis is not specified and can be selected from well-established techniques. Synthesis would involve either chemical conjugation or direct synthesis of the chimeric molecule by solid phase synthesis employing FMOC chemistry. See: Haralambidis, J, et al. (1987) Tetrahedron Lett. 28:5199-5202. Alternatively, the peptide-nucleic acid conjugate may be synthesized directly by solid phase synthesis as a peptide-peptide nucleic acid chimera by solid phase synthesis. Nielsen, PE, et al. (1994) Sequence-specific transcription arrest by peptide nucleic acid bound to the DNA template strand. Gene 149:139-145.

In some embodiments, polylysine can be complexed to conjugated compositions of the invention in a non-covalent fashion to nucleic acids and used to enhance delivery of these molecules to the cytoplasm of cells. In addition, peptides and proteins can be conjugated to polylysine in a covalent fashion and this conjugate complexed with nucleic acids in a non-covalent fashion to further enhance the specificity and efficiency of uptake of the nucleic acids into cells. Thus, SI ligand is conjugated chemically to polylysine by established techniques. The polylysine-SI translation product ligand conjugate may be complexed with nucleic acids of choice. Thus, polylysine-orosomucoid conjugates were employed to specifically plasmids containing genes to be expressed to hepatoma cells expressing the orosomucoid receptor. This approach can be used to delivery whole genes, or oligonucleotides. Thus, it has the potential to terminate the expression of an undesired gene (eg. MYC, ras) or replace: the function of a lost or deleted gene (eg. hMSH2, hMLH1, hPMS1, and hPMS2).

According to a preferred embodiment, Myc serves as a gene whose expression is inhibited by an antisense molecule within a conjugated composition. SI binding moieties are used to deliver a 15-based antisense oligonucleotide to myc complementary to the translation initiation region of exon II. The 15-base antisense oligonucleotide to MYC is synthesized as reported in Collins, JF, Herman, P, Schuch, C, Bagby GC, Jr. Journal of Clinical Investigation. 89(5):1523-7, 1992 May. In some embodiments, the conjugated composition is conjugated to polylysine as reported previously. Wu, GY, and Wu, CH. (1988) Evidence for ed gene delivery to Hep G2 hepatoma cells in vitro. Biochem. 27:887-892.

Conjugated compositions may be synthesized as a chimeric molecule directly by solid phase synthesis. pmolar to nanomolar concentrations for this conjugate suppress MYC synthesis in colorectal cancer cells *in vitro.*

Antisense molecules are preferably hybridize to, i.e. are complementary to, a nucleotide sequence that is 5-50 nucleotides in length, more preferably 5-25 nucleotides and in some embodiments 10-15 nucleotides.

In addition, mismatches within the sequences identified above, which achieve the methods of the invention, such that the mismatched sequences are substantially complementary to the cancer gene sequences are also considered within the scope of the disclosure. Mismatches which permit substantial complementarity to the cancer gene sequences will be known to those of skill in the art once armed with the present disclosure. The oligos may also be unmodified or modified.

Therapeutic compositions and methods may be used to combat colorectal, stomach or esophageal cancer in cases where the cancer is localized and/or metastasized. Individuals are administered a therapeutically effective amount of conjugated compound. A therapeutically effective amount is an amount which is effective to cause a cytotoxic or cytostatic effect on cancer cells without causing lethal side effects on the individual. An individual who has been administered a therapeutically effective amount of a conjugated composition has a increased chance of eliminating colorectal, stomach or esophageal cancer as compared to the risk had the individual not received the therapeutically effective amount.

To treat localized colorectal, stomach or esophageal cancer, a therapeutically effective amount of a conjugated compound is administered such that it will come into contact with the localized tumor. Thus, the conjugated compound may be administered orally or intratumorally. Oral and rectal formulation are taught in Remington's Pharmaceutical Sciences, 18th Edition, 1990, Mack Publishing Co., Easton PA.

The pharmaceutical compositions according to the present invention may be administered as either a single dose or in multiple doses. The pharmaceutical compositions of the present invention may be administered either as individual therapeutic agents or in combination with other therapeutic agents. The treatments of the present invention may be combined with conventional therapies, which may be administered sequentially or simultaneously.

The present invention is directed to a method of delivering antisense compounds to normal and cancerous colorectal cells and to stomach or esophageal cancer cells and inhibiting expression of cancer genes in mammals. The methods comprise administering to a mammal an effective amount of a conjugated composition which comprises a SI binding moiety conjugated to an antisense oligonucleotide having a sequence which is complementary to a region of DNA or mRNA of a cancer gene.

The conjugated compounds may be administering to mammals in a mixture with a pharmaceutically-acceptable carrier, selected with regard to the intended route of administration and the standard pharmaceutical practice. Dosages will be set with regard to weight, and clinical condition of the patient. The conjugated compositions of the present invention will be administered for a time sufficient for the mammals to be free of undifferentiated cells and/or cells having an abnormal phenotype. In therapeutic methods treatment extends for a time sufficient to inhibit transformed cells from proliferating and conjugated compositions may be administered in conjunction with other chemotherapeutic agents to manage and combat the patient's cancer.

The conjugated compounds of the invention may be employed in the method of the invention singly or in combination with other compounds. The amount to be administered will also depend on such factors as the age, weight, and clinical condition of the patient. See Gennaro, Alfonso, ed., Remington's Pharmaceutical Sciences, 18th Edition, 1990, Mack Publishing Co., Easton PA.

### Therapeutic and Prophylactic Vaccines

The invention relates to prophylactic and therapeutic vaccines for protecting individuals against metastasized colorectal cancer cells and primary and/or metastatic stomach or esophageal cancer cells and for treating individuals who are suffering from metastasized colorectal cancer cells and primary and/or metastatic stomach or esophageal cancer cells.

According to the present invention, SI, CDX1 or CDX2 serves as targets against which a protective and therapeutic immune response can be induced. Specifically, vaccines are provided which induce an immune response against SI, CDX1 or CDX2. The vaccines of the invention include, but are not limited to, the following vaccine technologies:
1) DNA vaccines, i.e. vaccines in which DNA that encodes at least an epitope from SI, CDX1 or CDX2 is administered to an individual's cells where the epitope is expressed and serves as a target for an immune response;
2) infectious vector mediated vaccines such as recombinant adenovirus, vaccinia, *Salmonella,* and BCG wherein the vector carries genetic information that encodes at least an epitope from SI, CDX1 or CDX2 protein such that when the infectious vector is administered to an individual, the epitope is expressed and serves as a target for an immune response;
3) killed or inactivated vaccines which a) comprise either killed cells or inactivated viral particles that display at least an epitope from SI, CDX1 or CDX2 protein and b) when administered to an individual serves as a target for an immune response;
4) haptenized killed or inactivated vaccines which a) comprise either killed cells or inactivated viral particles that display at least an epitope from SI, CDX1 or CDX2 protein, b) are haptenized to be more immunogenic and c) when administered to an individual serves as a target for an immune response;
5) subunit vaccines which are vaccines that include protein molecules that include at least an epitope from SI, CDX1 or CDX2 protein; and
6) haptenized subunit vaccines which are vaccines that a) include protein molecules that include at least an epitope from SI, CDX1 or CDX2 protein and b) are haptenized to be more immunogenic.

The present invention relates to administering to an individual a protein or nucleic acid molecule that comprises or encodes, respectively, an immunogenic epitope against which an therapeutic and prophylactic immune response can be induced. Such epitopes are generally at least 6-8 amino acids in length. The vaccines of the invention therefore comprise proteins which are at least, or nucleic acids which encode at least, 6-8 amino acids in length from SI protein. The vaccines of the invention may comprise proteins which are at least, or nucleic acids which encode at least 10 to about 1000 amino acids in length. The vaccines of the invention may comprise proteins which are at least, or nucleic acids which encode at least, about 25 to about 500 amino acids in length. The vaccines of the invention may comprise proteins which are at least, or nucleic acids which encode at least, about 50 to about 400 amino acids in length. The vaccines of the invention may comprise proteins which are at least, or nucleic acids which encode at least, about 100 to about 300 amino acids in length.

The present invention relates to compositions for and methods of treating individuals who are known to have metastasized colorectal cancer cells and primary and/or metastatic stomach or esophageal cancer cells. Metastasized colorectal cancer and primary and/or metastatic stomach or esophageal cancer may be diagnosed by those having ordinary skill in the art using the methods described herein or art accepted clinical and laboratory pathology protocols. The present invention provides an immunotherapeutic vaccine useful to treat individuals who have been diagnosed as suffering from metastatic colorectal cancer and primary and/or metastatic stomach or esophageal cancer. The immunotherapeutic vaccines of the present invention may be administered in combination with other therapies.

The present invention relates to compositions for and methods of preventing metastatic colorectal cancer and primary and/or metastatic stomach or esophageal cancer in individual is suspected of being susceptible to colorectal, stomach or esophageal cancer. Such individuals include those whose family medical history indicates above average incidence of colorectal, stomach or esophageal cancer among family members and/or those who have already developed colorectal, stomach or esophageal cancer and have been effectively treated who therefore face a risk of relapse and recurrence. Such individuals include those which have been diagnosed as having colorectal, stomach or esophageal cancer including localized only or localized and metastasized colorectal, stomach or esophageal cancer which has been resected or otherwise treated. The vaccines of the present invention may be to susceptible individuals prophylactically to prevent and combat metastatic colorectal cancer and primary and metastatic stomach or esophageal cancer.

The invention relates to compositions which are the active components of such vaccines or required to make the active components, to methods of making such compositions including the active components, and to methods of making and using vaccines.

The present invention relates to recombinant vectors, including expression vectors, that comprise the SI gene transcript or a fragment thereof. The present invention relates to recombinant vectors, including expression vectors that comprise nucleotide sequences that encode SI, CDX1 or CDX2 protein or a functional fragment thereof.

The present invention relates to host cells which comprise such vectors and to methods of making SI, CDX1 or CDX2 protein using such recombinant cells.

The present invention relates to the isolated SI, CDX1 or CDX2 gene transcript and to the isolated SI, CDX1 or CDX2 proteins and to isolated antibodies specific for such protein and to hybridomas which produce such antibodies.

The present invention relates to the isolated SI, CDX1 or CDX2 and functional fragments thereof. Accordingly, some aspects of the invention relate to isolated proteins that comprise at least one epitope of an SI, CDX1 or CDX2

Some aspects of the invention relate to the above described isolated proteins which are haptenized to render them more immunogenic. That is, some aspects of the invention relate to haptenized proteins that comprise at least one SI, CDX1 or CDX2 epitope.

Accordingly, some aspects of the invention relate to isolated nucleic acid molecules that encode proteins that comprise at least one SI, CDX1 or CDX2 epitope.

Naked DNA vaccines are described in PCT/US90/01515. Others teach the use of liposome mediated DNA transfer, DNA delivery using microprojectiles (U.S. Patent No. 4,945,050 issued July 31, 1990 to Sanford et al.), and DNA delivery using electroporation. In each case, the DNA may be plasmid DNA that is produced in bacteria, isolated and administered to the animal to be treated. The plasmid DNA molecules are taken up by the cells of the animal where the sequences that encode the protein of interest are expressed. The protein thus produced provides a therapeutic or prophylactic effect on the animal.

The use of vectors including viral vectors and other means of delivering nucleic acid molecules to cells of an individual in order to produce a therapeutic and/or prophylactic immunological effect on the individual are similarly well known. Recombinant vaccines that employ vaccinia vectors are, for example, disclosed in U.S. Patent Number 5,017,487 issued May 21, 1991 to Stunnenberg et al.*.*

In some cases, tumor cells from the patient are killed or inactivated and administered as a vaccine product. Berd et al. May 1986 Cancer Research 46:2572-2577 and Berd et al. May 1991 Cancer Research 51:2731-2734, describes the preparation and use of tumor cell based vaccine products. According to some aspects of the present invention, the methods and techniques described in Berd *et al.* are adapted by using colorectal, stomach or esophageal cancer cells instead of melanoma cells.

The manufacture and use of isolated translation products and fragments thereof useful for example as laboratory reagents or components of subunit vaccines are well known. One having ordinary skill in the art can isolate SI, CDX1 or CDX2 gene transcript or the specific portion thereof that encodes SI, CDX1 or CDX2 or a fragment thereof. Once isolated, the nucleic acid molecule can be inserted it into an expression vector using standard techniques and readily available starting materials.

The recombinant expression vector that comprises a nucleotide sequence that encodes the nucleic acid molecule that encodes SI, CDX1 or CDX2 or a fragment thereof or a protein that comprises the SI, CDX1 or CDX2 or a fragment thereof. The recombinant expression vectors of the invention are useful for transforming hosts to prepare recombinant expression systems for preparing the isolated proteins of the invention.

The present invention relates to a host cell that comprises the recombinant expression vector that includes a nucleotide sequence that encodes SI, CDX1 or CDX2 protein or a fragment thereof or SI, CDX1 or CDX2 or a fragment thereof. Host cells for use in well known recombinant expression systems for production of proteins are well known and readily available. Examples of host cells include bacteria cells such as E. *coli,* yeast cells such as *S*. *cerevisiae,* insect cells such as *S*. *frugiperda,* non-human mammalian tissue culture cells chinese hamster ovary (CHO) cells and human tissue culture cells such as HeLa cells.

The present invention relates to a transgenic non-human mammal that comprises the recombinant expression vector that comprises a nucleic acid sequence that encodes the proteins of the invention. Transgenic non-human mammals useful to produce recombinant proteins are well known as are the expression vectors necessary and the techniques for generating transgenic animals. Generally, the transgenic animal comprises a recombinant expression vector in which the nucleotide sequence that encodes SI, CDX1 or CDX2 or a fragment thereof or a protein that comprises SI, CDX1 or CDX2 or a fragment thereof operably linked to a mammary cell specific promoter whereby the coding sequence is only expressed in mammary cells and the recombinant protein so expressed is recovered from the animal's milk.

In some embodiments, for example, one having ordinary skill in the art can, using well known techniques, insert such DNA molecules into a commercially available expression vector for use in well known expression systems such as those described herein.

The expression vector including the DNA that encodes a SI, CDX1 or CDX2 or a functional fragment thereof or a protein that comprises a SI or a functional fragment thereof is used to transform the compatible host which is then cultured and maintained under conditions wherein expression of the foreign DNA takes place. The protein of the present invention thus produced is recovered from the culture, either by lysing the cells or from the culture medium as appropriate and known to those in the art. The methods of purifying the SI, CDX1 or CDX2 or a fragment thereof or a protein that comprises the same using antibodies which specifically bind to the protein are well known. Antibodies which specifically bind to a particular protein may be used to purify the protein from natural sources using well known techniques and readily available starting materials. Such antibodies may also be used to purify the protein from material present when producing the protein by recombinant DNA methodology. The present invention relates to antibodies that bind to an epitope which is present on one or more SI, CDX1 or CDX2 translation products or a fragment thereof or a protein that comprises the same. Antibodies that bind to an epitope which is present on the SI, CDX1 or CDX2 are useful to isolate and purify the protein from both natural sources or recombinant expression systems using well known techniques such as affinity chromatography. Immunoaffinity techniques generally are described in Waldman et al. 1991 Methods of Enzymol. 195:391-396. Antibodies are useful to detect the presence of such protein in a sample and to determine if cells are expressing the protein. The production of antibodies and the protein structures of complete, intact antibodies, Fab fragments and F(ab)₂ fragments and the organization of the genetic sequences that encode such molecules are well known and are described, for example, in Harlow, E. and D. Lane (1988) ANTIBODIES: A Laboratory Manual, Cold Spring Harbor Laboratory, Cold Spring Harbor, NY.

In some embodiments of the invention, transgenic non-human animals are generated. The transgenic animals according to the invention contain nucleotides that encode SI, CDX1 or CDX2 or a fragment thereof or a protein that comprises the same under the regulatory control of a mammary specific promoter. One having ordinary skill in the art using standard techniques, such as those taught in U.S. Patent No. 4,873,191 issued October 10, 1989 to Wagner and U.S. Patent No. 4,736,866 issued April 12, 1988 to Leder, can produce transgenic animals which produce SI or a fragment thereof or a protein that comprises the same. Preferred animals are goats and rodents, particularly rats and mice.

In addition to producing these proteins by recombinant techniques, automated peptide synthesizers may also be employed to produce SI, CDX1 or CDX2 or a fragment thereof or a fragment thereof or a protein that comprises the same. Such techniques are well known to those having ordinary skill in the art and are useful if derivatives which have substitutions not provided for in DNA-encoded protein production.

In some embodiments, the protein that makes up a subunit vaccine or the cells or particles of a killed or inactivated vaccine may be haptenized to increase immunogenicity. In some cases, the haptenization is the conjugation of a larger molecular structure to SI, CDX1 or CDX2 or a fragment thereof or a protein that comprises the same. In some cases, tumor cells from the patient are killed and haptenized as a means to make an effective vaccine product. In cases in which other cells, such as bacteria or eukaryotic cells which are provided with the genetic information to make and display a SI or a fragment thereof or a protein that comprises the same, are killed and used as the active vaccine component, such cells are haptenized to increase immunogenicity. Haptenization is well known and can be readily performed.

Methods of haptenizing cells generally and tumor cells in particular are described in Berd et al. May 1986 Cancer Research 46:2572-2577 and Berd et al. May 1991 Cancer Research 51:2731-2734. Additional haptenization protocols are disclosed in Miller et al. 1976 J. Immunol. 117(5:1):1591-1526.

Haptenization compositions and methods which may be adapted to be used to prepare haptenized immunogens according to the present invention include those described in the following U.S. Patents: U.S. Patent Number 5,037,645 issued August 6, 1991 to Strahilevitz; U.S. Patent Number 5,112,606 issued May 12, 1992 to Shiosaka et al.*;* U.S. Patent Number 4,526716 issued July 2, 1985 to Stevens; U.S. Patent Number 4,329,281 issued May 11, 1982 to Christenson et al.*;* and U.S. Patent Number 4,022,878 issued May 10, 1977 to Gross. Peptide vaccines and methods of enhancing immunogenicity of peptides which may be adapted to modify immunogens of the invention are also described in Francis et al. 1989 Methods of Enzymol. 178:659-676. Sad et al. 1992 Immunolology 76:599-603, teaches methods of making immunotherapeutic vaccines by conjugating gonadotropin releasing hormone to diphtheria toxoid. SI immunogens may be similarly conjugated to produce an immunotherapeutic vaccine of the present invention. MacLean et al. 1993 Cancer Immunol. Immunother. 36:215-222, describes conjugation methodologies for producing immunotherapeutic vaccines which may be adaptable to produce an immunotherapeutic vaccine of the present invention. The hapten is keyhole limpet hemocyanin which may be conjugated to an immunogen.

Vaccines according to some aspects of the invention comprise a pharmaceutically acceptable carrier in combination with an immunogen. Pharmaceutical formulations are well known and pharmaceutical compositions comprising such proteins may be routinely formulated by one having ordinary skill in the art. Suitable pharmaceutical carriers are described in *Remington's Pharmaceutical Sciences,* A. Osol, a standard reference text in this field. The present invention relates to an injectable pharmaceutical composition that comprises a pharmaceutically acceptable carrier and an immunogen. The immunogen is preferably sterile and combined with a sterile pharmaceutical carrier.

In some embodiments, for example, SI, CDX1 or CDX2 or a fragment thereof or a fragment thereof or a protein that comprises the same can be formulated as a solution, suspension, emulsion or lyophilized powder in association with a pharmaceutically acceptable vehicle. Examples of such vehicles are water, saline, Ringer's solution, dextrose solution, and 5% human serum albumin. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques.

An injectable composition may comprise the immunogen in a diluting agent such as, for example, sterile water, electrolytes/dextrose, fatty oils of vegetable origin, fatty esters, or polyols, such as propylene glycol and polyethylene glycol. The injectable must be sterile and free of pyrogens.

The vaccines of the present invention may be administered by any means that enables the immunogenic agent to be presented to the body's immune system for recognition and induction of an immunogenic response. Pharmaceutical compositions may be administered parenterally, i.e., intravenous, subcutaneous, intramuscular.

Dosage varies depending upon known factors such as the pharmacodynamic characteristics of the particular agent, and its mode and route of administration; age, health, and weight of the recipient; nature and extent of symptoms, kind of concurrent treatment, frequency of treatment, and the effect desired. An amount of immunogen is delivered to induce a protective or therapeutically effective immune response. Those having ordinary skill in the art can readily determine the range and optimal dosage by routine methods.

The following examples are illustrative but are not meant to be limiting of the present invention.

### EXAMPLES

### Example 1

As stated above, a SI binding moiety is a SI ligand that may be an antibody, a protein, a polypeptide, a peptide or a non-peptide. Peptides and non-peptide SI ligands may be identified using well known technology.

Over the past 10 years, it has become recognized that the specific high-affinity interaction of a receptor and a ligand, for example a SI and an anti-SI antibody, has its basis in the 3-dimensional conformational space of the ligand and the complimentary 3-dimensional configuration of the region of the molecule involved in ligand binding. In addition, it has become recognized that various arrays of naturally-occurring amino acids, non-natural amino acids, and organic molecules can be organized in configurations that are unrelated to the natural ligands in their linear structure, but resemble the 3-dimensional structure of the natural ligands in conformational space and, thus, are recognized by receptors with high affinity and specificity. Furthermore, techniques have been described in the literature that permit one of ordinary skill in the art to generate large libraries of these arrays of natural amino acids, non-natural amino acids and organic compounds to prospectively identify individual compounds that interact with receptors with high affinity and specificity which are unrelated to the native ligand of that receptor. Thus, it is a relatively straightforward task for one of ordinary skill in the art to identify arrays of naturally occurring amino acids, non-natural amino acids, or organic compounds which can bind specifically and tightly to the SI, which bear no structural relationship to an anti-SI antibody.

To identify SI ligands that are peptides, those having ordinary skill in the art can use any of the well known methodologies for screening random peptide libraries in order to identify peptides which bind to the SI. In the most basic of methodologies, the peptides which bind to the target are isolated and sequenced. In some methodologies, each random peptide is linked to a nucleic acid molecule which includes the coding sequence for that particular random peptide. The random peptides, each with an attached coding sequence, are contacted with a SI and the peptides which are unbound to the SI are removed. The nucleic acid molecule which includes the coding sequence of the peptide that binds to the SI can then be used to determine the amino acid sequence of the peptide as well as produce large quantities of the peptide. It is also possible to produce peptide libraries on solid supports where the spatial location on the support corresponds to a specific synthesis and therefore specific peptide. Such methods often use photolithography-like steps to create diverse peptide libraries on solid supports in which the spatial address on the support allows for the determination of the sequence.

The production of organic compound libraries on solid supports may also be used to produce combinatorial libraries of non-peptide compounds such as oligonucleotides and sugars, for example. As in the case of peptide libraries on solid supports, the spatial location on the support corresponds to a specific synthesis and therefore specific compound. Such methods often use photolithography-like steps to create diverse compound libraries on solid supports in which the spatial address on the support allows for the determination of the synthesis scheme which produced the compound. Once the synthesis scheme is identified, the structure of the compound can become known.

Gallop et al. 1994 J. Medicinal Chemistry 37:1233, provides a review of several of the various methodologies of screening random peptide libraries and identifying peptides from such libraries which bind to target proteins. Following these teachings, SI specific ligands that are peptides and that are useful as SI specific binding moieties may be identified by those having ordinary skill in the art.

Peptides and proteins displayed on phage particles are described in Gallop *et al. Supra.* Random arrays of nucleic acids can be inserted into genes encoding surface proteins of bacteriophage which are employed to infect bacteria, yielding phage expressing the peptides encoded by the random array of nucleotides on their surface. These phage displaying the peptide can be employed to determine whether those peptides can bind to specific proteins, receptors, antibodies, etc. The identity of the peptide can be determined by sequencing the recombinant DNA from the phage expressing the peptide. This approach has the potential to yield vast arrays of peptides in a library (up to 10⁹ unique peptides). This technique has been employed to identify novel binding peptides to the fibrinogen receptor on platelets, which bear no sequence homology to the natural occurring ligands of this receptor (Smith et al., 1993 Gene 128:37). Similarly, this technique has been applied to identify peptides which bind to the MHC class II receptor (Hammer et al., 1993 Cell 74:197) and the chaperonin receptor (Blond-Elguindi et al., 1993 Cell 75:717).

Peptides displayed on plasmids are described in Gallop *et al. Supra.* In this approach, the random oligonucleotides which encode the library of peptides can be expressed on a specific plasmid whose expression is under the control of a specific promoter, such as the lac operon. The peptides are expressed as fusion proteins coupled to the Lac I protein, under the control of the lac operon. The fusion protein specifically binds to the lac operator on the plasmid and so the random peptide is associated with the specific DNA element that encodes it. In this way, the sequence of the peptide can be deduced, by PCR of the DNA associated with the fusion protein. These proteins can be screened in solution phase to determine whether they bind to specific receptors. Employing this approach, novel substrates have been identified for specific enzymes (Schatz 1993).

A variation of the above technique, also described in Gallop *et al. Supra,* can be employed in which random oligonucleotides encoding peptide libraries on plasmids can be expressed in cell-free systems. In this approach, a molecular DNA library can be constructed containing the random array of oligonucleotides, which are then expressed in a bacterial in vitro transcription/translation system. The identity of the ligand is determined by purifying the complex of nascent chain peptide/polysome containing the mRNA of interest on affinity resins composed of the receptor and then sequencing following amplification with RT-PCR. Employing this technique permits generation of large libraries (up to 10¹¹ recombinants). Peptides which recognize antibodies specifically directed to dynorphin have been identified employing this technique (Cull et al., 1992 Proc. Natl. Acad. Sci. USA 89:1865).

Libraries of peptides can be generated for screening against a receptor by chemical synthesis. For example, simultaneous preparation of large numbers of diverse peptides have been generated employing the approach of multiple peptide synthesis as described in Gallop *et al. Supra.* In one application, random peptides are generated by standard solid-phase Merrifield synthesis on polyacrylamide microtiter plates (multipin synthesis) which are subsequently screened for their ability to compete with receptor binding in a standard competitive binding assay (Wang et al., 1993 Bioorg. Med. Chem. Lett. 3:447). Indeed, this approach has been employed to identify novel binding peptides to the substance P receptor (Wang *et al. Supra).* Similarly, peptide libraries can be constructed by multiple peptide synthesis employing the "tea bag" method in which bags of solid support resin are sequentially incubated with various amino acids to generate arrays of different peptides (Gallop *et al. Supra).* Employing this approach, peptides which bind to the integrin receptor (Ruggeri et al., 1986 Proc. Natl. Acad. Sci. USA 83:5708) and the neuropeptide Y receptor (Beck-Sickinger et al., 1990 Int. J. Peptide Protein Res. 36:522) have been identified.

In general, the generation and utility of combinatorial libraries depend on (1) a method to generate diverse arrays of building blocks, (2) a method for identifying members of the array that yield the desired function, and (3) a method for deconvoluting the structure of that member. Several approaches to these constraints have been defined.

The following is a description of methods of library generation which can be used in procedures for identifying SI ligands according to the invention.

Modifications of the above approaches can be employed to generate libraries of vast molecular diversity by connecting together members of a set of chemical building blocks, such as amino acids, in all possible combinations (Gallop *et al. Supra*) In one approach, mixtures of activated monomers are coupled to a growing chain of amino acids on a solid support at each cycle. This is a multivalent synthetic system.

Also, split synthesis involves incubating the growing chain in individual reactions containing only a single building block *(Gallop et al. Supra).* Following attachment, resin from all the reactions are mixed and apportioned into individual reactions for the next step of coupling. These approaches yield a stochastic collection of n^{x} different peptides for screening, where n is the number of building blocks and x is the number of cycles of reaction.

Alternatively, arrays of molecules can be generated in which one or more positions contain known amino acids, while the remainder are random (Gallop et al. *Supra*)*.* These yield a limited library which is screened for members with the desired activity. These members are identified, their structure determined, and the structure regenerated with another position containing defined amino acids and screened. This iterative approach ultimately yields peptides which are optimal for recognizing the conformational binding pocket of a receptor.

In addition, arrays are not limited to amino acids forming peptides, but can be extended to linear and nonlinear arrays of organic molecules (Gordon et al., 1994 J. Medicinal Chemistry 37:1385). Indeed, employing this approach of generating libraries of randomly arrayed inorganic building blocks, ligands which bound to 7-transmembrane receptors were identified *(*Zuckermann et al., 1994 J..Med. Chem. 37:2678).

Libraries are currently being constructed which can be modified after synthesis to alter the chemical side groups and bonds, to give "designer" arrays to test for their interaction with receptors (Osteresh et al., 1994 Proc. Natl. Acad. Sci. USA 91:11138). This technique, generating "libraries from libraries", was applied to the permethylation of a peptide library which yielded compounds with selective antimicrobial activity against gram positive bacteria.

Libraries are also being constructed to express arrays of pharmacological motifs, rather than specific structural arrays of amino acids (Sepetov et al., 1995 Proc. Natl. Acad. Sci. USA 92:5426). This technique seeks to identify structural motifs that have specific affinities for receptors, which can be modified in further refinements employing libraries to define structure-activity relationships. Employing this approach of searching motif libraries, generating "libraries of libraries", reduces the number of component members required for screening in the early phase of library examination.

The following is a description of methods of identifying SI ligands according to the invention from libraries of randomly generated molecules.

Components in the library which interact with receptors may be identified by their binding to receptors immobilized on solid support (Gordon *et al. Supra*)*.*

They may also be identified by their ability to compete with native ligand for binding to cognate receptors in solution phase *(*Gordon *et al. Supra).*

Components may be identified by their binding to soluble receptors when those components are immobilized on solid supports (Gordon *et al. Supra*)*.*

Once a member of a library which binds receptors has been identified, the structure of that member must be deconvoluted (deduced) in order to identify the structure and generate large quantities to work with, or develop further analogs to study structure-activity relationships. The following is a description of methods of deconvolution for deducing the structure of molecules identified as potential SI ligands according to the invention.

Peptide libraries may be expressed on the surface of bacteriophage particles (Gallop *et al. Supra*). Once the peptide interacting with the receptor has been identified, its structure can be deduced by isolating the DNA from the phage and determining its sequence by PCR.

Libraries expressed on plasmids, under the control of the Lac operon can be deconvoluted since these peptides are fused with the lac I protein which specifically interacts with the lac operon on the plasmid encoding the peptide (Gallop *et al. Supra)* The structure can be deduced by isolating that plasmid attached to the lac I protein and deducing the nucleotide and peptide sequence by PCR.

Libraries expressed on plasmids can also be expressed in cell-free systems employing transcription/translation systems (Gallop *et al. Supra*)*.* In this paradigm, the protein interacting with receptors is isolated with its attached ribosome and mRNA. The sequence of the peptide is deduced by RT-PCR of the associated mRNA.

Library construction can be coupled with photolithography, so that the structure of any member of the library can be deduced by determining its position within the substrate array (Gallop *et al. Supra*)*.* This technique is termed positional addressability, since the structural information can be deduced by the precise position of the member.

Members of a library can also be identified by tagging the library with identifiable arrays of other molecules (Ohhneyer et al., 1993 Proc. Natl. Acad. Sci. USA 90:10922, and Gallop *et al. Supra*)*.* This technique is a modification of associating the peptide with the plasmid of phage encoding the sequence, described above. Some methods employ arrays of nucleotides to encode the sequential synthetic history of the peptide. Thus, nucleotides are attached to the growing peptide sequentially, and can be decoded by PCR to yield the structure of the associated peptide. Alternatively, arrays of small organic molecules can be employed as sequencable tags which encode the sequential synthetic history of the peptide. Thus, nucleotides are attached to the growing peptide sequentially, and can be decoded by PCR to yield the structure of the associated peptide. Alternatively, arrays of small organic molecules can be employed as sequencable tags which encode the sequential synthetic history of the library member.

Finally, the structure of a member of the library can be directly determined by amino acid sequence analysis.

The following patents, describe methods of making random peptide or non-peptide libraries and screening such libraries to identify compounds that bind to target proteins. As used in the present invention, SI can be the targets used to identify the peptide and non-peptide ligands generated and screened as disclosed in the patents.

U.S. Patent Number 5,270,170 issued to Schatz et al. on December 14, 1993, and U.S. Patent Number 5,338,665 issued to Schatz et al. on August 16, 1994, refer to peptide libraries and screening methods which can be used to identify SI ligands.

U.S. Patent No. 5,395,750 issued to Dillon et al. on March 7, 1995, refers to methods of producing proteins which bind to predetermined antigens. Such methods can be used to produce SI ligands.

U.S. Patent No. 5,223,409 issued to Ladner et al. on June 29, 1993, refers to the directed evolution to novel binding proteins. Such proteins may be produced and screened as disclosed therein to identify SI ligands.

U.S. Patent No. 5,366,862 issued to Venton et al. on November 22, 1994, refers to methods for generating and screening useful peptides. The methods herein described can be used to identify SI ligands.

U.S. Patent No. 5,340,474 issued to Kauvar on August 23, 1994 as well as U.S. Patent No. 5,133,866, U.S. Patent No. 4,963,263 and U.S. Patent No. 5,217,869, can be used to identify SI ligands.

U.S. Patent No. 5,405,783 issued to Pirrung et al. on April 11, 1995, refers to large scale photolithographic solid phase synthesis of an array of polymers. The teachings therein can be used to identify SI ligands.

U.S. Patent No. 5,143,854 issued to Pirrung et al. on September 1, 1992, refers to a large scale photolithographic solid phase synthesis of polypeptides and receptor binding screening thereof.

U.S. Patent No. 5,384,261 issued to Winkler et al. on January 24, 1995, refers to very large scale immobilized polymer synthesis using mechanically directed flow patterns. Such methods are useful to identify SI ligands.

U.S. Patent No. 5,221,736 issued to Coolidge et al. on June 22, 1993, refers to sequential peptide and oligonucleotide synthesis using immunoaffinity techniques. Such techniques may be used to identify SI ligands.

U.S. Patent No. 5,412,087 issued to McGall et al. on May 2, 1995, refers to spatially addressable immobilization of oligonucleotides and other biological polymers on surfaces. Such methods may be used to identify SI ligands.

U.S. Patent No. 5,324,483 issued to Cody et al. on June 28, 1994, refers to apparatus for multiple simultaneous synthesis. The apparatus and method disclosed therein may be used to produce multiple compounds which can be screened to identify SI ligands.

U.S. Patent No. 5,252,743 issued to Barrett et al. on October 12, 1993, refers to spatially addressable immobilization of anti ligands on surfaces. The methods and compositions described therein may be used to identify SI ligands.

U.S. Patent No. 5,424,186 issued to Foder et al. on June 13, 1995, refers to a very large scale immobilized polymer synthesis. The method of synthesizing oligonucleotides described therein may be used to identify SI ligands.

U.S. Patent No. 5,420,328 issued to Campbell on May 30, 1995, refers to methods of synthesis of phosphonate esters. The phosphonate esters so produced may be screened to identify compounds which are SI ligands.

U.S. Patent No. 5,288,514 issued to Ellman on February 22, 1994, refers to solid phase and combinatorial synthesis of benzodiazepine compounds on a solid support. Such methods and compounds may be used to identify SI ligands.

As noted above, SI ligands may also be antibodies and fragments thereof. Indeed, .antibodies raised to unique determinants of these receptors will recognize that protein, and only that protein and, consequently, can serve as a specific targeting molecule which can be used to direct novel diagnostics and therapeutics to this unique marker. In addition, these antibodies can be used to identify the presence of SI or fragments there of in biological samples.

### Example 2: USE OF EXPRESSION PROFILING FOR IDENTIFYING MOLECULAR MARINERS USEFUL FOR DIAGNOSIS OF METASTATIC CANCER

Cancer represents a significant worldwide health problem. Cancer is an uncontrolled growth and spread of cells. For many cancers, metastasis to adjacent or distant tissues results in physiologic impairment and often death. Early diagnosis and the ability to diagnosis metastasis of primary tumors represent significant challenges in the effective treatment of neoplastic disease.

Stage at diagnosis is the single most important prognostic determinant for patients with cancer and dictates the role of adjuvant chemotherapy in this disease. Given the prognostic and therapeutic importance of staging, accurate histopathologic evaluation of lymph nodes to detect invasion by cancer cells is crucial. Specific diagnosis of cancer metastasis is currently preformed by histologic and cytologic resemblance to normal tissue. Cancer cells frequently maintain their phenotypic characteristics of their normal cell of origin.

However, conventional microscopic lymph node examination has methodological limitations. Differentiation of single or even small clumps of tumor cells from other cell types can be difficult, limiting sensitivity. The standard practice of examining only several tissue sections from each lymph node can omit from review >99% of each specimen, introducing sampling error. These limitations are evident when the frequency of recurrence in patients with stage I and II colorectal cancer is considered. By definition, these patients do not have extra-intestinal disease at the time of curative resection. However, recurrence rates of 10% to 30% for lesions confined to the mucosa (stage I) and 30% to 50% for lesions confined to the bowel wall (stage II) have been reported.

Alternative methods to detect small numbers of tumor cells have been applied to staging, including intensive review of serial tissue sections, PCR to detect tumor-specific mutations, immunohistochemistry or and RT-PCR to detect the expression of biomarkers that are specifically expressed in cells that have undergone neoplastic transformation (Sloane, 1995, Lancet 345: 1255-6; Abati and Liotta, 1996, Cancer 78: 10-66). In some colorectal cancer studies, staging by these sensitive methods has correlated with disease. However, the labor- and cost-intensity of serial sectioning, the lack of uniform association between mutations and neoplastic transformation, and the lack of specificity of many biomarkers limit the applicability of these methods.

Easily detected molecular markers that are uniformly expressed by larger numbers of metastasized tumor would therefore be useful for metastasis detection and disease staging. Particularly needed is methodology to isolate useful molecule markers for the detection of metastatic tumor cells in tissues and/or bodily fluids. Such methodology would ideally be high throughput and utilize established robust protocols.

One embodiment of the present invention relates to methods to identify and characterize molecular markers useful for detecting metastasized tumor cells. Most commonly, molecule markers used to detect tumor cells are transcripts or proteins specifically expressed as a result of the hyperproliferative state of the cell. In contrast, the molecular markers that are identified and characterized by the method of the present invention are specifically expressed in terminally differentiated tissues and are not specific to tumor cells. Tumor cells continue to express the genes associated with terminal differentiation of their tissue of origin. The transcripts and proteins of these genes are ideally suited to detect tumor cells that have metastasized to a destination tissue, such as a lymph node, because the origin tissue specific markers will be out of place in the destination tissue. Because these molecular markers are specific to the origin tissue and not a particular tumor, they will broadly recognize many tumors metastasized from the origin tissue.

The method for identifying molecular markers useful for detecting metastasized tumor cells identifies "candidate" tissue-specific molecule markers and determines which of these candidate markers are suitable for the detection of metastatic cancer. Tissue-specific markers associated with the terminal differentiation of a desired origin tissue are characterized by down-regulating the activity of a transcription factor associated with terminal differentiation of origin tissue, comparing the expression profiles of the down-regulated origin tissue with unaltered control origin tissue, and identifying transcripts or proteins that are candidate tissue-specific markers by virtue of their expression being up- or down-regulated in conjunction with the down-regulation of the transcription factor. The expression of the candidate tissue-specific markers are compared in the control origin tissue, tumors derived from the origin tissue, and destination tissues of interest for biopsy. Candidate markers that are expressed in control origin tissue and tumors, but not destination tissue are useful markers for detecting metastatic tumor cells.

As used herein, the term "terminal differentiation" refers to a differentiation state of a cell or tissue from which no further differentiation can occur.

The origin tissue of the invention is any terminally differentiated tissue of the body in which tumor cells first arise. By "arise", it is meant to confer to cells the hyperproliferative phenotype associated with tumor cells. The origin tissue is preferably a tissue from which cancer cells are most likely to metastasize. In a preferred embodiment, the tissue is mammalian, and in a most preferred embodiment, the tissue is human. In preferred embodiments, the origin tissue includes, but is not limited to, colorectal, intestine, stomach, liver, mouth, esophagus, throat, thyroid, skin, brain, kidney, pancreas, breast, cervix, ovary, uterus, testicle, prostate, bone, muscle, bladder and lung. It is particularly advantageous to use established cell lines in the method of the invention. The cell lines of particular interest represent terminally differentiated cells of the origin tissue, including embryonic tissue cell lines and immortalized cell lines (Yeager and Reddel, 1999, Curr. Opin. Biotechnology 10:465-469). Cell lines of particular interest include, but are not limited to, T84, Caco2, HT29, SW480, SW620, NCI H508, SW1116, SW1463, Hep G2, HS766T, and HeLa cells. These and additional cell lines of origin tissue may be obtained from the American Type Culture Collection (Manassas, VA), as well as from commercial sources.

Cancerous origin tissues are isolated from tumors that arise in the origin tissue. Cancerous cells may be obtained by removing tumors from patients. Established populations of tumor tissue, i.e. cell lines of tumor cells, can be used to advantage in the method of the invention. Cancer cell lines of interest include, but are not limited to, T84, Caco2, HT29, SW480, SW620, NCI H508, SW1116, SW1463, Hep G2, HS766T, and HeLa cells. These cell lines and other useful cell lines may be obtained from the American Type Culture Collection (Manassas VA), as well as from commercial sources.

The destination tissue of the invention is any tissue or bodily fluid that may be biopsied to detect metastasized tumor cells. Several tissues of the body are well known to those in the art for their propensity to accumulate metastasized tumor cells, and these tissues are preferred for the destination tissue. However, the destination tissue may be any tissue of the body. Destination tissues of particular interest include, but are not limited to, lymph node, blood, cerebral spinal fluid, and bone marrow. Additional cell lines for origin tissue cells may be obtained from the American Type Culture Collection (Manassas, VA), as well as from commercial sources. Preferably, biopsy or resected tissue is used as the destination tissue.

The transcription factors used in the method of the invention are transcription factors that are associated with terminal differentiation of the origin tissue. Many such transcription factors are already know to those skilled in the art. In preferred embodiments, the transcription factor is associated with the terminal differentiation of a preferred origin tissue. In preferred embodiments, the transcription factors include, but are not limited to, Cdx2 (intestine) (Mallo, G.V. et al., 1997 Int J Cancer 74:35-44; Genbank Accession No. BF591065), STAT5 (breast) (Hou, J. et al., 1995 Immunity 2:321-329; Genbank Accession No. L41142), NKX3.1 (prostate) (Genbank Accession No. AF247704), GBX2 (prostate) (Lin, X. et al., 1996 Genomics 31: 35-342; Genbank Accession No. NM U13219), FREAC-2 (lung) (Pierrou, S. et al., 1994 EMBO J. 13:5002-5012; Genbank Accession No. U13220), Pitl (thyroid) ( Wu, W. et al., 1998 Nat Genet 18:147-9; Genbank Accession No. NM 006261) HNF4 (liver) (Chartier, F.L. et al., 1994 Gene 147:269-272; Kritis, A.A. et al., 1996 Gene 173:275-80; Genbank Accession Nos. X76930, X87870, X87872, X87871), LFB1 (liver) (Bach, I. et al., 1990 Genomics 8:155-164; Genbank Accession No. NM 000545), IPF1 (pancreas) (Stoffel, M. et al., 1995 Genomics 28:125-126;Genbank Accession Nos. NM 000209, U30329), Isl1 (pancreas) (Wang, M. and Drucker, D.J., 1994 Endocrinology 134:1416-1422; Genbank Accession Nos. XM 003669, NM 002202) and MyoD (muscle) (Pearson-White, S.H., 1991 Nucleic Acids Res. 19:1148; Genbank Accession No. X56677).

The method of the present invention may, in some embodiments, further comprise steps to identify a transcription factor gene associated with terminal differentiation. These additional steps comprise identifying the transcription factor that binds to the regulatory regions of a gene associated with terminal differentiation in the origin tissue. There are many protocols currently available and known to those skilled in the art to characterized transcription factors and transcription factor genes. In a preferred embodiment, electromobility shift assays and/ or supershift assays are used to characterize the transcription factor that binds to the regulatory region of a gene whose expression is associated with terminal differentiation. Example 1 illustrates the characterization of transcription factor Cdx2 by its binding to the regulatory regions of the gene encoding the intestine-specific protein guanylyl cyclase C.

In the method of the invention, the activity of transcription factor associated with terminal differentiation is "down-regulated" in a population of origin tissue cells. By "down-regulated", it is meant that the activity of the transcription factor is reduced in the cell population as compared to a "normal" or control cell population. As used herein, a "cell population" refers to a cell culture, tissue culture, resected tissue or biopsy sample, or any group of cells from the desired tissue type. A population of normal or control origin cells refers is a population of origin cells from the culture of origin tissue cells used for down-regulating the transcription factor, but without modification of the activity of the transcription factor.

The activity of the transcription factor may be down-regulated in cell populations by several means well known to those in the art. In some embodiments, the transcription factor gene is down regulated by site-directed mutagenesis of the coding or regulatory regions of the gene, or the transcription of an antisense gene constructed from the coding sequence of the transcription factor gene. Alternately, in other embodiments, the activity of the transcription factor is blocked or inhibited by specific antibodies, DNA-binding molecules, or small molecules that interfere with the activity of the transcription factor by interfering with the assembly and/or initiation of the transcriptional complex. Inhibitor polynucleotide molecules of interest include, but are not limited to, FP1, FP1B and SIF1 (see Example 1). Finally, in other embodiments, the transcription factor may be down-regulated by activating a signaling event that inactivates the transcription factor, such as the addition of an extracellular ligand that initiates a cell-signaling event that phosphorylates and inactivates the transcription factor. These methods will be well known by those skilled in the art, and protocol can be found in many laboratory manuals, such as Ausubel et al. Current Protocols in Molecular Biology. New York: John Wiley & Sons, Inc., 2000. These embodiments are meant to illustrate methods by which to generate down-regulated origin cells. Other manners of down-regulation will be well known to those skilled in the art and are included in the scope of the method of the present invention.

In a preferred embodiment, the down-regulated origin cells are cdx2-null polyps. Cdx2-null polyps can be resected from a mouse that is heterozygous for an inactive copy of the homeobox gene cdx2, which controls cell differentiation in the intestinal epithelium (Chawengsaksophak et al., 1997, Nature 386:84-87; Tamai et al., 1999, Cancer Res. 59:2965-2970; Beck et al., 1999, PNAS 96:7318-7323; incorporated by reference herein). Cdx2 stimulates the markers of endocyte differentiation. These heterozygous mice develop multiple intestinal polyp-like lesions that do not express active Cdx2 and the Cdx2-related markers. In this embodiment, the comparison of the expression profiles of Cdx2-null polyps with surrounding intestinal tissue will identify the Cdx2 stimulated markers of endocyte differentiation.

The method of the invention comprises the step of comparing the expression profile of the population of down-regulated origin cells with the expression profile of the population of control origin cells. By "expression profile" it is meant the array of nucleic acids or proteins that are expressed in a cell population. Most commonly, expression profiles are arrays of nucleic acid molecules, primarily mRNA molecules, that are found in the profiled cell population. Methods to compare RNA expression profiles are well known to those in the art. Some methods of particular interest include, but are not limited to, differential display (Welsh et al., 1992, Nucleic Acids Res. 20:4695-4970; Liang and Pardee, 1992, Science 257:967-970; Barnes, 1994, Proc. Natl. Acad. Sci. USA 91:2216-2220; Cheng et al., 1994, Proc. Natl. Acad. Sci. USA 91: 5695-5699; and the references cited therein), subtractive hybridization (Diatchenko et al., 1996, Proc. Natl. Acad. Sci. USA 93:6025-6030; Gurskaya et al., 1996, Anal. Biochem. 240:90-97; Endege et al., 1999, Biotechniques 26: 542-550; and the references cited therein), expression arrays (Schena et al., 1995, Science 270: 467-470; Shalon et al., 1996, Genome Res. 6: 639-645; .Cheung et al., 1999, Nature Genetics 21(Suppl.): 15-19; and the references cited therein), Serial Analysis of Gene Expression (SAGE) (Velculescu et al., 1995, Science 270: 484-487; Zhang et al., 1997, Science 276: 1268-1272; Adams et al., 1996, Bioessays 18: 261-262; and the references cited therein), Rapid Analysis of Gene Expression (RAGE) (Wang et al., 1999, Nucleic Acids Res. 27: 4609-4618; and the references cited therein), Massively Parallel Signature Sequencing (MPSS) (Brenner et al., 2000, Nature Biotech. 18: 630-634; and references therein) and Tandem Arrayed Ligation of Expressed Sequence Tags (TALEST) (Spinella et al., 1999, Nucleic Acids Res. 27: e22 (I-VIII); and references therein).

Many of the aforementioned techniques may be preformed using commercially available kits, reagents and apparatuses. Commercial kits for differential display may be purchased, such as the Delta^{®} Differential Display Kit (Clontech, Palo Alto, CA), among others. Commercial kits for subtractive hybridization may be purchased, such as Clontech PCR-Select^{®} Subtraction (Clontech, Palo Alto, CA), among others. Micro-arrays of popular cDNA populations may be purchased (Incyte Genomics, Inc, St. Louis. MO), or custom micro-arrays may be ordered from commercial sources (Radius Biosciences, Medfield MA ; ProtoGene Laboratories, Inc., Menlo Park CA). A preferred membrane-format microarray is LifeGrid™ Sequence-Verified Gene Expression Array Kits (Incyte Pharmaceuticals, Inc., St. Louis, MO) and a preferred slide-format microarray is GEM^{®} Gene Expression Microarray (Incyte Pharmaceuticals, Inc., St. Louis, MO). Commercial kits for RAGE are available from Kirkegaard & Perry Laboratories, Inc. (Gaithersburg, MD). GeneTag^{®}, a proprietary technology developed by Celera Genomics (Roch-ville, MD), may also be used to quantify gene expression in a profile of RNA transcripts.

Protein expression profiles may also be compared by methods that will be well known to those in the art. Methods of particular interest include, but are not limited to, 2-Dimensional Electrophoresis - Mass Spectroscopy (2DE-MS) (O'Farrell, 1975, J. Biol. Chem. 250: 4007-4021; Patterson and Aebersold, 1995, Electrophoresis 16: 1791-1814; Gygi et al., (2000) Curr. Opinion in Biotech. 11: 396-401; and refemces cited therein) and Isotope-Coded Affinity Tags (ICAT) (Gygi et al., 1999, Nature Biotech. 17: 994-999; Gygi et al., 2000, Curr. Opinion in Biotech. 11: 396-401; and references cited therein).

Nucleic acid molecules or protein molecules of interest identified by the comparison of expression profiles may additionally be isolated using methods that will be well known to those skilled in the art. The isolation method chosen depends in many cases on the method used to compare the expression profiles, and the preferred method will often be described in the reference that describes the method of comparison (see aforementioned citations). For example, nucleic acid bands may be removed from a polyacrylamide gel, agarose gel or nitrocellulose, the nucleic acids eluted and cloned using techniques well known in the art (Ausubel et al. Current Protocols in Molecular Biology. New York: John Wiley & Sons, Inc., 2000).

The method of the invention comprises the step of comparing the expression of the candidate markers in several kinds of cells. There are many methods to compare the expression of single genes which will be well know to those in the art (Ausubel et al. Current Protocols in Molecular Biology. New York: John Wiley & Sons, Inc., 2000), including but not limited to, northern analysis, Southern analysis with cDNA, RNase protection assays, quantitative PCR, competitive PCR, 5' nuclease assays (Lie and Petropoulos, 1998, Curr. Opin. Biotech. 9:43-48 and the references cited therein), western analysis, dot blot western, ELISA and other immunoassays, and immunohistochemistry.

The molecular markers identified by the method of the invention may be used to diagnose and stage cancer in mammalian patients, including following the development of recurrence of cancer after surgery and screening normal patients for the development of cancer. In the case of cancer patients, the molecular markers utilized would be identified ideally from the same tissue that the patients cancer arose. In the case of patients without a history of cancer, a selection of molecular markers isolated from different origin tissues is preferred. The metastases may be diagnosed by any technique that will detect the nucleic acid or protein molecular marker. The sensitively of the technique will determine in part the size of metastasis that can be detected. Preferred techniques utilize PCR, ELISA, and the like. Example 2 illustrates a particularly preferred method to diagnose metastasized cancer with the molecular markers of the method.

Tissue specific molecular markers can also be utilized to localize therapeutics to specific tissue and organ systems. This use is particularly appropriate for tissue-specific molecular markers that are localized on the surface of the tissue cells. These therapeutics include, but are not limited to, chemotherapeutics, analgesics, antibiotics, anti-inflamatories, hormones and stimulants.

Protein molecular markers may be used to generate antibodies that may be used in diagnosis method and to localize therapeutics. Polyclonal antibodies and monoclonal antibodies, and fragments thereof, and various conjugates of them can be made by methods well known in the art.

### Example 3 Cdx2 is a Transcription Factor Associated with the Intestinal-Specific Expression of Guanylyl Cyclase C

This illustrates the identification of a transcriptional activating factor required for intestine-specific expression of guanylyl cyclase C (GC-C). A region of the proximal GC-C promoter required for specific expression in intestinal cells that contains a protected region, FP1, with a consensus binding sequence for Cdx2. FP1 formed a complex specifically with nuclear proteins only from intestinal cells, and this complex was recognized by anti-Cdx2 antibody. Elimination or mutation of the Cdx2 consensus binding sequence within FP1 reduced reporter gene activity in intestinal cells to that obtained in extra-intestinal cells. These data suggest that Cdx2 activates tissue-specific transcription of GC-C.

### Materials and Methods

**Genomic Library Screening and Sequencing**. The GC-C gene 5' regulatory region was cloned from a λFLXII human genomic library (Stratagene, La Jolla CA). The library was screened by hybridization with a probe specific for exon 1 of the guanylyl cyclase C (GC-C) cDNA. A 2.8 kb XbaI fragment that included 2 kb upstream of the start site of transcription was subcloned into Bluescript KS (Stratagene). All constructs were generated from this Bluescript/human GC-C gene construct. The nucleic acid sequence of each construct was confirmed by BigDye terminatot^{®} reaction chemistry for sequence analysis on the Applied Biosystems Model 377 DNA sequencing systems (Perkin-Elmer, Norwalk CN; Applied Biosystems, Foster City CA).

**Reporter Gene Constructs**. Fragments -835 to +117, -257 to +117, -129 to +117, and -46 to +117, relative to the start site of transcription, were isolated from Bluescript KS constructs by digestion with selected restriction endonucleases (Mann et al., 1996, Biochim Biophys Acta 1305:7-10). These fragments were blunt-ended and ligated into the EcoRV site of Bluescript KS. Inserts were excised from Bluescript KS with SmaI and KpnI and ligated into the pGL3-Basic Luciferase Vector (Promega, Madison WI). The pGL3 Control Vector containing an SV40 promoter with enhancers, was used as a positive control.

Mutations were created in the -835 to +117 pGL3 construct utilizing the PCR-based Ex-site Mutation Kit (Stratagene). Deletion constructs were created using primers flanking the sites of interest. The FP 1 "GCC" mutant was created using the phosphorylated primers:
5' GCCCATAGCTCTGACCTTTCTG 3' (SEQ ID NO:7) and
5' AGAGAGATTAGCTGGGCCTCACCC 3'(SEQ ID NO:8).

**Cell Culture and Transfection**. All cell lines were obtained from American Type Culture Collection (Rockville, MD). T84 cells were grown in DMEM/F12 (Life Technologies, Rockville MD), Caco2 cells in DMEM (Life Technologies), HepG2 and HS766T cells in DMEM High Glucose (Cellgro^{®}, Mediatech, Inc., Herndon VA ), and HeLa cells in MEM with glutamine (Life Technologies). All cell lines were maintained at 37°C in a 5% CO₂95% air atmosphere and passaged every four days. Assays of reporter gene activity were conducted with cells plated in 6-well seeded at either 5.0 x 10⁵ (T84, Caco2, and HeLa) or 1.0 x 10⁶ cells per well (HepG2 and HS766T). Cells were incubated overnight, washed one time with PBS, and supplemented with fresh media before transfection.

Plasmids purified with the Qiafilter Kit (Qiagen, Valencia CA) were transfected into cells with the non-liposomal lipid transfection reagent Effectene^{®} (Qiagen). All cell lines were co-transfected with both 0.4 mg of firefly luciferase experimental reporter constructs, modified from pGL3-Basic, and 0.1 mg of the *Renilla* luciferase control reporter, pRL-TK, driven by a viral thymidine kinase promoter (Promega). Cells were incubated with transfection complexes for 24 h, rinsed with PBS, then supplemented with appropriate media and incubated for a further 24 h. After a total of 48 h, cells were lysed and assayed using the protocol and materials in the Dual-Luciferase Reporter Assay system (Promega). Luminesence was measured with a BioOrbit 1251 Luminometer (Pharmacia LKB, Uppsala Sweden). Luciferase expression from pGL3 constructs was normalized to pRL-TK expression.

**Nuclear Protein Extraction**. Nuclear extracts were prepared essentially as previously described (Ausubel et al. Current Protocols in Molecular Biology. New York: John Wiley & Sons, Inc., 2000). Nuclear protein concentration was determined using Coomassie Protein Assay Reagent (Pierce, Rockford IL).

**DNAse I Footprinting**. A fragment of the GC-C gene regulatory region -46 to -257 relative to the start of transcription was obtained by digestion with Drain and AflII, blunt-ended, and subcloned into the Bluescript^{®} KS EcoRV site, as described above, and then digested with EcoRI and HinDIII to ensure that the coding strand of the probe was singly end-labeled with [α-32P]dCTP. Products obtained from footprinting reactions were separated on a denaturing 6% polyacrylamide gel and visualized by a Phosphorimager SI (Molecular Dynamics, Sunnyvale, CA).

**Electromobility Shift Assay (EMSA)**. Protein-DNA binding reactions performed in the same buffer as the DNase I protection assay (4% glycerol, 10 mM Tris-HCl (pH 7.5) 50 mM NaCl, 2.5 mM MgCl₂ and 5 mM DTT) included 1 mg of Poly(dl-dC)-Poly(dI-dC) (Amersham Pharmacia Biotech, Piscataway, NJ) and 30 kcpm of probe. Reactions were initiated by the addition of nuclear extract and incubated for 30 min at room temp to produce protein complexes which were separated on a 6% non-denaturing, polyacrylamide (37.5:1) gel in 0.5 x TBE running buffer. Gels were dried prior to visualization of radiolabelled complexes by autoradiography. In competition assays, unlabelled competitor was added to the reaction mixtures at concentrations ranging from 25-fold to 250-fold molar excess of the labeled probe prior to the addition of the nuclear extract. Supershift assays were performed by adding 2 ml of murine Cdx2 antibody after an initial incubation period of 30 min; incubation was then continued for an additional 30 min. Transcribed and translated murine Cdx2 protein was generated *in vitro* using linearized pRc/CMV-Cdx2 expression vector as a template for the TNT-Quickcoupled Kit (Promega)..

Oligonucleotide probes for EMSA were synthesized. Complementary oligonucleotides in 10 mM Tris-HCl (pH 7.5), 1mM EDTA were annealed in a Hybaid Thermal Cycler by a programmed ramp in temp from 95°C to 25°C over the course of 1 h. The single stranded sequences of the probes were:
FP1: 5' CAGCTAATCTCTC**TGTTTATAG**CTCTGACCTTTC 3'(SEQ ID NO:9)
FP1B: 5' ATCTCTC**TGTTTATAG**CTCTGACCTTTCTGGGTGC 3'(SEQ ID NO: 10)
FP1-CCC: 5' CAGCTAATCTCTCTGCCCATAGCTCTGACCTTTC3'(SEQID NO: 11)
SIF1: 5' GATCCGGCTGGTGAGGGTGC**AATAAAACTTTATG**AGTA 3'(SEQ ID NO: 12)

Bolded sequences indicate specific Cdx2 binding sites. A mutation created in the FP1 protected site is underlined. Five pmol of annealed oligonucleotide probe were end-labeled employing 1 unit of T4 polynucleotide kinase and 2 inl of 7,000 Ci/mmol [γ-³²p]ATP (Ausubel et al. Current Protocols in Molecular Biology. New York: John Wiley & Sons, Inc., 1999). Labeled probes were purified over Qiaquick nucleotide purification columns (Qiagen).

**Southwestern and Western Blotting**. Nuclear extracts were denatured in reducing SDS sample buffer, separated on an 8% Tris-glycine-SDS polyacrylamide gel_{;} and transferred to nitrocellulose. For Southwestern analysis, the blotted proteins were blocked for 1 h at 4° in Z' buffer (25 mM Hepes-KOH (pH 7.6), 12.5 mM MgC₁₂, 20 % glycerol, 0.1 % Nonidet P-40, 100 mM KCl, 10 mM ZnSO4, 1 mM DTT) containing 3% non-fat dry milk (Hames and Higgins. Gene Transcription: A Practical Approach. The Practical Approach Series. New York: Oxford University Press, 1993.). The membrane was rinsed for 5 min in EMSA binding buffer and hybridized with 20 ml of EMSA binding buffer with 100 kcpm/ml of labeled FP1 probe for 1 h at room temp. The membrane was then washed for 5 min each in three changes of EMSA binding buffer, dried and visualized by autoradiography.

Western blots were blocked in TBS/0.1% Tween-20 with 5% non-fat dry milk, and probed with Cdx2 antibody diluted 1:5000. Binding of primary antibody was visualized using goat anti-rabbit alkaline phosphatase-conjugated secondary antibody diluted 1:10,000 (Sigma). Alkaline phosphatase substrates BCIP and NBT were used in an AP Color Kit (Biorad).

### Results

**Determination of elements controlling intestine-specific expression in the 5' regulatory region of the GC-C gene**. Minimal luciferase activity was obtained when various cell lines were transfected with the -46 construct (Fig. 1). In contrast, luciferase activity increased in intestinal cells transfected with each of the other reporter gene constructs (Fig. 1). Luciferase activity did not increase when extra-intestinal cells were transfected with these constructs (Fig. 1). These results are consistent with previous studies of GC-C gene regulation, and suggest that there are one or more tissue-specific regulatory elements within the +118 to -257 region. 12 Since transfection with the -46 to -129 construct resulted in a significant increase in activity of the reporter gene in intestinal cells only (Fig. 1), and since this region is highly conserved evolutionarily, it was chosen for detailed structure-function analysis.

**DNAse I protection by intestine-specific nuclear protein binding to the 5' regulatory region of GC-C**. DNAse I protection assay revealed two regions (-75 to -83, FP1; -164 to -178, FP3) which were protected only by nuclear extracts from intestinal cells (T84; Fig. 2). Regions -104 to -137 (FP2) and -180 to -217 (FP4) were protected by nuclear extracts from either intestinal (T84) or extra-intestinal (HepG2) cells, although the proximal and distal ends of FP2 exhibited different patterns of protection. These data suggest that the protected regions designated FP1 and FP3 were specific binding sites for nuclear proteins from intestinal cells. In addition, an intestine-specific site of open chromatin structure in the proximal 5'-flanking region of the GC-C gene was identified by a DNAse I hypersensitive site at base -163 (Fig. 2).

**Transcriptional activity of the -857 construct following deletion of FP1 or FP3**. Transfection of T84 cells revealed that deletion of FP3 increased luciferase activity 2.5-fold relative to the wild-type construct (Fig. 3). In contrast, elimination of FP1 reduced luciferase activity in T84 cells to levels observed in HepG2 cells (Fig. 3). These data suggest that FP3 contains a negative regulatory element, and that FP 1 contains an intestine-specific positive regulatory element. Analysis by TRANSFAC (Heinemeyer et al., 1998, Nucleic Acids Res. 26: 364-370), a database of transcription factor binding sites, revealed that FP1 contains the consensus binding site for the homeodomain protein Cdx2 (Quandt et al., Nucleic Acids Res 1995; 23:4878-84). Since Cdx2 is a transcription factor that directs intestine-specific expression of several genes, FP1 was more closely examined (Traber and Silberg, 1996, Annu Rev Physiol 58:275-97).

**Specific complexes are formed by intestinal nuclear extract and FP1 probe**. The ability of the protected site FP1 to form intestine-specific complexes was determined by incubating an oligonucleotide probe with nuclear extracts prepared from T84, Caco2, HepG2, or HeLa cells. Indeed, several complexes were obtained by EMSA when the FP 1 probe was incubated with nuclear extracts from those cells (Fig. 4). However, only one complex satisfied criteria for intestinal specificity, including formation by nuclear extracts from T84 and Caco2 cells, but not from HepG2 or HeLa cells. Extracts from T84 and Caco2 cells, but not from HepG2 or HeLa cells, also formed complexes with SIF1 that were identical to those obtained previously with that probe, demonstrating the integrity of the extracts (Suh et al., 1994, Mol Cell Biol 14:7340-51). All of the EMSA complexes formed with T84 nuclear extracts were competed with increasing amounts of unlabelled FP1 probe in a concentration-dependent manner. In contrast, an unlabelled competitor in which the Cdx2 binding site was specifically mutated (FP1-CCC probe, see Materials and Methods) did not compete against the intestine-specific complex. SIF1, an oligonucleotide containing two consensus binding sites for Cdx2, selectively prevented the formation of the FP1-dependent intestine-specific complex with greater potency than unlabelled FP1, but generally did not affect the binding of the remaining T84-EMSA complexes (Suh et al., 1994). These data suggest that the intestine-specific factor that binds to the FP 1 protected site is most likely Cdx2.

**Cdx2 binds specifically to the FP1 probe.** To determine whether FP1 is a binding site for Cdx2, labeled FP1 was incubated with in vitro transcribed and translated murine Cdx2. This resulted in a complex whose mobility was identical to the intestine-specific complex formed by T84 nuclear extract. In contrast, labeled FP1-CCC did not form the intestine-specific complex with either Cdx2 or T84 nuclear extract. An antibody against Cdx2 decreased the mobility of the specific complex formed between labeled FP1 and either T84 nuclear extract or *in vitro* transcribed and translated Cdx2. In contrast, an antibody against a related homeodomain transcription factor, Cdx1, did not alter the mobility of the intestine-specific complex. These data lead to the conclusion that the FP 1 protected site is a binding site for Cdx2.

**Identification of the intestine-specific nuclear factor by Southwestern and Western blots.** Whether the FP1 probe and anti-Cdx2 antibody bound to the same intestine-specific protein was examined. Labeled FP1B, which is highly homologous to FP 1 probe, specifically bound to an intestine-specific protein of~40 kDa in T84 and Caco2, but not HepG2, nuclear extracts. In addition, FP1B probe bound to a~131 kDa protein present in all cell lines examined. Similarly, anti-Cdx2 antibody recognized a protein doublet of~40 kDa expressed in T84, but not in HepG2 or HeLa, cell nuclear extracts, a pattern which is characteristic of Cdx2 (James et al., 1994, J Biol Chem 269:I5229-37). Thus, the FP1 protected region binds to an intestine-specific factor of the same molecular weight and antigenic recognition as Cdx2. Furthermore, Southwestern blots revealed that FP1 probe binds directly to Cdx2.

**Role** of **the Cdx2 binding element (FP1) in intestine-specific gene expression of the GC-C promoter.** The 'CCC' mutation was introduced into the FP1 element of the -835 luciferase reporter gene construct. This mutated reporter gene construct exhibited reduced activity in T84 cells that was comparable to the construct from which the entire FP1 region was deleted (Fig. 5). Neither the FP1 deletion nor the 'CCC' mutation in FP1 altered luciferase expression in HepG2 cells (Fig. 5). These data demonstrate that an intact Cdx2 binding site is required for activity of the GC-C promoter. Indeed, disruption of the Cdx2 binding site resulted in minimal activity.

### Example 4 Guanylyl Cyclase C Messenger RNA is used as a Molecular Marker to Detect Recurrent State II Colorectal Cancer

This example illustrates the use of a tissue-specific molecule marker to diagnosed metastases. Detection of GCC mRNA by RT-PCR enhances the accuracy of colorectal cancer staging. The expression in lymph nodes of GCC mRNA, a molecular marker for colorectal cancer cells in extraintestinal tissues, is associated with disease recurrence in patients with histologically negative nodes (stage In. Expression of GCC mRNA reflects the presence of colorectal cancer micrometastases below the limit of detection by standard histopathology. GCC-specific RT-PCR can reliably and reproducibly detect a single human colorectal cancer cell (T84 cells, ATCCC, Rockville, MD) in 10⁷ nucleated blood cells (Carrithers et al., 1996, Proc Natl Acad Sci USA, 93:14827-32).

GCC, a member of the guanylyl cyclase family of receptors, is specifically expressed only in intestinal mucosal cells. However, GCC expression persists in intestinal cells that undergo neoplastic transformation to colorectal cancer cells. Examination of >300 surgical specimens demonstrated that GCC was specifically expressed by all primary and metastatic colorectal cancer cells, but not by any other extraintestinal tissues or tumors. GCC is identified only in lymph nodes from stage II patients who suffered recurrence ≤3 y, but not in lymph nodes from patients without recurrent disease 6 y, following diagnosis.

### Materials and Methods

**Patients and tissues**. The Thomas Jefferson University Hospital tumor registry database was examined for patients who had undergone treatment for colorectal cancer between 1989 and 1995, an interval permitting adequate follow-up of patients for this study. This initial search was designed to exclude patients with recurrent disease >3 y following index surgery to avoid inadvertent inclusion of patients with metachronous, rather than recurrent, cancer. This search yielded 445 patients with invasive colon or rectal carcinoma with no evidence of metastases (N₀M₀) at the time of surgery. Of these, 260 patients underwent surgery at Thomas Jefferson University that yielded lymph nodes. Subsequently, 167 patients were excluded because they had TNM stage I disease or less (T₀, T₁ or T₂N₀M₀), developed recurrent disease locally or at unspecified sites, or received neoadjuvant chemo- or radiotherapy. Fifty-six patients with no evidence of recurrence were then excluded because they had <6 y of follow up. After these exclusions, a total of 18 patients with no evidence of disease for ≥6y following surgery and considered clinically cured remained. These patients formed the control group. Similarly, all 19 patients who developed metastases ≤3 y following surgery were included in the case group. Sixteen patients in the control group and 12 patients in the case group had pathology specimens available for further analysis. Two patients in the control group (patients 9 and 16; 12.5%) and 1 patient in the case group (patient 24; 8.3%) received 5-fluorouracil-based adjuvant chemotherapy following surgery.

**Reverse transcriptase-polymerase chain reaction**. Preliminary studies demonstrated that mRNA isolated from 10 µm sections from individual lymph nodes yielded insufficient RNA for RT-PCR analyses. Consequently, at least five 10 µm sections of representative lymph nodes for each patient were pooled and de-paraffinized, and the total RNA isolated (Waldman et al. 1996, Dis Colon Rectum 41:1-6.). RT-PCR was performed employing RNA PCR kit ver.2 (Takara Shuzo Co., Ltd., Kyoto, Japan; Carrithers et al., 1996, Proc Natl Acad Sci USA 93:14827-32; Waldman et al., 1996, Dis Colon Rectum 41:1-6). Only total RNA that yielded amplicons following β-actin-specific RT-PCR was employed in studies outlined below. GCC-specific and nested carcinoembryonic antigen-specific RT-PCR was performed as described previously (Carrithers et al., 1996, Proc Natl Acad Sci USA 93:14827-32; Waldman et al., 1996, Dis Colon Rectum 41:1-6; Liefers et al., 1998, New Engl J Med 1998;339:223-8). RT-PCR reactions were separated by electrophoresis on 4 % NuSieve 3:1 agarose^{®} (FMC Bioproducts, Rockland, ME) and amplification products visualized by ethidium bromide. Positive controls, consisting of RNA isolated from human colorectal cancer cells expressing GCC and carcinoembryonic antigen (Caco2 cells; American Type Culture Collection, Rockville, MD) and negative controls, consisting of incubations in which no template was added and RNA from lymph nodes devoid of colorectal cancer, were included. Amplicon identity was confirmed by sequencing. Production of GCC-specific amplicons was confirmed by Southern analysis, employing a ³²P-labeled antisense probe complimentary to a sequence internal to primers used for amplification (Kroczek, 1993, J Chromatog 618:133-145).

**Statistical analysis**. Results are expressed as the mean ± SD except disease-free and overall survival, which are expressed as the median ± range. P values were calculated using Fisher's Exact test. The odds ratio with exact 95% confidence interval (CI) was calculated employing the StatXact 4.0 statistical software package (CYTEL Software Corp., Cambridge, MA).

### Results

**Characteristics of patients evaluated by RT-PCR**. The age of patients ranged from 37 to 85 y (68.1 ± 9.5 y). The ages of females (range = 52 - 85 y; 64.5 ± 10.5 y) and males (range = 37 - 82 y; 70.9 ± 7.8 y) were similar. The ratio of males to females was balanced between control (8:9) and case (5:7) groups. One female patient was African-American; all other patients were Caucasian. The ratio of T₃ to T₄ disease was 3:13 in the control group and 4:8 in the case group. Patients were followed for 9 to 105 months (67.4 ± 30.7 months). Patients in the control group were followed for 73 to 105 months (89.9 ± 7.8 months) while those in the case group were followed for 9 to 78 months (37.3 ± 22.6 months). In the control group, one patient (6.3%) developed a new primary colonic lesion 96 months after initial diagnosis, one (6.3%) died of causes unrelated to colorectal cancer, and the remaining 14 (87.5%) were alive and free of disease 88 (range, 73-97) months following diagnosis. In the case group, 8 (66.6%) patients died of recurrent colorectal cancer following intervals of disease-free and overall survival of 13 (range, 3-35) and 19 (range, 9-64) months, respectively. Four (33%) were alive with metastases following intervals of disease-free and overall survival of 12 (range, 2-36) and 52 (range, 17-78) months, respectively.

**RT-PCR analysis of RNA expression in lymph nodes**. For the 28 patients in the control and case groups, a total of 524 (18.4 ± 12.5 lymph nodes/patient) lymph nodes collected at surgery were reported free of tumor by histologic review. The number of lymph nodes obtained from each patient at the time of initial operative staging was similar between control (19.9 ± 13.2) and case (17.2 ± 12.7) groups. Twenty-one patients (75%) yielded 159 paraffin-embedded lymph nodes (7.6 ± 5.2 lymph nodes/patient) that could be adequately evaluated by RT-PCR. Lymph nodes omitted from RT-PCR analysis were not available from pathology (326 lymph nodes from 28 patients; 62.2% of 524 lymph nodes obtained at surgery) or did not yield RNA (39 lymph nodes from 7 patients; 7.4% of 524 lymph nodes obtained at surgery; 19.7% of 198 lymph nodes available for RT-PCR analysis). The number of lymph nodes available for RT-PCR analysis was balanced between control (6.4 ± 3.0) and case (8.1 ± 6.3) groups.

β-Actin-specific amplicons (an indicator of intact RNA) were not detected in total RNA from pooled sections of lymph nodes of 5 (41.7%) patients from the case group and 2 (16.7%) patients from the control group and these patients were excluded from further analysis. Total RNA extracted from pooled lymph node sections from the remaining 21 patients was analyzed by RT-PCR using GCC-specific primers. GCC-specific amplicons were not detected in any reaction using RNA from lymph nodes of patients in the control group (p=0.004; Table 1). The absence of GCC-specific amplicons in these reactions was confirmed by Southern analysis and suggests the absence of colorectal cancer micrometastases in lymph nodes of patients free of disease. In contrast; GCC-specific amplicons were detected in all reactions using RNA from lymph nodes of patients in the case group (Table 1). The presence of GCC-specific amplicons in these reactions was confirmed by sequencing and/or Southern analyses and suggests the presence of colorectal cancer micrometastases in lymph nodes of patients with recurrent disease. Of note, GCC mRNA was not expressed in any of 39 lymph nodes from 21 other patients without colorectal cancer (negative controls) that have been analyzed by RT-PCR to date.

**Table 1. GCC mRNA expression in lymph nodes and patient outcome.**

| **Patient** | **GCC mRNA*** | **DFI^{†}** | **OS^{§}** | **Vital Status** |
|---|---|---|---|---|
| **Controls** | | | | |
| 6 | (-) | 97 | 97 | Alive, NED^{¶} |
| 7 | (-) | 96 | 105 | Alive, New 1° Colon Cancer (T₃N₁M₁) |
| 8 | (-) | 96 | 96 | Alive |
| 9 | (-) | 82 | 82 | Alive |
| 10 | (-) | 86 | 86 | Died of Dehydration |
| 11 | (-) | 89 | 89 | Alive |
| 12 | (-) | 94 | 94 | Alive |
| 13 | (-) | 87 | 87 | Alive |
| 14 | (-) | 86 | 86 | Alive |
| 15 | (-) | 87 | 87 | Alive |
| 16 | (-) | 73 | 73 | Alive |

| **Cases** | | | | |
|---|---|---|---|---|
| 17 | (+) | 13 | 15 | Dead 2° to Liver Metastases |
| 18 | (+) | 15 | 52 | Dead 2° to Liver Metastases |
| 19 | (+) | 3 | 9 | Dead 2° to Liver Metastases |
| 20 | (+) | 14 | 20 | Dead 2° to Liver Metastases |
| 21 | (+) | 2 | 78 | Alive with Liver Metastases |
| 22 | (+) | 12 | 25 | Alive with Liver Metastases |
| 23 | (+) | 9 | 55 | Dead 2° to Lung and CNS Metastases |
| 24 | (+) | 29 | 64 | Alive with Lung and Bone Metastases |
| 25 | (+) | 17 | 19 | Dead 2° to Liver, Lung and Bone Metastases |
| 26 | (+) | 11. | 17 | Alive with Lung Metastases |

| | | | | |
|---|---|---|---|---|
| *GCC mRNA detected (+) or absent (-) in lymph nodes. ^{†}disease-free interval (months after diagnosis). ^{§}Overall Survival (months after diagnosis). ^{¶}NED, no evidence of disease. | | | | |

Carcinoembryonic antigen is a glycoprotein expressed by <60% of colorectal cancers and by other tumors, normal cells, and in some non-malignant pathological conditions. RT-PCR analysis of carcinoembryonic antigen expression has been suggested to be a marker of colorectal cancer micrometastases in lymph nodes. In the present study, total RNA extracted from pooled lymph node sections was analyzed by RT-PCR using carcinoembryonic antigen-specific primers (Liefers et al., 1998, New Engl J Med 339:223-8). Nested RT-PCR failed to yield CEA-specific amplicons in reactions using total RNA from patients in the control group, but detected carcinoembryonic antigen-specific amplicons in 1 patient in the case group. The presence of carcinoembryonic antigen-specific amplicons was confirmed by sequence analysis.

**GCC mRNA expression in lymph nodes and clinicopathological prognostic indicators**. Case and control groups (28 patients) were compared for tumor and disease characteristics associated with disease recurrence. Groups appeared balanced with respect to: tumor grade (well differentiated: control, 2 (12.5%); case, 1 (8.3%); moderately differentiated: control, 13 (81.3%); case, 9 (75%); poorly differentiated: control, 1 (8.3%); case, 2 (12.5%); tumor size (control, 5.7 ± 2.3 cm; case, 4.8 ± 1.7 cm); tumor location (right colon: control, 7 (43.8%); case, **4** (33.3%); transverse colon: control, 3 (18.8%); case, 0; sigmoid colon: control, 5 (31.3%); case, 8 (66.6%); rectum: control, 1 (6.3%), case, 0); and depth of penetration and extension into pericolic fat of tumors. Angiolymphatic invasion was observed in 3 patients in the case group but not in patients in the control group, reflecting a likely mechanism underlying metastasis in the former. Expression of GCC mRNA in lymph nodes was associated with disease recurrence in all cases (p=0.004). The odds ratio for mortality associated with GCC mRNA expression in regional lymph nodes was 16.5 (1.1 - 756.7, 95% CI). Sensitivity analysis demonstrated that an incremental "false negative" (death of a patient in the control group) or "false positive" (survival of a patient in the case group) result would yield an odds ration with a 95% confidence interval encompassing 1 (no excess risk), reflecting the limitations of the small sample population employed in this analysis.

## Claims

1. An *in vitro* method of screening an individual for metastatic colorectal cancer cells comprising the steps of examining a sample of extraintestinal tissue and/or body fluids from an individual to determine whether CDX2 is being expressed by cells in said sample wherein expression of said CDX2 indicates a possibility of metastatic colorectal cancer cells in said sample.

2. The method of claim 1 wherein expression of said CDX2 by said cells is determined by detecting the presence of CDX2 mRNA.

3. The method of claim 1 wherein expression of said CDX2 by said cells is determined by polymerase chain reaction wherein said sample is contacted with primers that selectively amplify mRNA or cDNA generated therefrom.

4. The method of claim 1 wherein expression of said CDX2 by said cells is determined by immunoassay wherein said sample is contacted with antibodies that specifically bind to CDX2 protein.

5. The method of any of claims 1-4 wherein said sample is body fluid.

6. The method of any of claims 1-4 wherein said sample is blood.

7. The method of any of claims 1-4 wherein said sample is lymphatic tissue and/or fluid.

8. The method of any of claims 1-4 wherein said sample is a lymph node sample.

9. The method of any of claims 1-4 wherein the individual has previously been diagnosed with having colorectal cancer.

10. The method of any of claims 1-4 wherein the individual has previously been diagnosed with and treated for colorectal cancer.

11. An *in vitro* method of screening an individual for metastatic colorectal cancer cells comprising the steps of examining a sample of extraintestinal tissue and/or body fluids from an individual to determine whether CDX2 mRNA or protein is present in said sample wherein the presence of CDX2 mRNA or protein in said sample indicates that the individual may have metastatic colorectal cancer cells in said sample.

12. The method of claim 11 comprising the steps of examining a sample of extraintestinal tissue and/or body fluids from an individual to determine whether CDX2 mRNA is present in said sample.

13. The method of claim 12 wherein the presence of CDX2 mRNA is determined by polymerase chain reaction wherein said sample is contacted with primers that selectively amplify CDX2 mRNA or cDNA generated therefrom.

14. The method of claim 11 wherein the presence of CDX2 protein is determined by immunoassay wherein said sample is contacted with antibodies that specifically bind to CDX2 protein.

15. The method of any of claims 11-1 wherein said sample is body fluid.

16. The method of any of claims 11-15 wherein said sample is blood.

17. The method of any of claims 11-15 wherein said sample is lymphatic tissue and/or fluid.

18. The method of any of claims 11-15 wherein said sample is a lymph node sample.

19. The method of any of claims 11-15 wherein the individual has previously been diagnosed with having colorectal cancer.

20. The method of any of claims 11-15 wherein the individual has previously been diagnosed with and treated for colorectal cancer.

## Patentansprüche

1. *In* vitro-Verfahren zum Screenen eines Individuums auf metastatische kolorektale Krebszellen, umfassend die Schritte des Untersuchens einer Probe von extraintestinalem Gewebe und/oder Körperflüssigkeiten von einem Individuum, um zu bestimmen, ob CDX2 durch Zellen in der Probe exprimiert wird, wobei die Expression von dem CDX2 eine Möglichkeit von metastatischen kolorektalen Krebszellen in der Probe anzeigt.

2. Verfahren nach Anspruch 1, wobei die Expression des CDX2 durch die Zellen durch Nachweisen der Gegenwart von CDX2-mRNA bestimmt wird.

3. Verfahren nach Anspruch 1, wobei die Expression von dem CDX2 durch die Zellen durch Polymerasekettenreaktion bestimmt wird, wobei die Probe mit Primern in Kontakt gebracht wird, welche die mRNA oder davon erzeugte cDNA selektiv amplifizieren.

4. Verfahren nach Anspruch 1, wobei die Expression des CDX2 durch die Zellen durch ein Immunoassay bestimmt wird, wobei die Probe mit Antikörpern in Kontakt gebracht wird, welche spezifisch an CDX2-Protein binden.

5. Verfahren nach einem der Ansprüche 1 bis 4, wobei die Probe Körperflüssigkeit ist.

6. Verfahren nach einem der Ansprüche 1 bis 4, wobei die Probe Blut ist.

7. Verfahren nach einem der Ansprüche 1 bis 4, wobei die Probe Lymphgewebe und/oder -flüssigkeit ist.

8. Verfahren nach einem der Ansprüche 1 bis 4, wobei die Probe eine Lymphknotenprobe ist.

9. Verfahren nach einem der Ansprüche 1 bis 4, wobei das Individuum zuvor diagnostiziert wurde, kolorektalen Krebs zu haben.

10. Verfahren nach einem der Ansprüche 1 bis 4, wobei das Individuum zuvor mit kolorektalem Krebs diagnostiziert und auf kolorektalen Krebs behandelt wurde.

11. *In vitro*-Verfahren zum Screenen eines Individuums auf metastatische kolorektale Krebszellen, umfassend die Schritte des Untersuchens einer Probe von extraintestinalem Gewebe und/oder Körperflüssigkeiten von einem Individuum, um zu bestimmen, ob CDX2-mRNA oder -Protein in der Probe vorliegt, wobei die Gegenwart von CDX2-mRNA oder -Protein in der Probe anzeigt, daß das Individuum metastatische kolorektale Krebszellen in der Probe aufweisen kann.

12. Verfahren nach Anspruch 11, umfassend die Schritte des Untersuchens einer Probe von extraintestinalem Gewebe und/oder Körperflüssigkeiten von einem Individuum, um zu bestimmen, ob CDX2-mRNA in der Probe vorliegt.

13. Verfahren nach Anspruch 12, wobei die Gegenwart von CNX2-mRNA durch Polymerasekettenreaktion bestimmt wird, wobei die Probe mit Primern in Kontakt gebracht wird, welche die CDX2-mRNA oder davon erzeugte -cDNA selektiv amplifizieren.

14. Verfahren nach Anspruch 11, wobei die Gegenwart von CDX2-Protein durch ein Immunoassay bestimmt wird, wobei die Probe mit Antikörpern in Kontakt gebracht wird, welche spezifisch an CDX2-Protein binden.

15. Verfahren nach einem der Ansprüche 11 bis 15, wobei die Probe Körperflüssigkeit ist.

16. Verfahren nach einem der Ansprüche 11 bis 15, wobei die Probe Blut ist.

17. Verfahren nach einem der Ansprüche 11 bis 15, wobei die Probe Lymphgewebe und/oder -flüssigkeit ist.

18. Verfahren nach einem der Ansprüche 11 bis 15, wobei die Probe eine Lymphknotenprobe ist.

19. Verfahren nach einem der Ansprüche 11 bis 15, wobei das Individuum zuvor diagnostiziert wurde, kolorektalen Krebs zu haben.

20. Verfahren nach einem der Ansprüche 11 bis 15, wobei das Individuum zuvor mit kolorektalem Krebs diagnostiziert und auf kolorektalen Krebs behandelt wurde.

## Revendications

1. Procédé *in vitro* de criblage d'un individu pour rechercher des cellules métastatiques de cancer colorectal, comprenant les étapes consistant à examiner un échantillon de tissu extra-intestinal et/ou de fluides corporels d'un individu pour déterminer si CDX2 est exprimé par des cellules dans ledit échantillon, où l'expression de ladite protéine CDX2 indique une éventualité de cellules métastatiques de cancer colorectal dans ledit échantillon.

2. Procédé selon la revendication 1, dans lequel l'expression de ladite protéine CDX2 par lesdites cellules est déterminée en détectant la présence d'ARNm de CDX2.

3. Procédé selon la revendication 1, dans lequel l'expression de ladite protéine CDX2 par lesdites cellules est déterminée par une réaction en chaîne par polymérase, dans laquelle ledit échantillon est mis en contact avec des amorces qui amplifient de façon sélective l'ARNm ou l'ADNc généré à partir de celles-ci.

4. Procédé selon la revendication 1, dans lequel l'expression de ladite protéine CDX2 par lesdites cellules est déterminée par immunoessai, dans lequel ledit échantillon est mis en contact avec des anticorps qui lient de façon spécifique la protéine CDX2.

5. Procédé selon l'une des revendications 1 à 4, dans lequel ledit échantillon est un fluide corporel.

6. Procédé selon l'une des revendications 1 à 4, dans lequel ledit échantillon est du sang.

7. Procédé selon l'une des revendications 1 à 4, dans lequel ledit échantillon est un tissu et/ou un fluide lymphatique(s).

8. Procédé selon l'une des revendications 1 à 4, dans lequel ledit échantillon est un échantillon de ganglion lymphatique.

9. Procédé selon l'une des revendications 1 à 4, dans lequel l'individu a été au préalable diagnostiqué comme ayant un cancer colorectal.

10. Procédé selon l'une des revendications 1 à 4, dans lequel l'individu a été au préalable diagnostiqué et traité pour un cancer colorectal.

11. Procédé *in vitro* de criblage d'un individu pour détecter des cellules métastatiques de cancer colorectal, comprenant les étapes consistant à examiner un échantillon de tissu extra-intestinal et/ou de fluides corporels d'un individu pour déterminer si de l'ARNm de CDX2 ou la protéine est présent(e) dans ledit échantillon, dans lequel la présence d'ARNm de CDX2 ou de la protéine dans ledit échantillon indique que l'individu peut avoir des cellules métastatiques de cancer colorectal dans ledit échantillon.

12. Procédé selon la revendication 11, comprenant les étapes consistant à examiner un échantillon de tissu extra-intestinal et/ou de fluides corporels d'un individu pour déterminer si l'ARNm de CDX2 est présent dans ledit échantillon.

13. Procédé selon la revendication 12, dans lequel la présence d'ARNm de CDX2 est déterminée par une réaction en chaîne par polymérase, dans laquelle ledit échantillon est mis en contact avec des amorces qui amplifient de façon sélective l'ARNm ou l' ADNc de CDX2 généré à partir de celles-ci.

14. Procédé selon la revendication 11, dans lequel la présence d'une protéine CDX2 est déterminée par immunoessai, dans lequel ledit échantillon est mis en contact avec des anticorps qui lient de façon spécifique la protéine CDX2.

15. Procédé selon l'une des revendications 11 à 15, dans lequel ledit échantillon est un fluide corporel.

16. Procédé selon l'une des revendications 11 à 15, dans lequel ledit échantillon est du sang.

17. Procédé selon l'une des revendications 11 à 15, dans lequel ledit échantillon est un tissu et/ou un fluide lymphatique(s).

18. Procédé selon l'une des revendications 11 à 15, dans lequel ledit échantillon est un échantillon de ganglion lymphatique.

19. Procédé selon l'une des revendications 11 à 15, dans lequel l'individu a été au préalable diagnostiqué comme ayant un cancer colorectal.

20. Procédé selon l'une des revendications 11 à 15, dans lequel l'individu a été au préalable diagnostiqué et traité pour un cancer colorectal.
